# EUROPEAN PATENT SPECIFICATION

(11) **EP 2 438 187 B1**
(45) Date of publication and mention of the grant of the patent: **22.03.2017**
(21) Application number: 10722116.0
(22) Date of filing: 07.06.2010
(51) Int. Cl.: C12Q 1/68

(54) **MIRNA FINGERPRINT IN THE DIAGNOSIS OF MULTIPLE SCLEROSIS**
MIRNA-FINGERABDRUCK BEI DER DIAGNOSE VON MULTIPLER SKLEROSE
EMPREINTE D'ARNMI DANS LE DIAGNOSTIC DE LA SCLÉROSE EN PLAQUES

(30) Priority: 05.06.2009 US 184452 P; 03.08.2009 US 213971 P; 17.12.2009 US 287521 P; 17.12.2009 EP 09015668
(43) Date of publication of application: 11.04.2012
(60) Divisional application: 17155238.3
(73) Proprietor: Hummingbird Diagnostics GmbH, 69120 Heidelberg (DE)
(72) Inventor: KELLER, Andreas, 66346 Püttlingen (DE); MEESE, Eckart, 66882 Huetschenhausen (DE); BORRIES, Anne, 68169 Mannheim (DE); STÄHLER, Peer Friedrich, 68167 Mannheim (DE); BEIER, Markus, 69469 Weinheim (DE)
(74) Representative: Geling, Andrea
(86) International application number: PCT/EP2010/057943
(87) International publication number: WO 2010/139811

(56) References cited:
- US-A1- 2007 161 004
- ARNETT H A ET AL: "MICRORNA EXPRESSION PROFILING IN MULTIPLE SCLEROSIS AND EAE" JOURNAL OF NEUROCHEMISTRY, vol. 108, no. Suppl. 1, March 2009 (2009-03), page 81, XP002596357 & 40TH ANNUAL MEETING OF THE AMERICAN-SOCIETY-FOR-NEUROCHEMISTRY; CHARLESTON, SC, USA ISSN: 0022-3042
- ACHIRON A ET AL: "Peripheral blood gene expression signature mirrors central nervous system disease: the model of multiple sclerosis." AUTOIMMUNITY REVIEWS, vol. 5, no. 8, October 2006 (2006-10), pages 517-522, XP002596358 ISSN: 1568-9972
- PAULEY KALEB M ET AL: "Upregulated miR-146a expression in peripheral blood mononuclear cells from rheumatoid arthritis patients." ARTHRITIS RESEARCH & THERAPY, vol. 10, no. 4, R101, 29 August 2008 (2008-08-29), pages 1-10, XP002596359 ISSN: 1478-6362
- KELLER ANDREAS ET AL: "Multiple sclerosis: microRNA expression profiles accurately differentiate patients with relapsing-remitting disease from healthy controls." PLOS ONE, vol. 4, no. 10, E7440, 13 October 2009 (2009-10-13), pages 1-7, XP002596360 ISSN: 1932-6203
- OTAEGUI DAVID ET AL: "Differential micro RNA expression in PBMC from multiple sclerosis patients." PLOS ONE, vol. 4, no. 7, E6309, 20 July 2009 (2009-07-20), pages 1-9, XP002596361 ISSN: 1932-6203

## Description

### Background of the invention

MicroRNAs (miRNA) are a recently discovered class of small non-coding RNAs (17-14 nucleotides). Due to their function as regulators of gene expression they play a critical role both in physiological and in pathological processes, such as cancer (Calin and Croce 2006; Esquela-Kerscher and Slack 2006; Zhang, Pan et al. 2007; Sassen, Miska et al. 2008).

There is increasing evidence that miRNAs are not only found in tissues but also in human blood both as free circulating nucleic acids (also called circulating miRNAs) and in mononuclear cells. A recent proof-of-principle study demonstrated miRNA expression pattern in pooled blood sera and pooled blood cells, both in healthy individuals and in cancer patients including patients with lung cancer (Chen, Ba et al. 2008). In addition, a remarkable stability of miRNAs in human sera was recently demonstrated (Chen, Ba et al. 2008; Gilad, Meiri et al. 2008). These findings make miRNA a potential tool for diagnostics for various types of diseases based on blood analysis.

Multiple sclerosis (MS), also known as disseminated sclerosis or encephalomyelitis disseminata) is an inflammatory autoimmune disease of the central nervous system (CNS). Causing MS appears to be a combination of immunological, genetic and environmental factors. It is a chronic demyelinating disease, which primarily affects young adults and is characterized by a highly variable course. The heterogeneous presentation of MS is characterized by a variety of clinical problems arising from multiple regions of demyelination and inflammation along axonal pathways. The signs and symptoms of MS are determined by the location of the affected regions.

Mostly, the disease begins in the third or fourth decade of life. Its initial course is characterized by acute episodes of neurological dysfunction, such as decreased vision, followed by subsequent recovery. This course is known as relapsing-remitting MS. Over time, the improvement after attacks may be incomplete and the relapsing-remitting course may evolve into one of increasing progression of disability, termed secondary progressive MS.

The diagnosis of MS generally relies on the presence of a neurological problem that remits and then returns at an unrelated site. This is confirmed by magnetic resonance imaging (MRI) or functional evidence of lesions in a particular pathway by abnormal evoked potentials. The histological hallmark of MS at postmortem exam is multiple lesions at different sites showing loss of myelin and infiltration by a characteristic complement of inflammatory cells.

The key to identifying predictive markers is a deeper understanding of the factors that underlie the therapeutic response. Identification of biomarkers will in turn allow for stratification of MS patients for their response to a specific treatment, ultimately leading to improved therapeutic benefits and a personalized treatment approach for MS patients.

Identification of reliable biomarkers in MS sclerosis patients bears the potential for an improved MS diagnosis, monitoring the disease activity and progression and also to evaluate response to treatments. The field of biomarker discovery has gradually shifted from the aim to find the perfect surrogate marker to the construction of composite markers with higher performances, taking advantage of technologies allowing unbiased screening, including microarray analyses. However, suitable biomarker sets allowing for a non-invasive diagnosis of MS based on peripheral profiles have not been detected, so far.

Various markers have been proposed to indicate specific types of disorders such as cancer or MS. However, there is still a need for more efficient and effective methods and compositions for the diagnosis of diseases.

### Summary of the invention

The present invention provides novel methods for diagnosing diseases based on the determination of specific miRNAs that have altered expression levels in disease states compared to healthy or other relevant controls. The present invention particularly provides novel methods for the diagnosis and/or prognosis and/or monitoring of multiple sclerosis or related diseases in human individuals based on miRNA analysis from samples derived from blood.

A first subject-matter of the invention is a method for diagnosing multiple sclerosis, comprising the steps
(a) determining an expression profile of a predetermined set of miRNAs in a biological sample from a patient; and
(b) comparing said expression profile to a reference expression profile,
wherein the comparison of said determined expression profile to said reference expression profile allows for the diagnosis of multiple sclerosis.

A "biological sample" in terms of the invention means a sample of biological tissue or fluid. Examples of biological samples are sections of tissues, blood, blood fractions, plasma, serum, etc. A biological sample may be provided by removing a sample of cells from a subject, but can also be provided by using a previously isolated sample. For example, a tissue sample can be removed from a subject suspected of having a disease by conventional biopsy techniques. In a preferred embodiment, a blood sample is taken from the subject. In one embodiment, the blood or tissue sample is obtained from the subject prior to initiation of radiotherapy, chemotherapy or other therapeutic treatment. According to the invention, the biological sample preferably is a blood, plasma, or PBMC (peripheral blood mononuclear cell), or a serum sample. Further, it is also preferred to use blood cells, e.g. erythrocytes, leukocytes or thrombocytes.

A biological sample from a patient means a sample from a subject suspected to be affected by a disease. As used herein, the term "subject" refers to any mammal, including both human and other mammals. Preferably, the methods of the present invention are applied to human subjects.

In step (a) of the method of the invention, an expression profile of a predetermined set of miRNAs is determined. The determination may be carried out by any convenient means for determining nucleic acids. For expression profiling, qualitative, semi-quantitative and preferably quantitative detection methods can be used. A variety of techniques are well known to those of skill in the art. In particular, the determination may comprise nucleic acid hybridization and/or nucleic acid amplification steps.

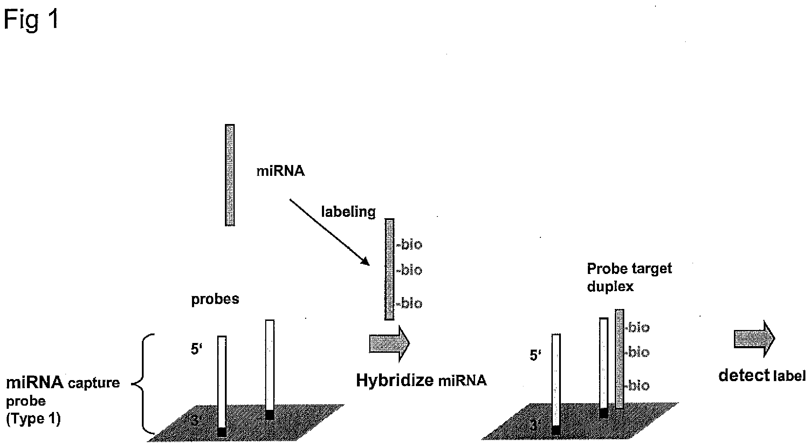

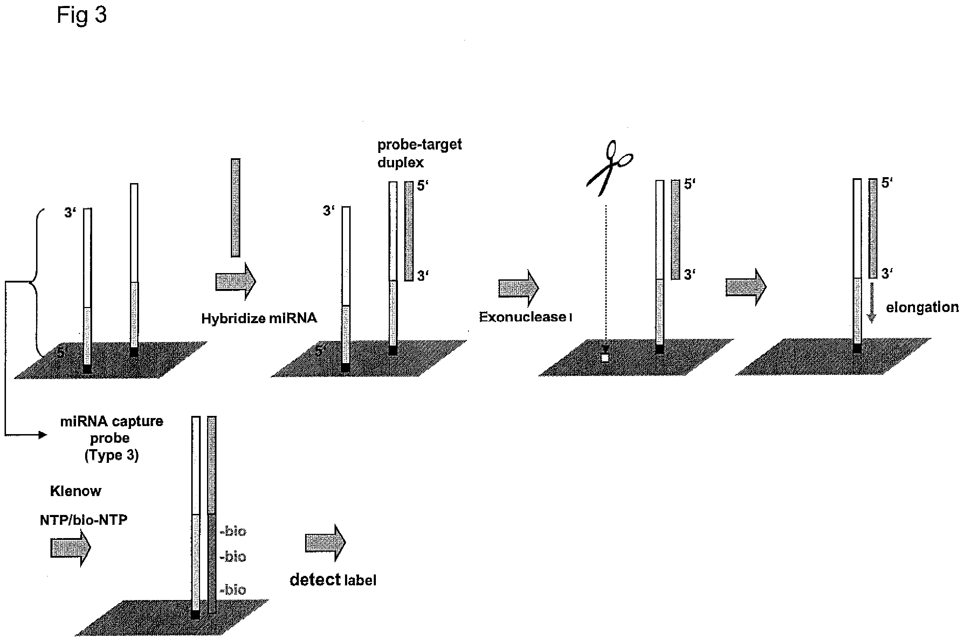

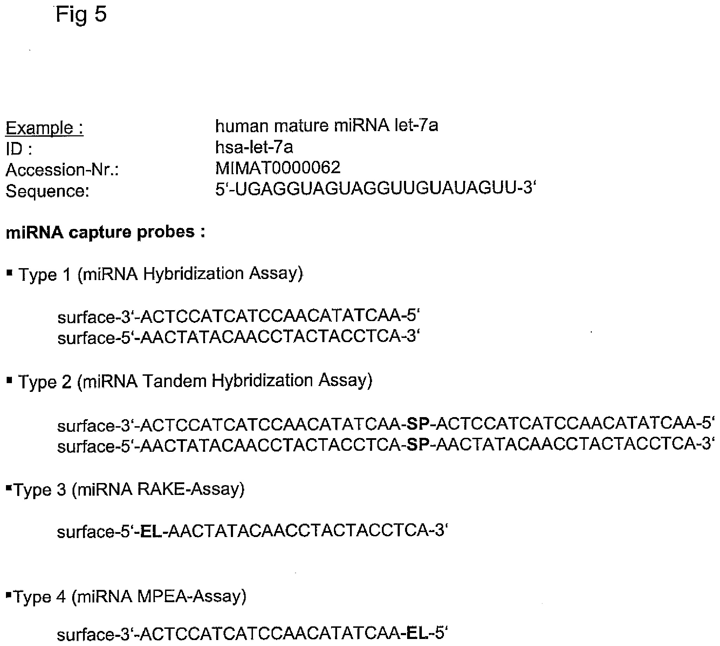

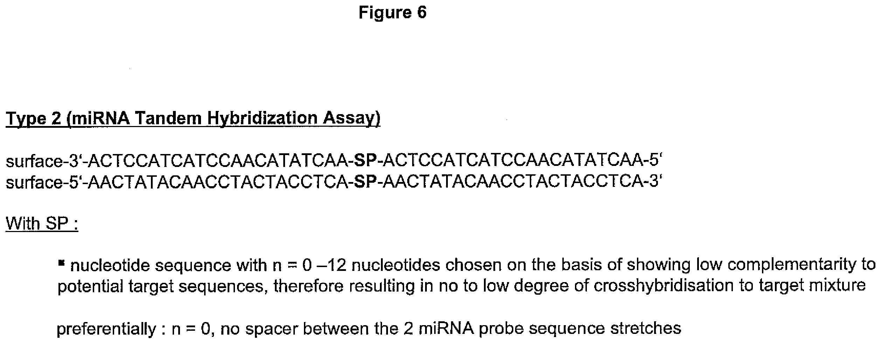

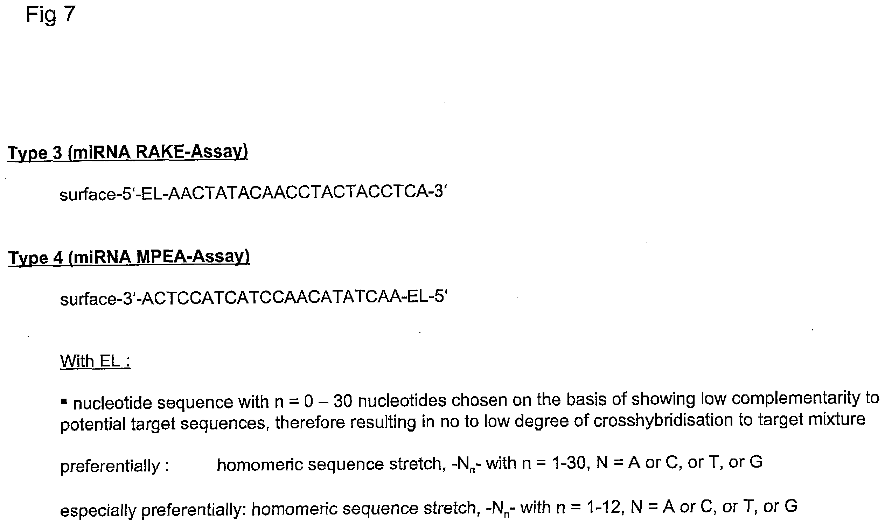

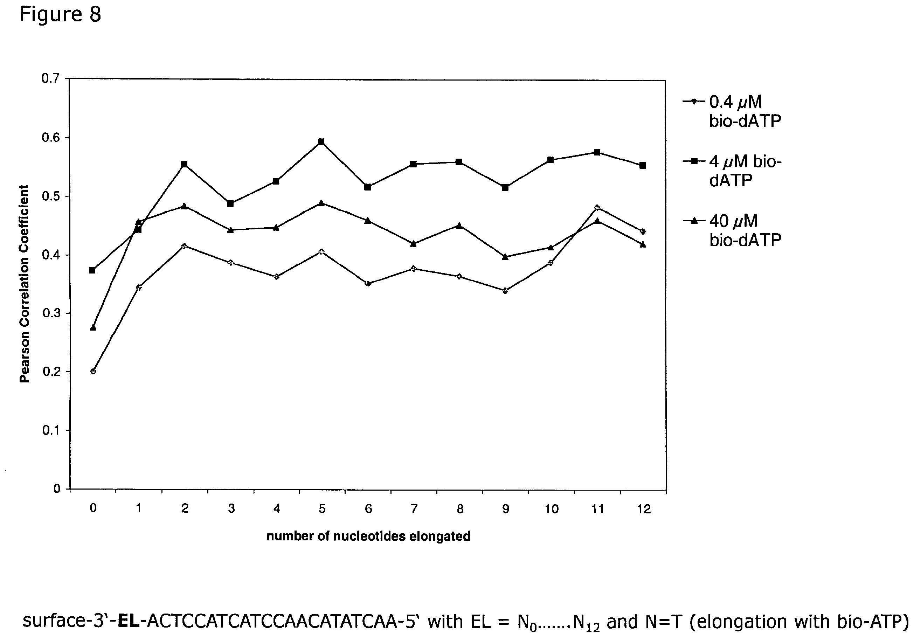

Nucleic acid hybridization may for example be performed using beads, a solid phase nucleic acid biochip array, in particular a microarray, beads, or *in* situ hybridization. The miRNA microarray technology affords the analysis of a complex biological sample for all expressed miRNAs. Nucleotides with complementarity to the corresponding miRNAs are spotted, or synthesized on coated carriers. E.g., miRNAs isolated from the sample of interest may be labelled, e.g. fluorescently labelled, so that upon hybridization of the miRNAs to the complementary sequences on the carrier the resulting signal indicates the occurrence of a distinct miRNA. Preferably, microarray methods are employed that do not require labeling of the miRNAs prior to hybridization (Fig. 3-4) and start directly from total RNA input. On one miRNA microarray, preferably the whole predetermined set of miRNAs can be analyzed. Examples of preferred hybridization assays are shown in Figures 1-4. The design of exemplary miRNA capture probes for use in hybridization assays is depicted in Figures 5 and 6.

Further, quantitative real-time polymerase chain reaction (RT-PCR) can be used to detect also low abandoned miRNAs. Furthermore, bead-based assays, e.g. the luminex platform, are also suitable.

Furthermore, bead-based assays, e.g. the Luminex platform, are also suited to carry out the present invention.

Alternative methods for obtaining expression profiles may also contain sequencing, next generation sequencing or mass spectroscopy.

The predetermined set of miRNAs in step (a) of the method of the invention depends on the disease to be diagnosed. The inventors found out that single miRNA biomarkers lack sufficient accuracy, specificity and sensitivity, and therefore it is preferred to analyze more complex miRNA expression patterns, so-called miRNA signatures. The predetermined set of miRNAs comprises one or more, preferably a larger number of miRNAs (miRNA signatures) that are differentially regulated in samples of a patient affected by a particular disease compared to healthy or other relevant controls.

The expression profile determined in step (a) is subsequently compared to a reference expression profile or a relevant reference in step (b). This reference may be a mathematical function or algorithm that allows for the diagnosis of the disease. This mathematical function or algorithm has been developed on the basis of a plurality of reference expression profiles. The reference expression profile is the expression profile of the same set of miRNAs in a biological sample originating from the same source as the biological sample from a patient but obtained from a healthy subject. Preferably, both the reference expression profile and the expression profile of step (a) are determined in a blood or serum sample including whole blood, plasma, serum or fractions thereof, or in a sample of peripheral blood mononuclear cells, erythrocytes, leukocytes and/or thrombocytes. It is understood that the reference expression profile is not necessarily obtained from a single healthy subject but may be an average expression profile of a plurality of healthy subjects. It is preferred to use a reference expression profile obtained from a person of the same gender, and a similar age as the patient. It is also understood that the reference expression profile is not necessarily determined for each test. Appropriate reference profiles stored in databases may also be used. These stored references profiles may, e.g., be derived from previous tests. The reference profile may also be a mathematical function or algorithm which is developed on a plurality of measured reference profiles.

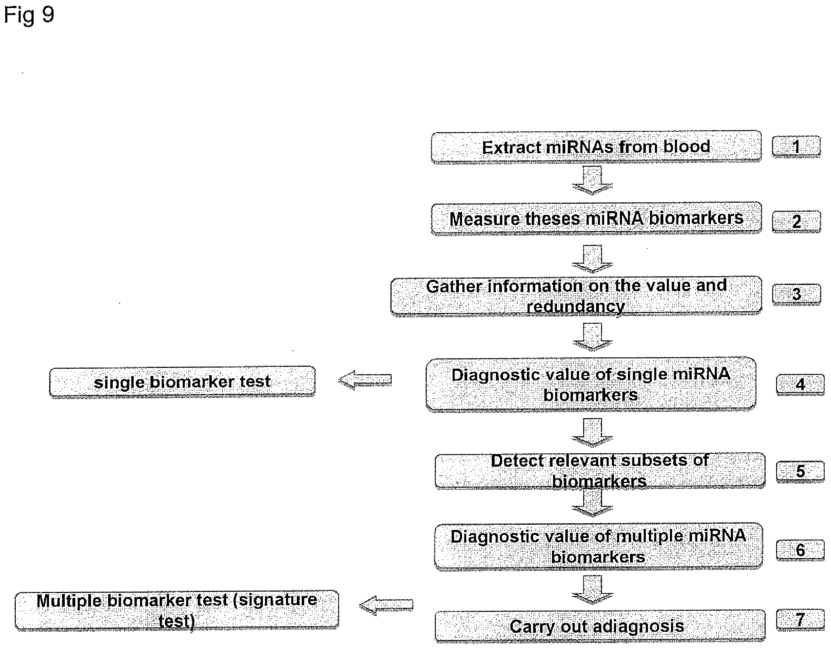

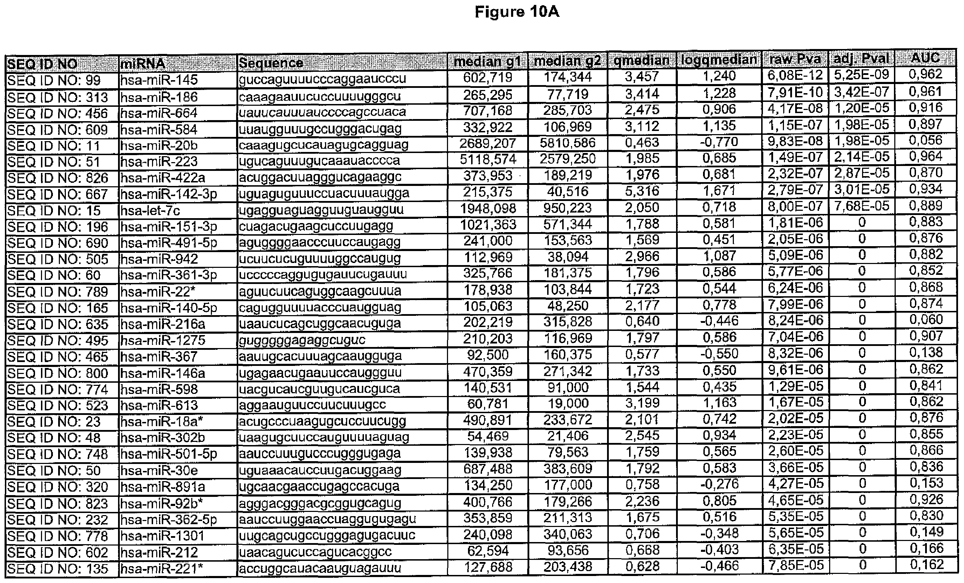

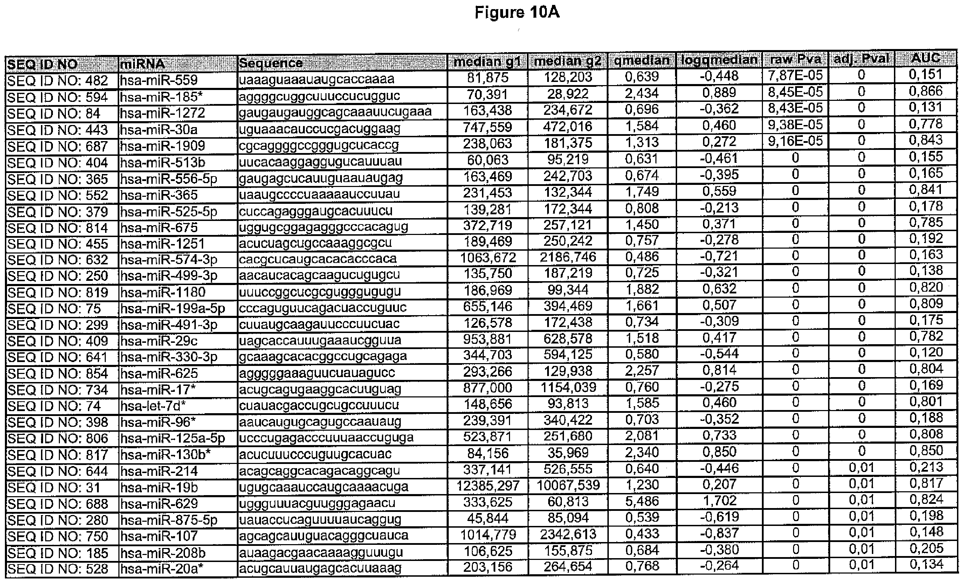

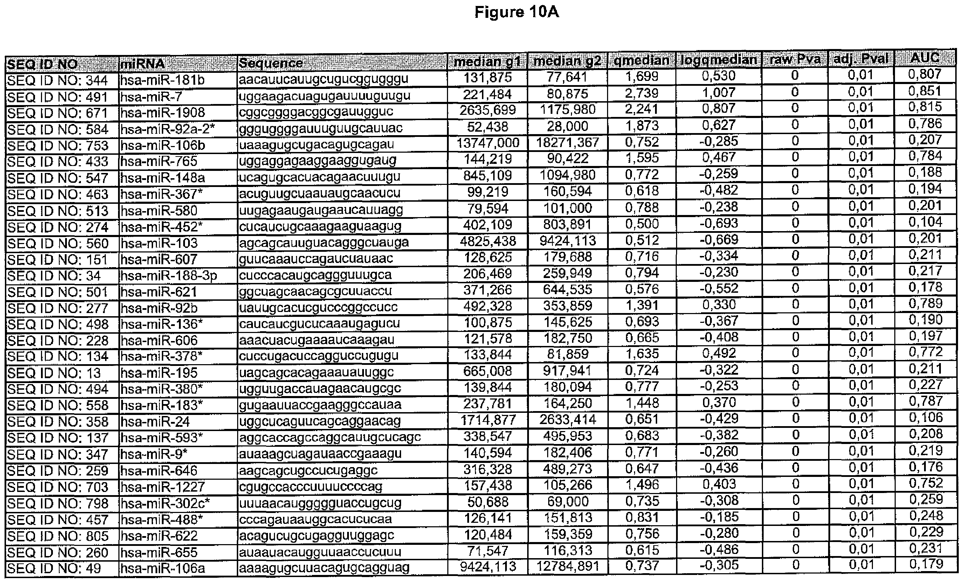

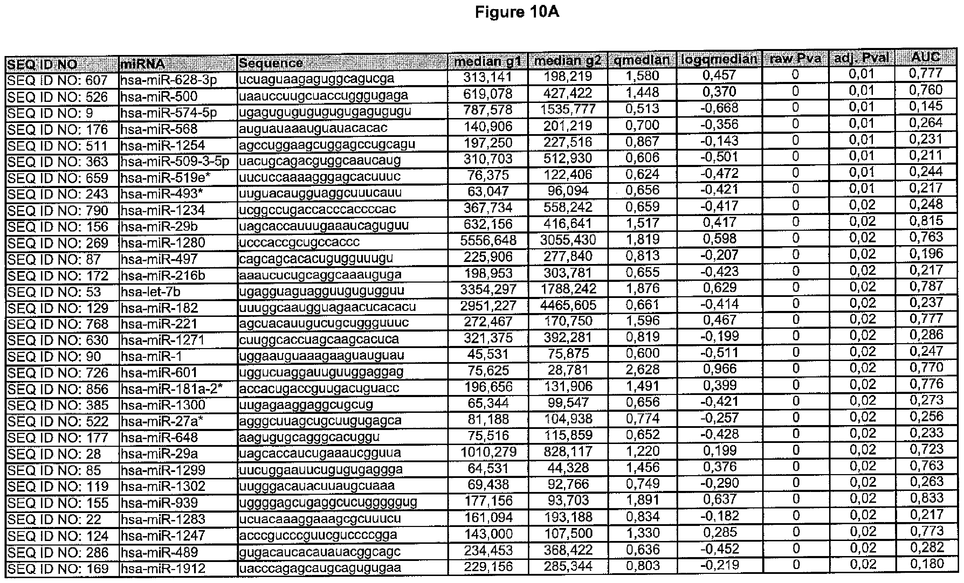

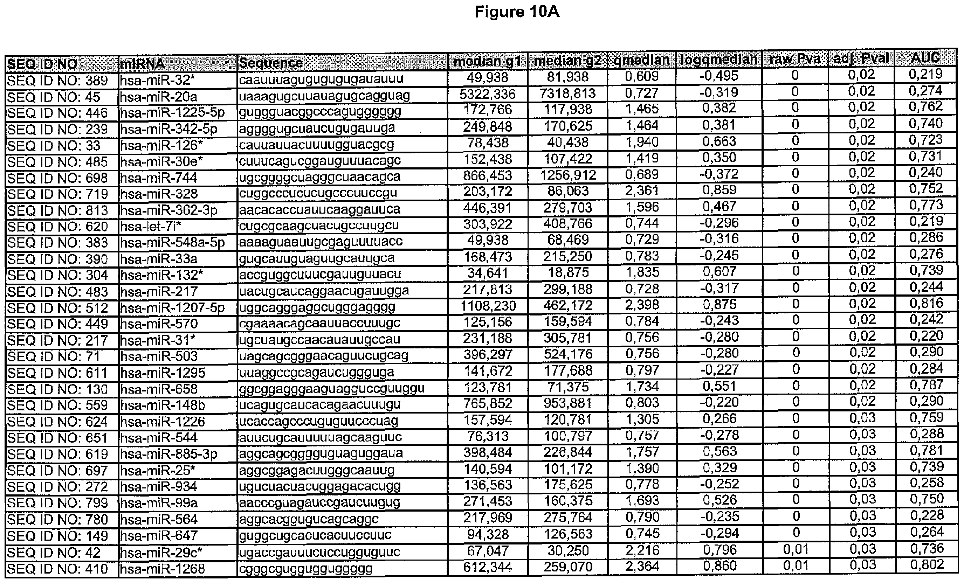

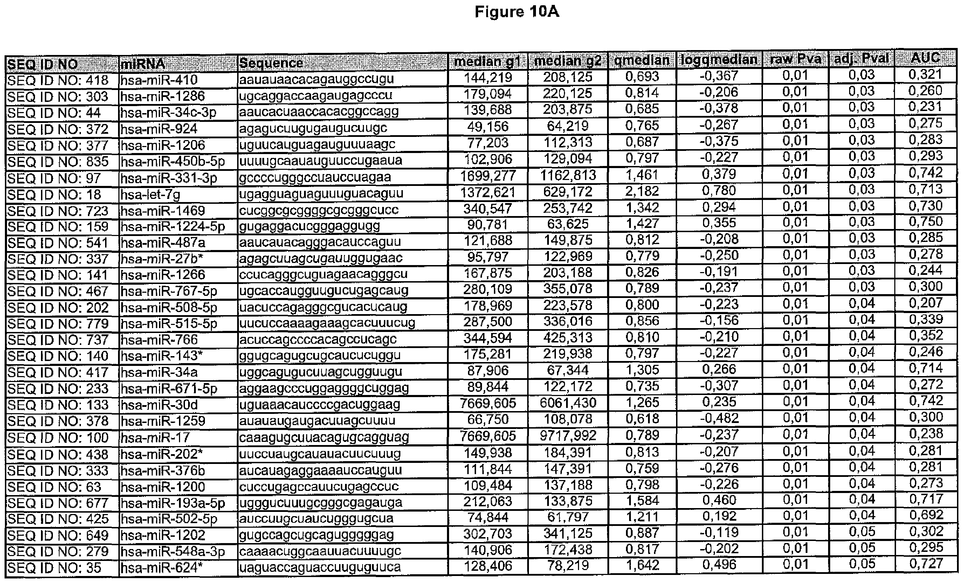

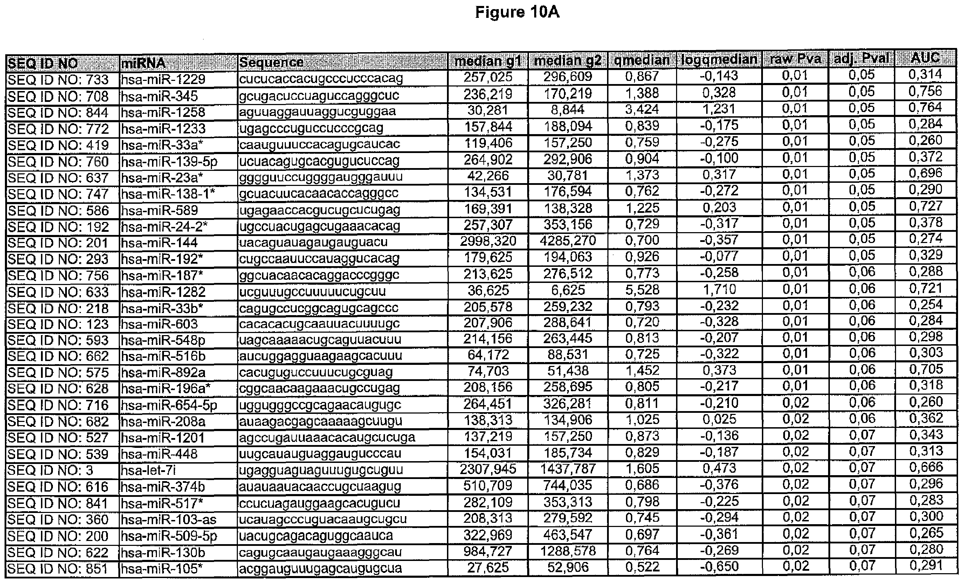

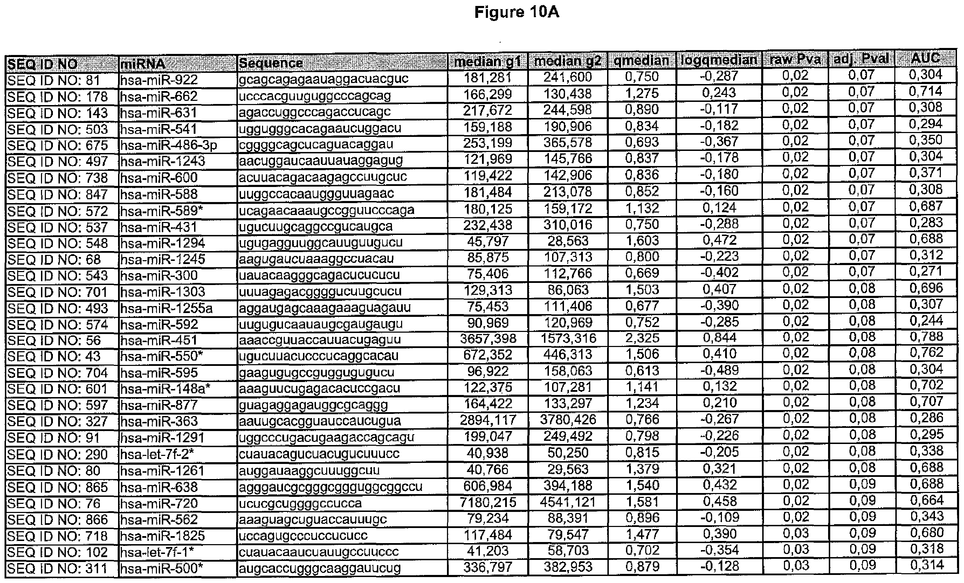

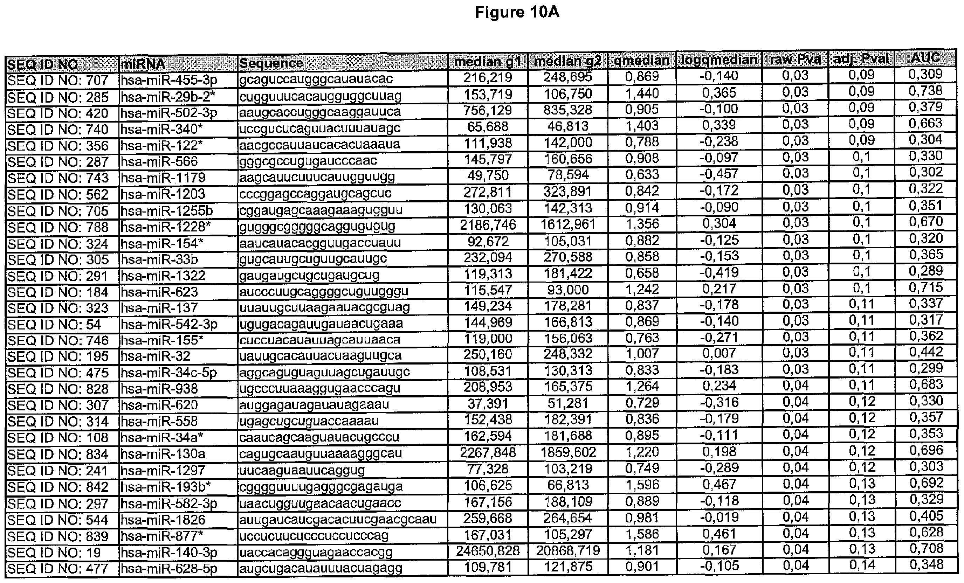

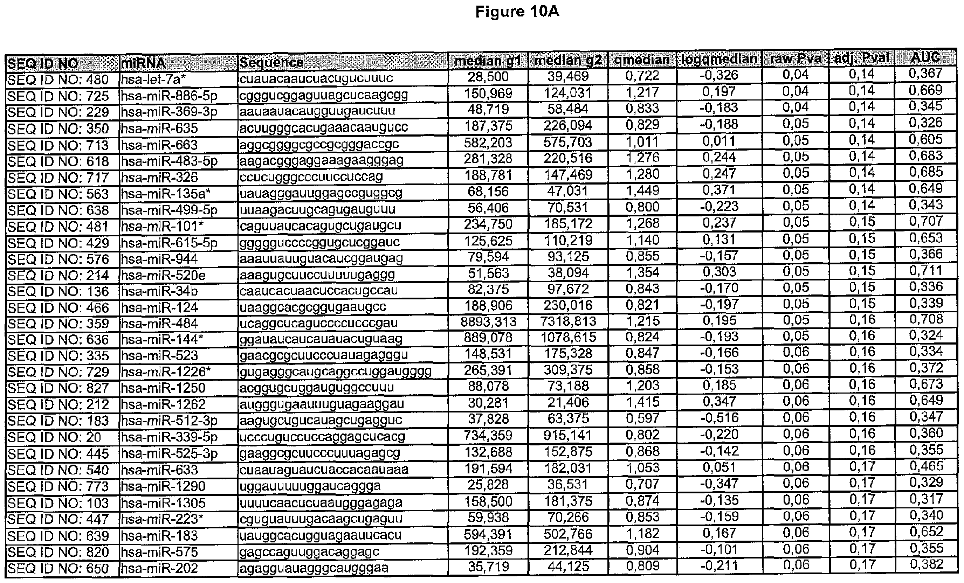

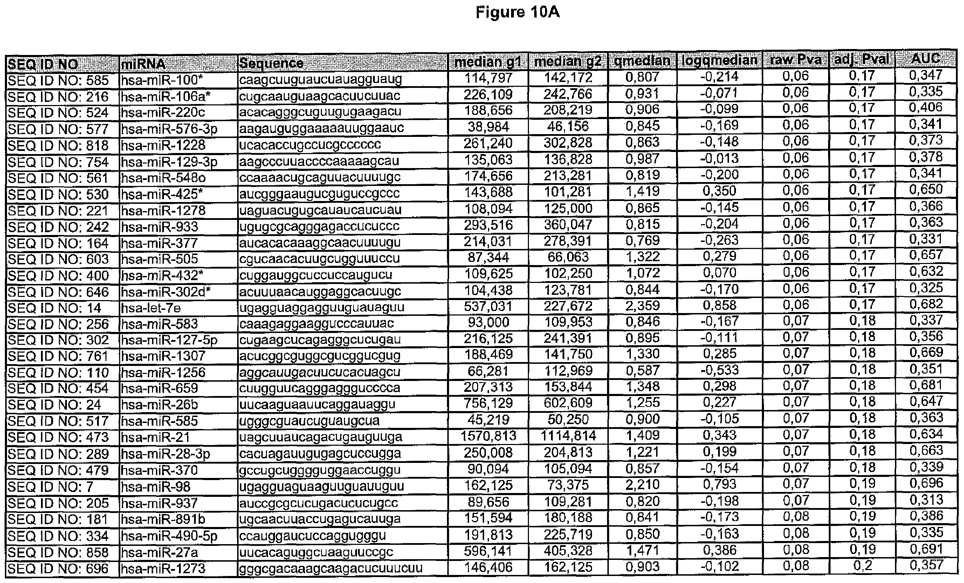

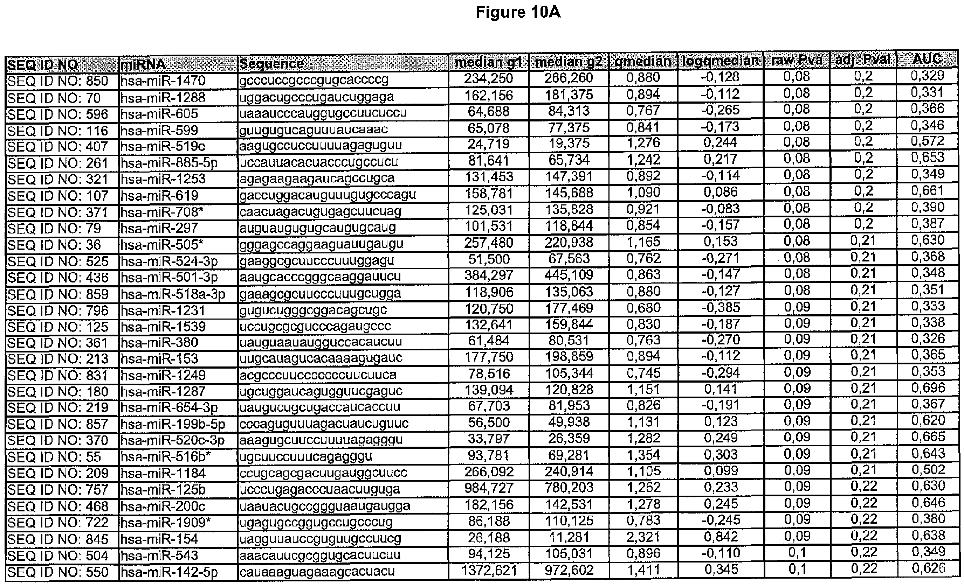

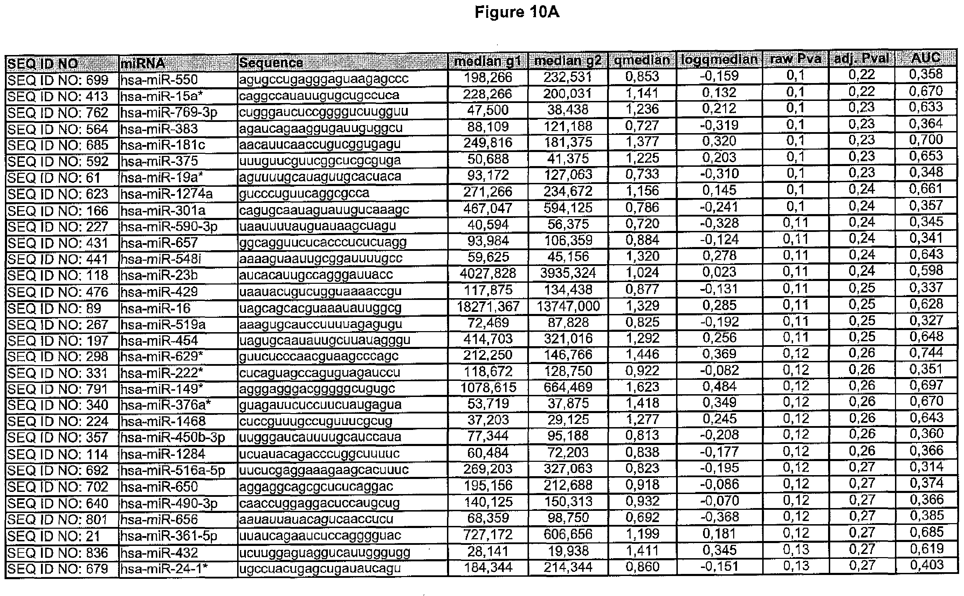

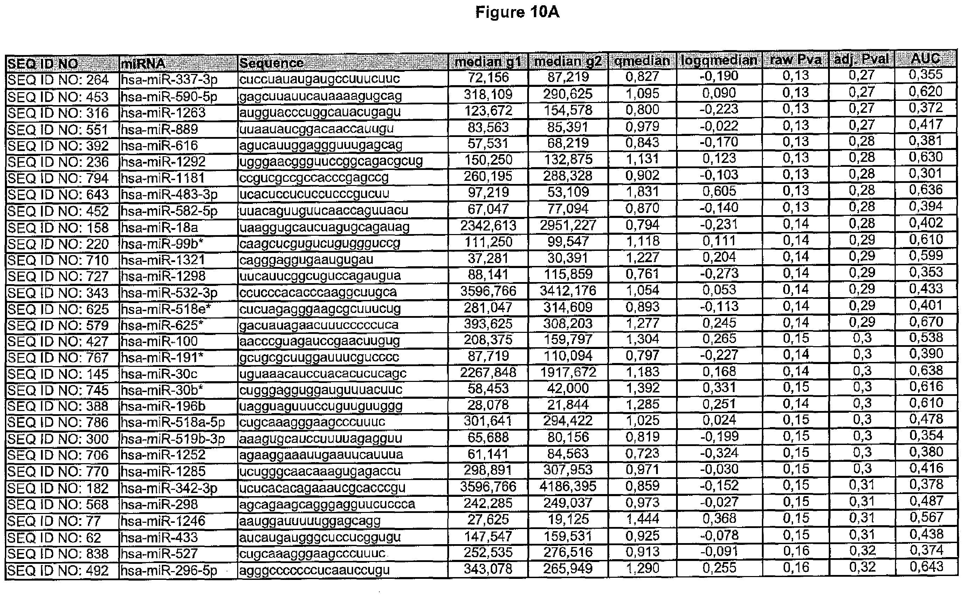

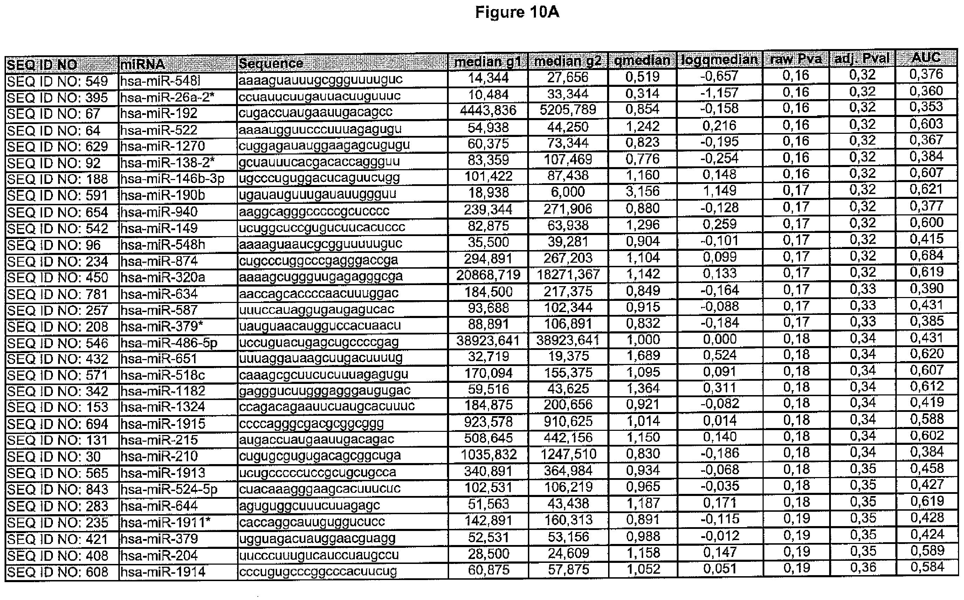

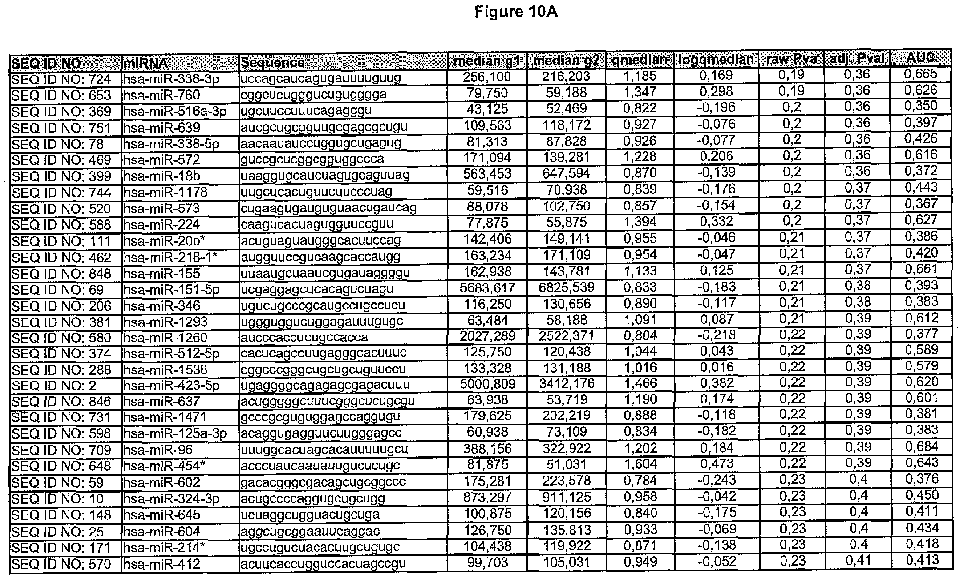

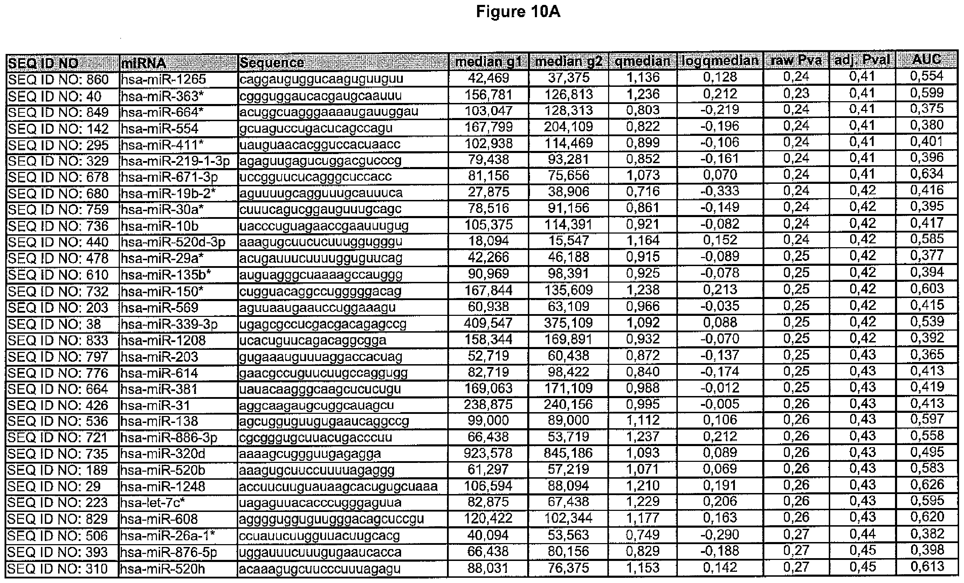

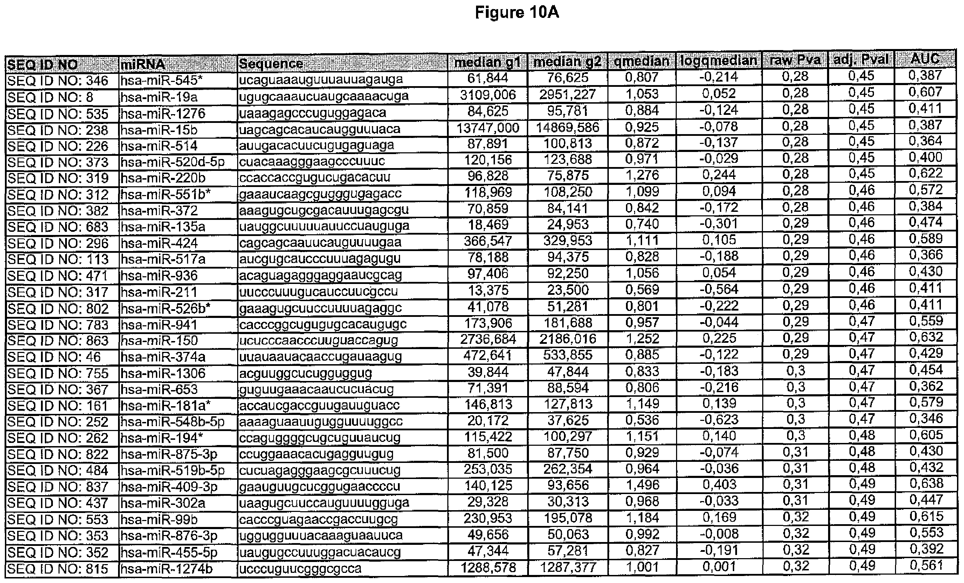

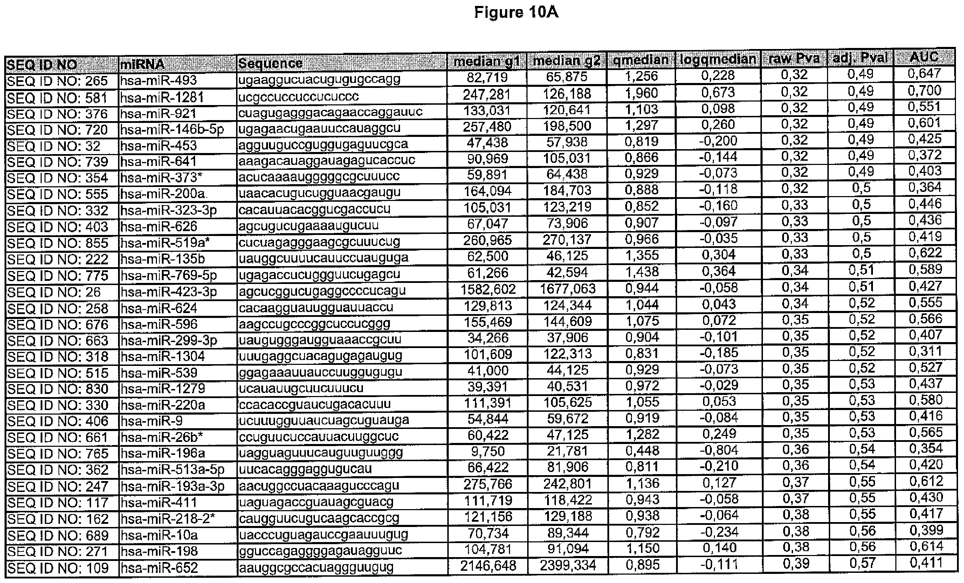

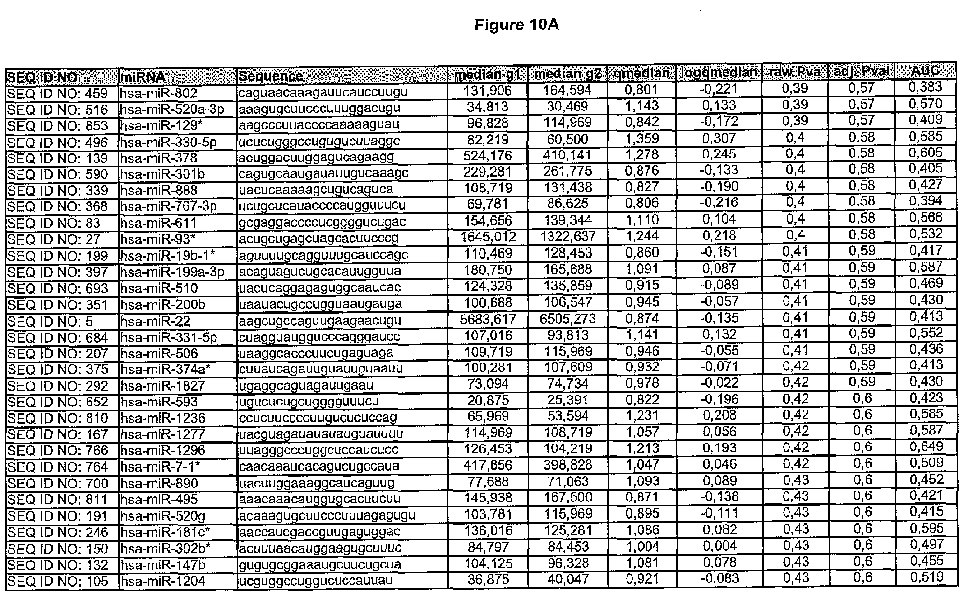

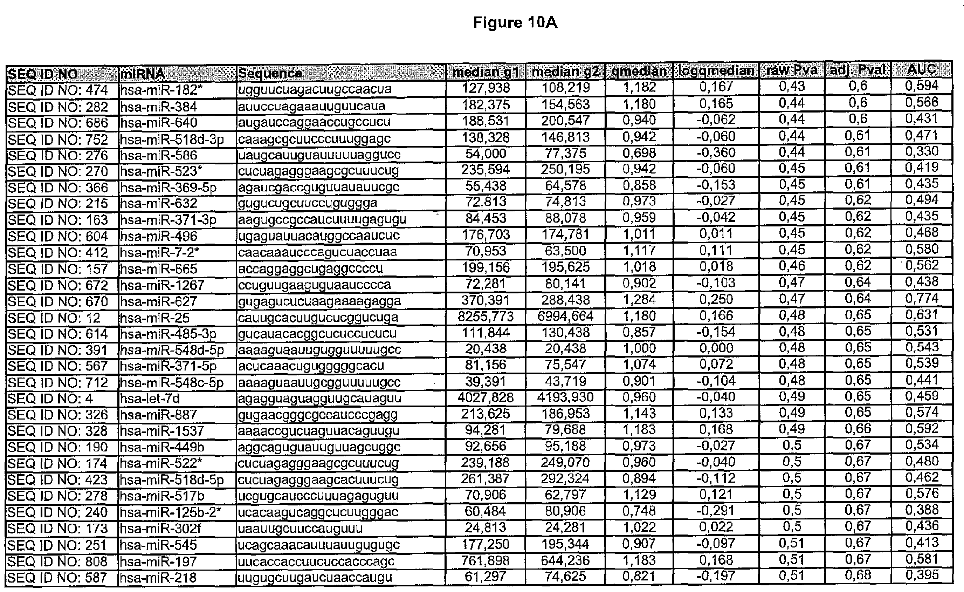

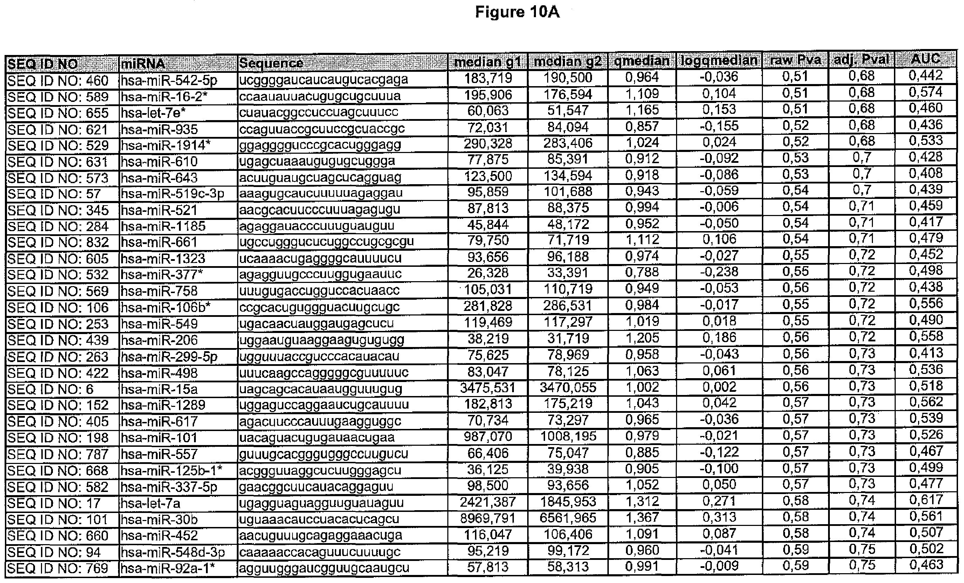

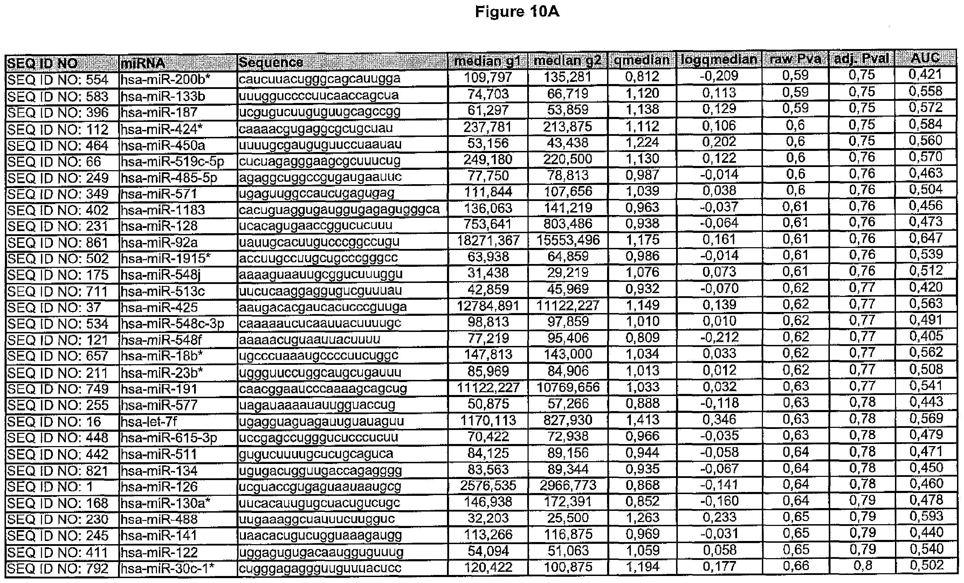

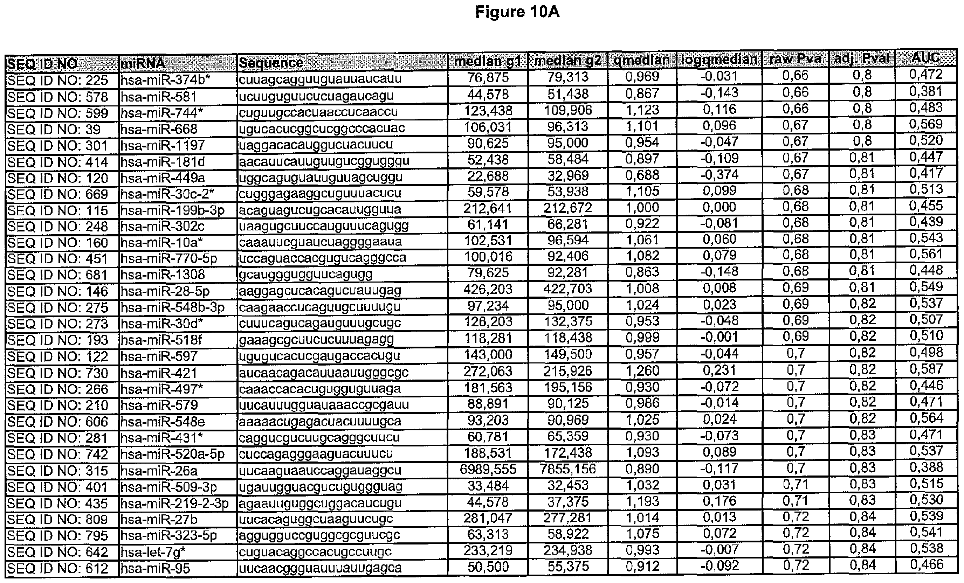

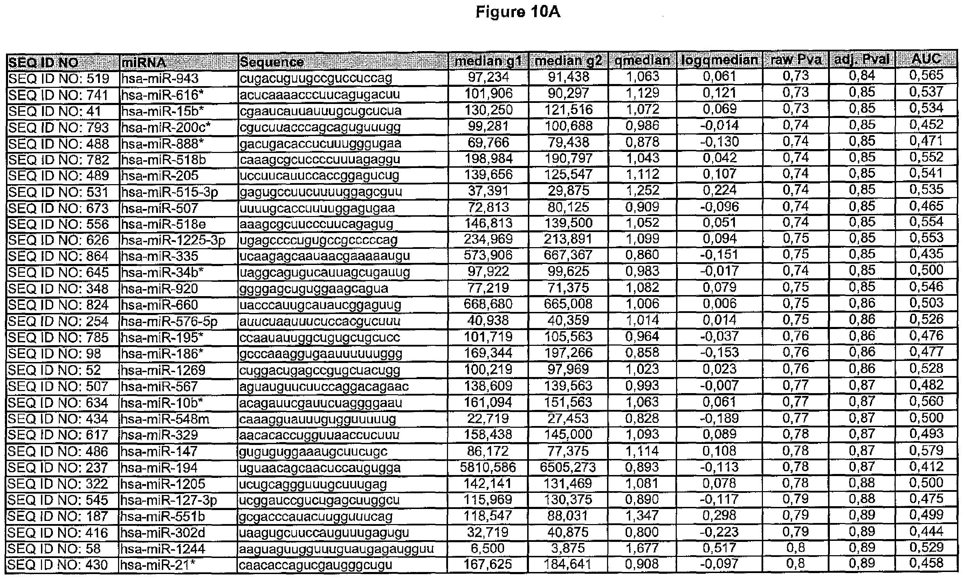

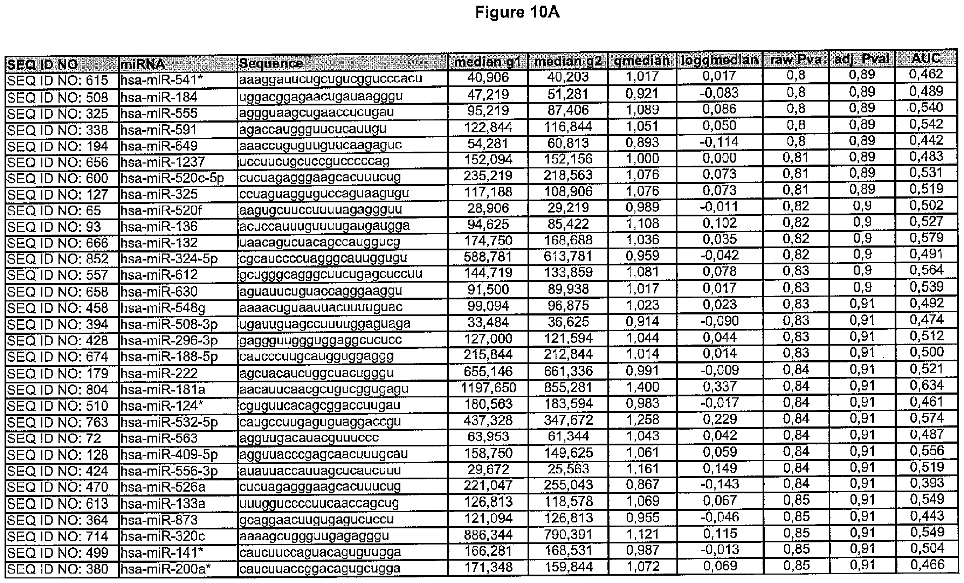

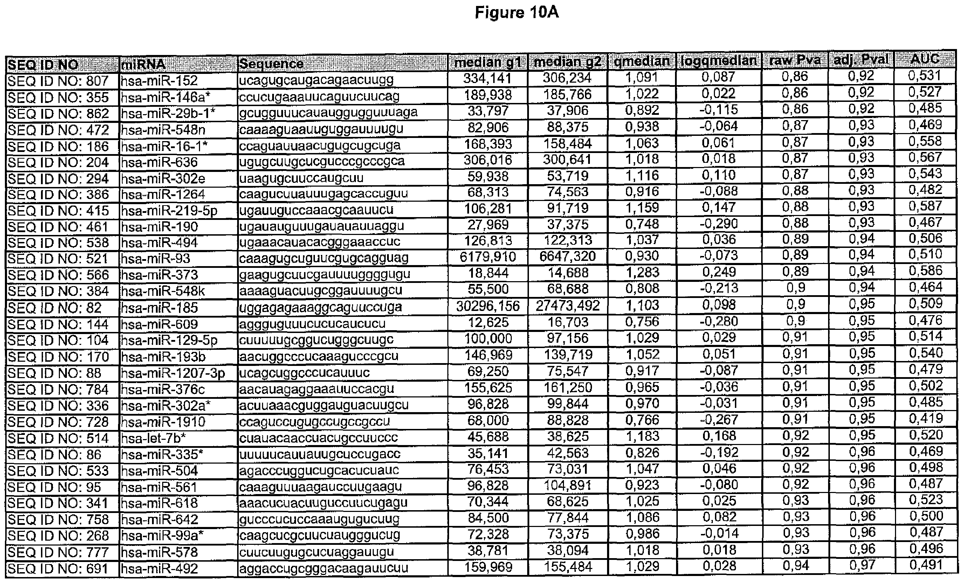

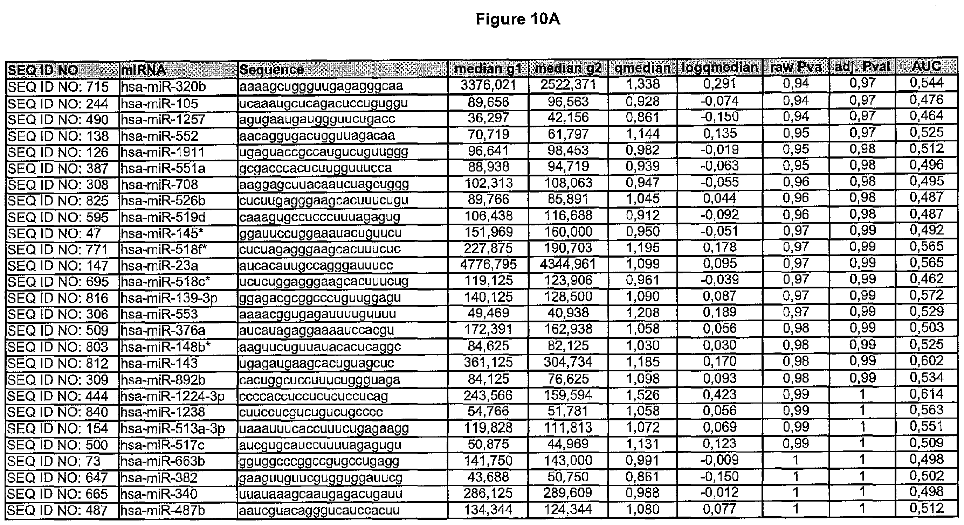

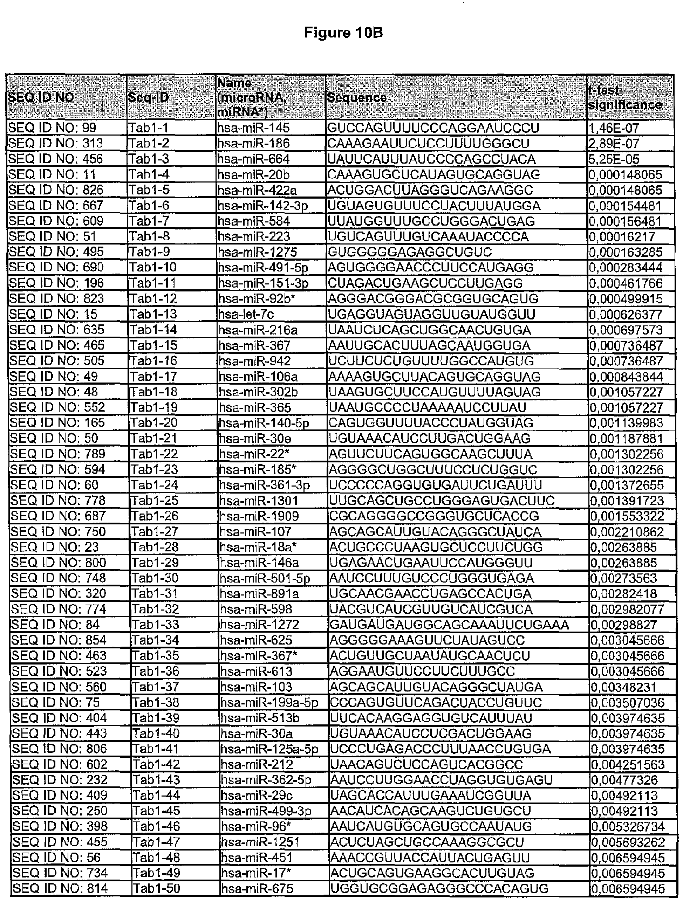

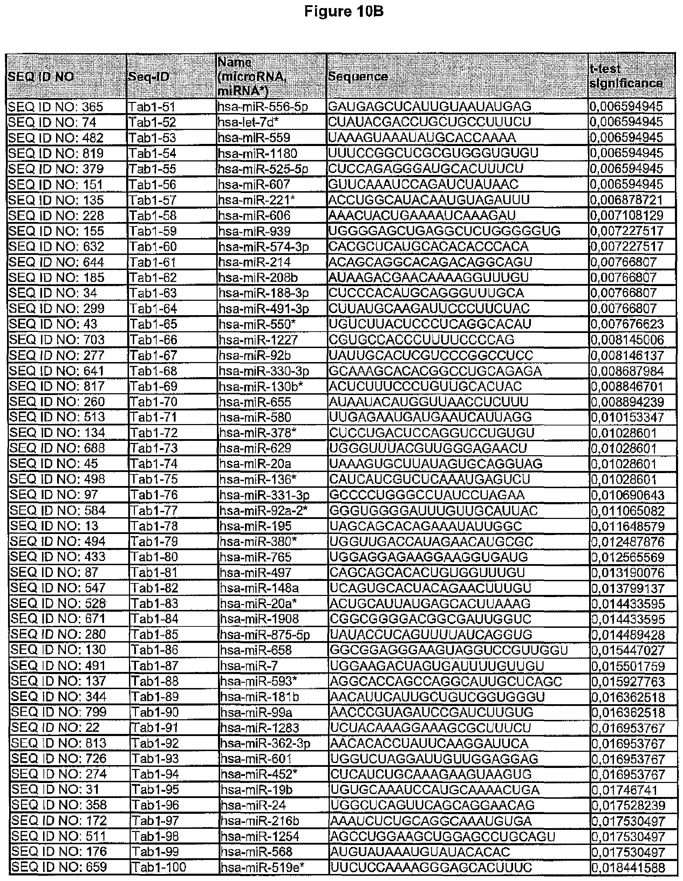

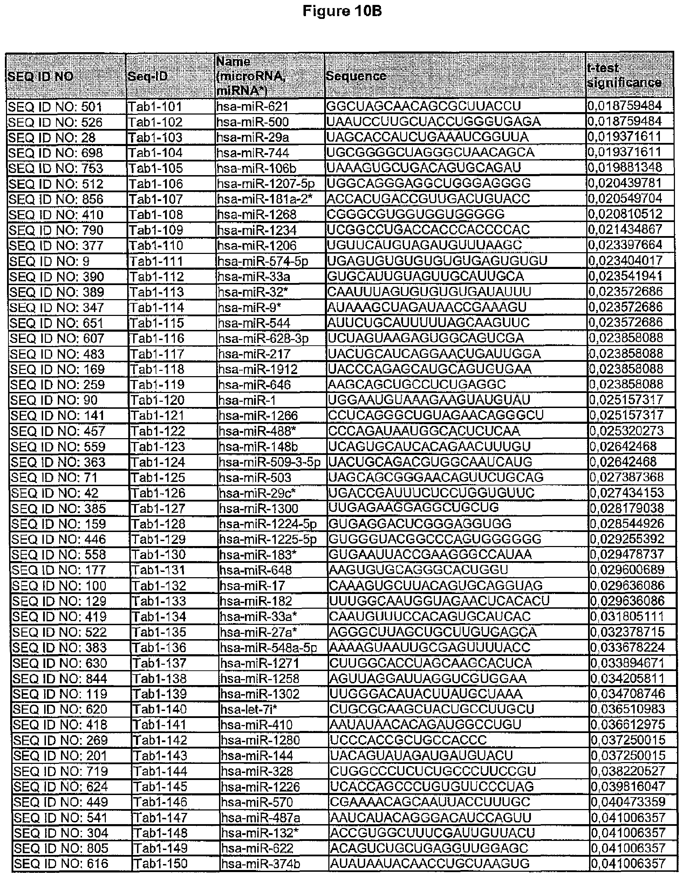

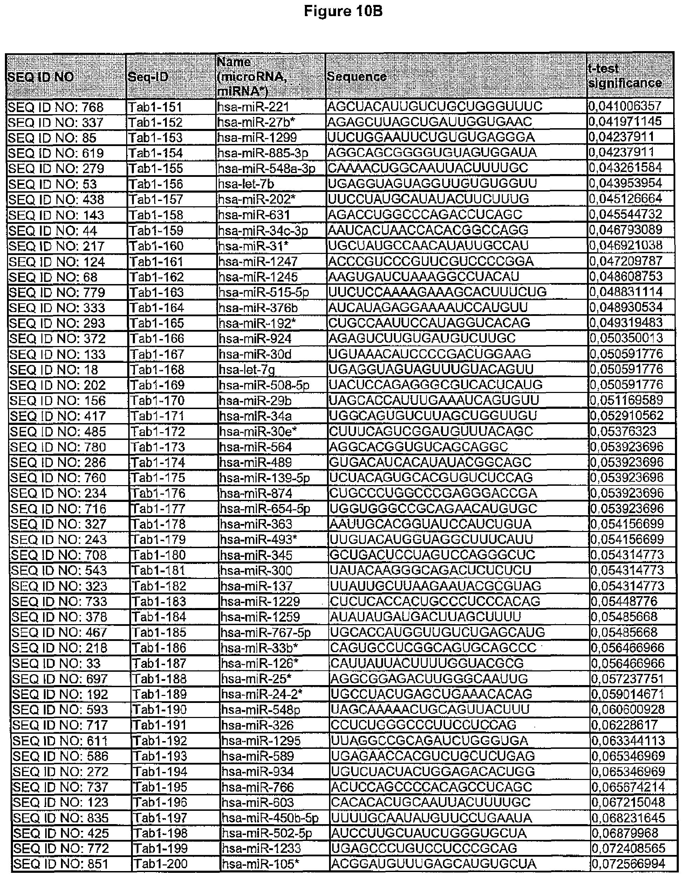

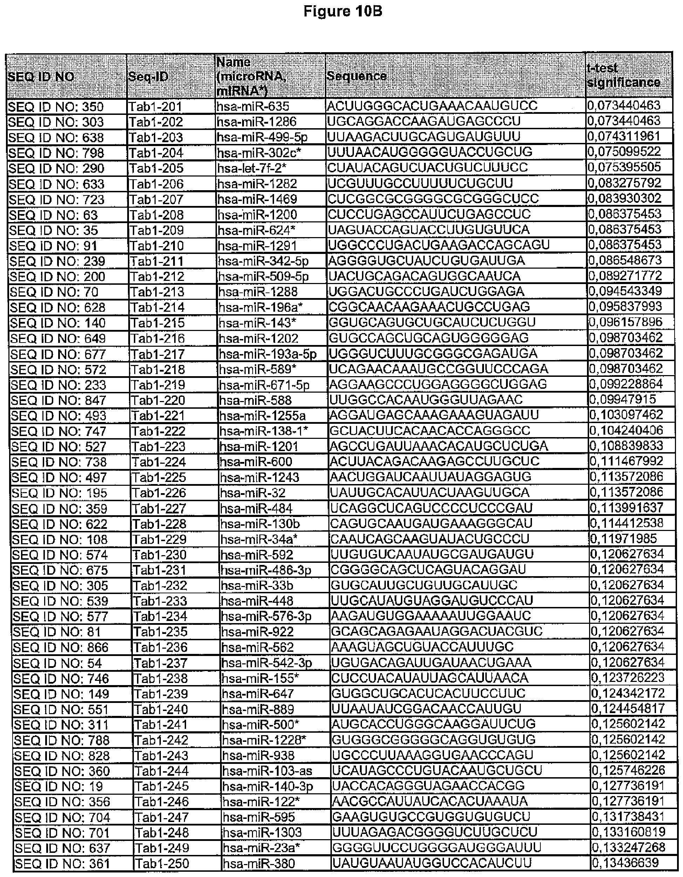

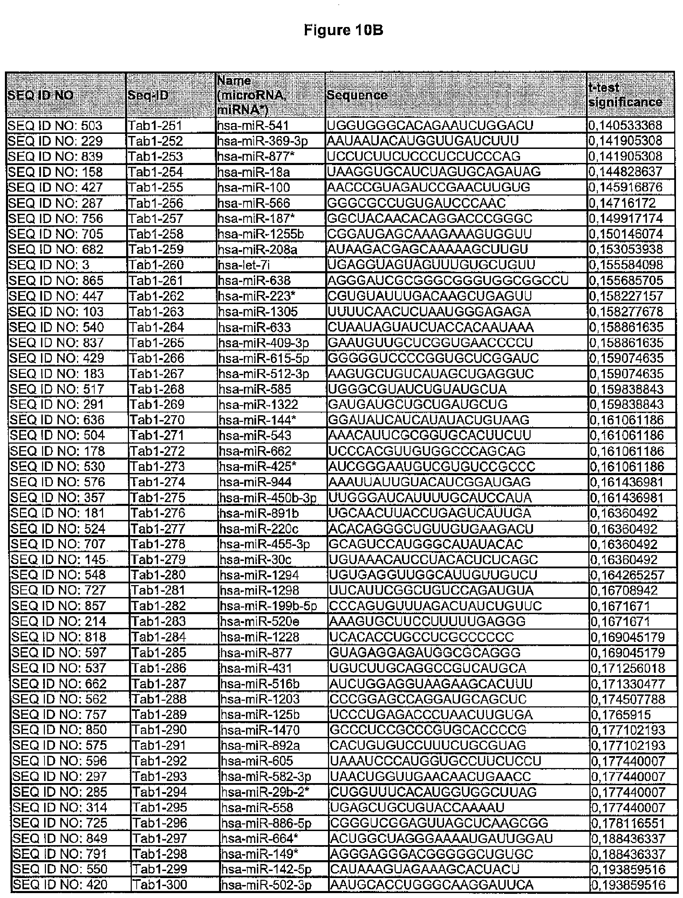

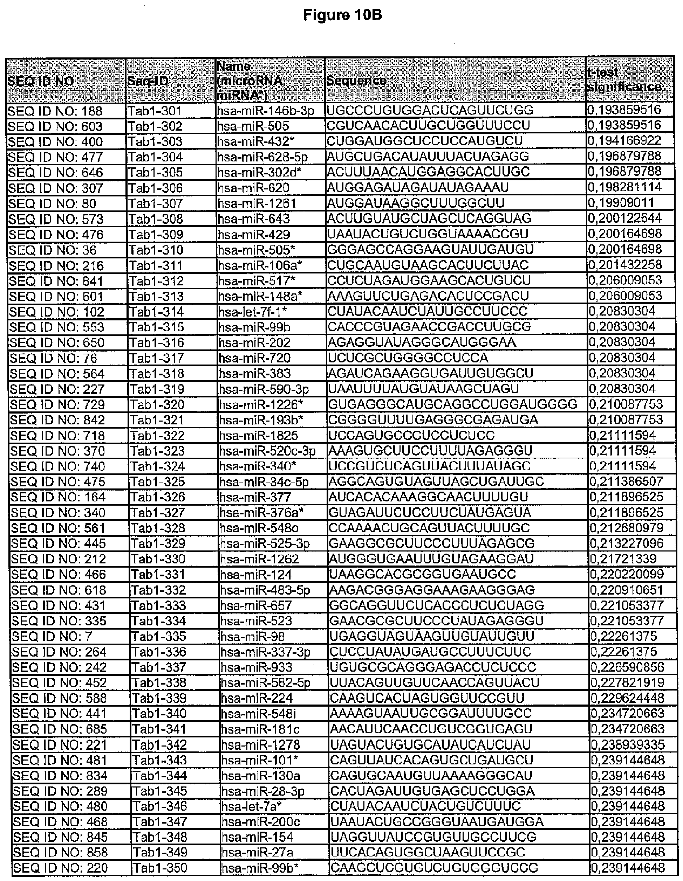

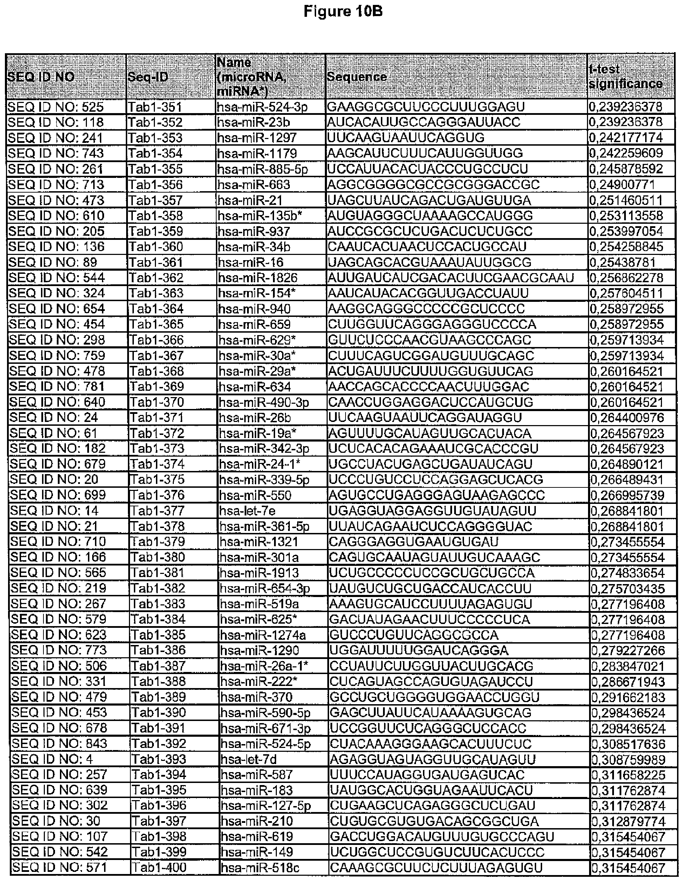

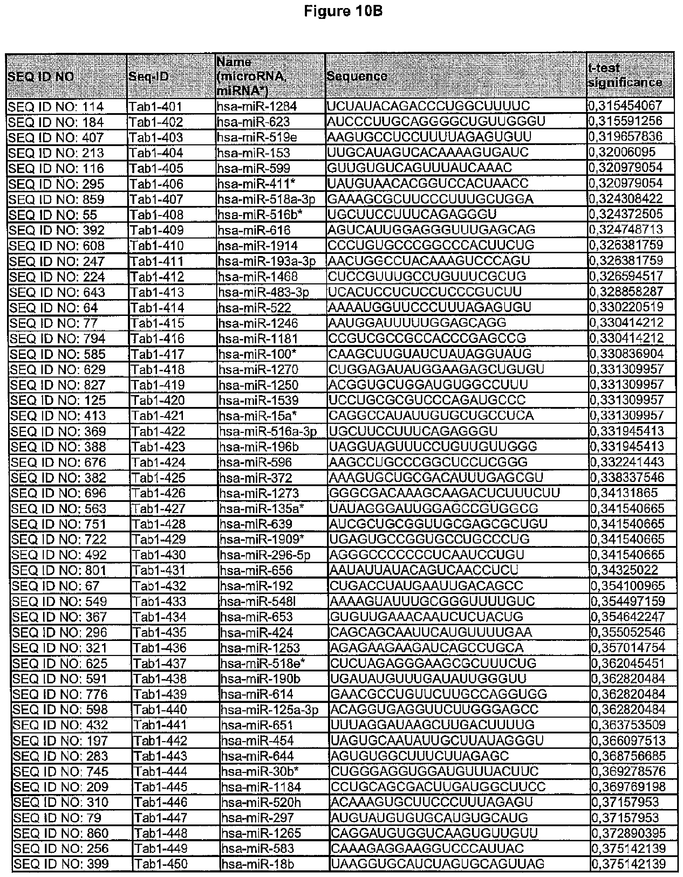

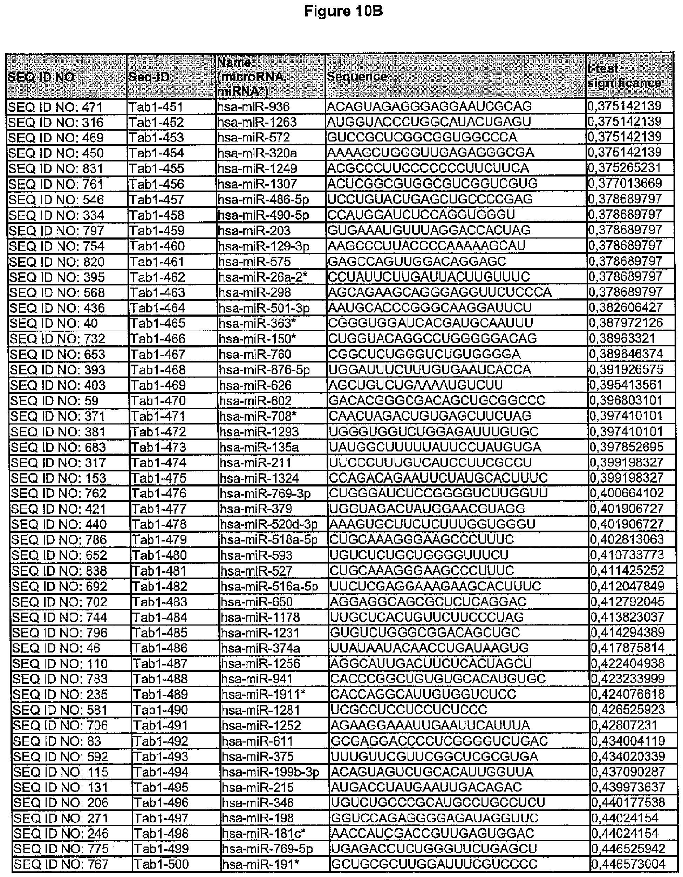

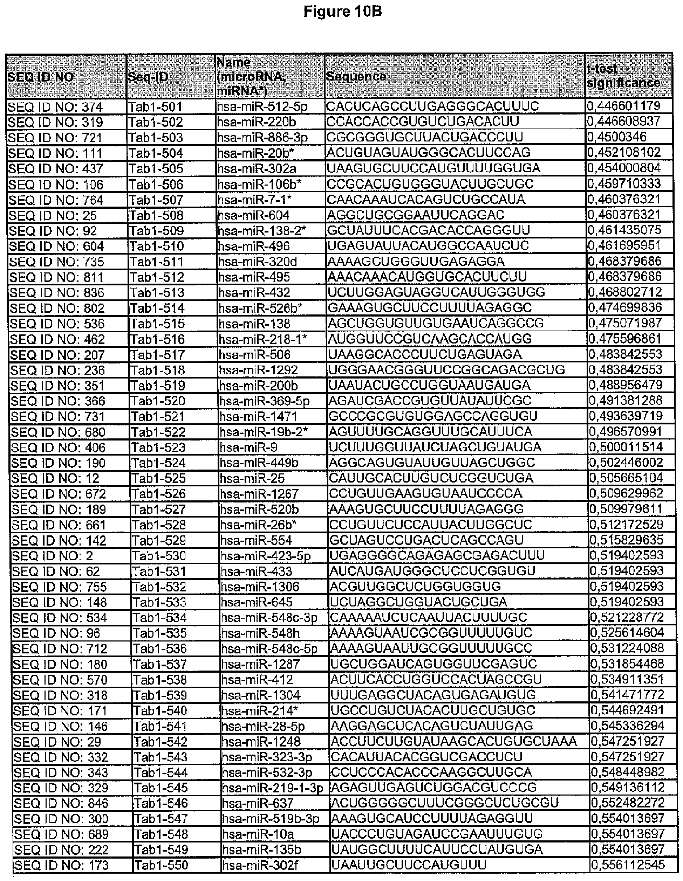

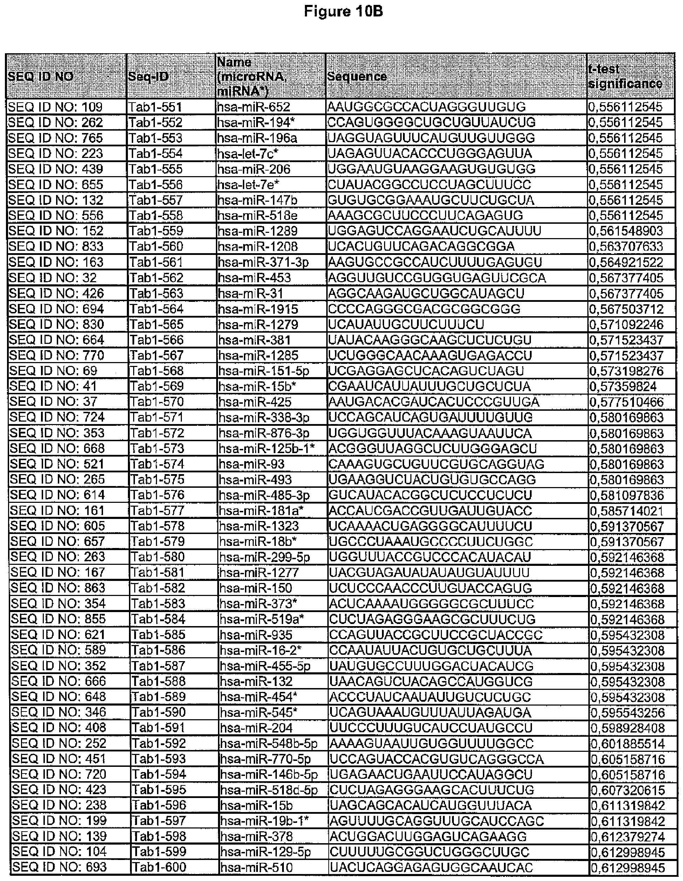

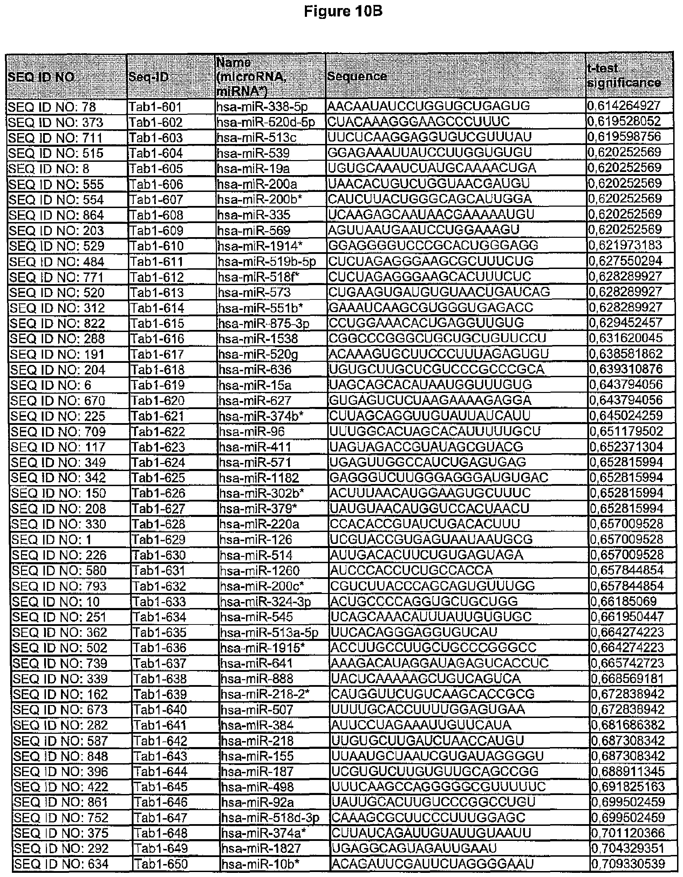

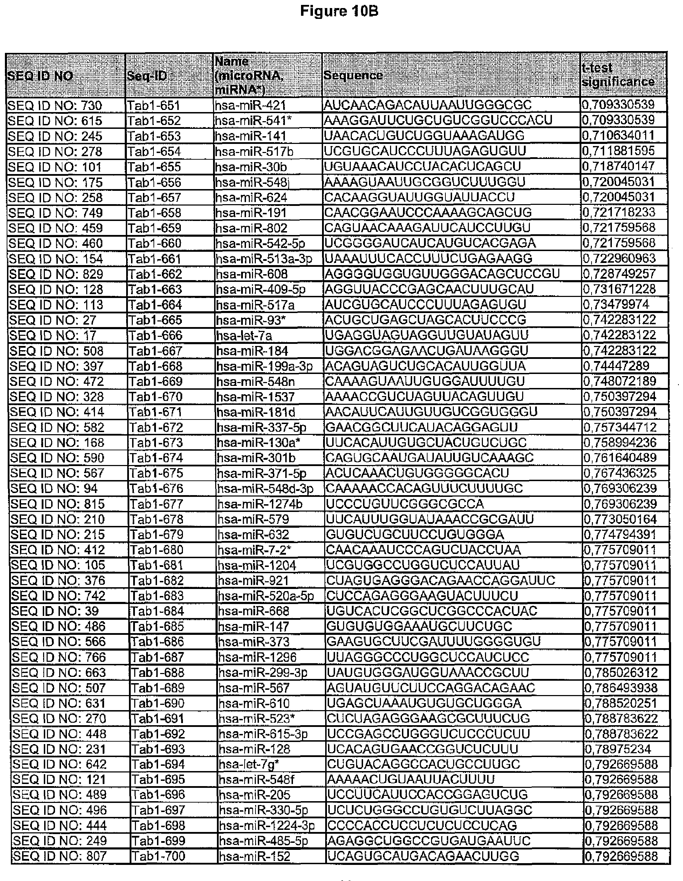

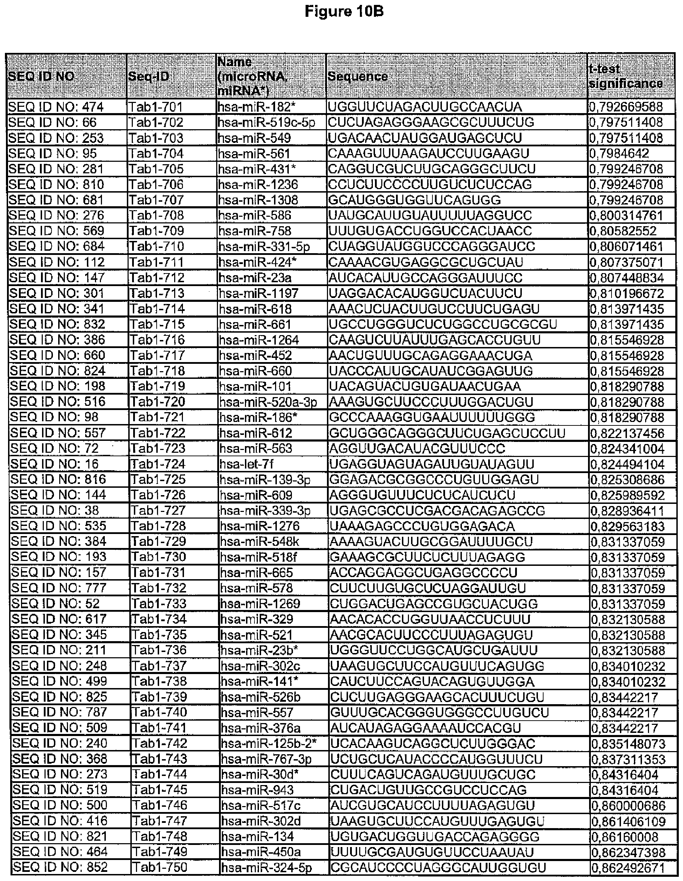

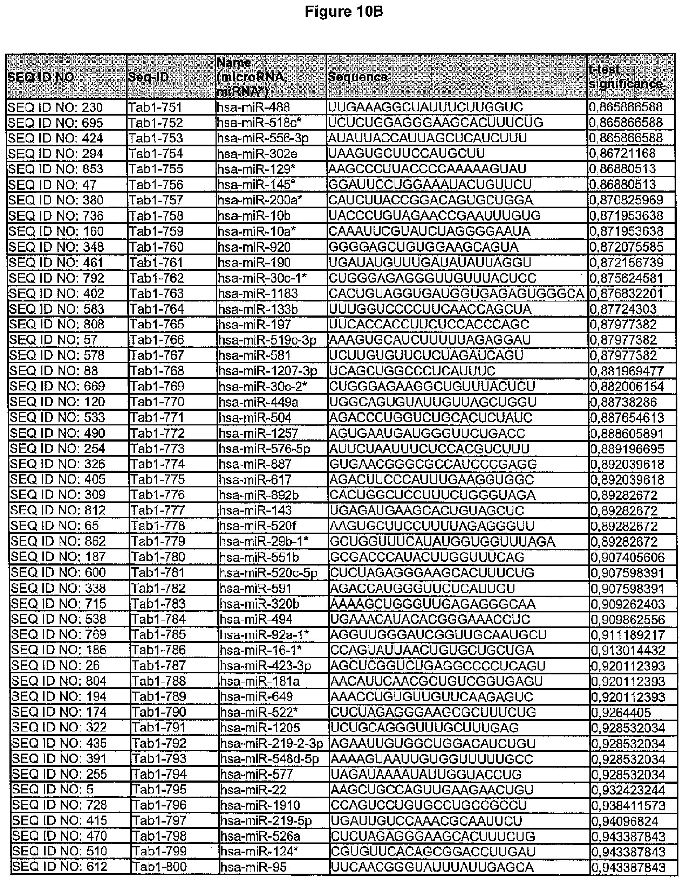

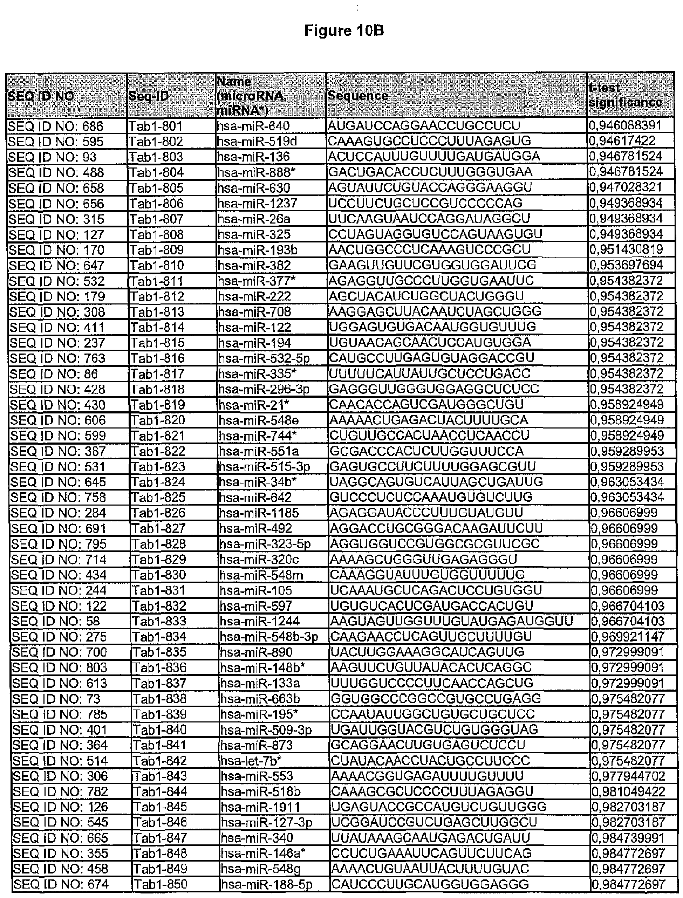

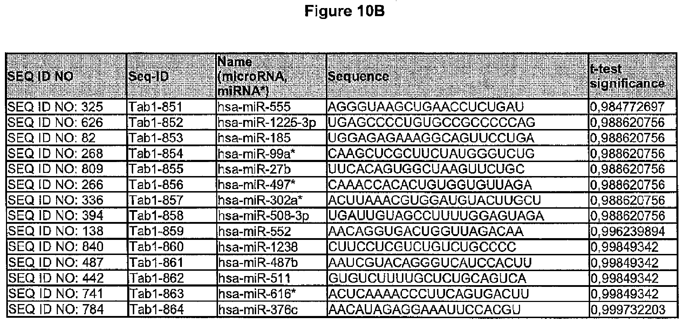

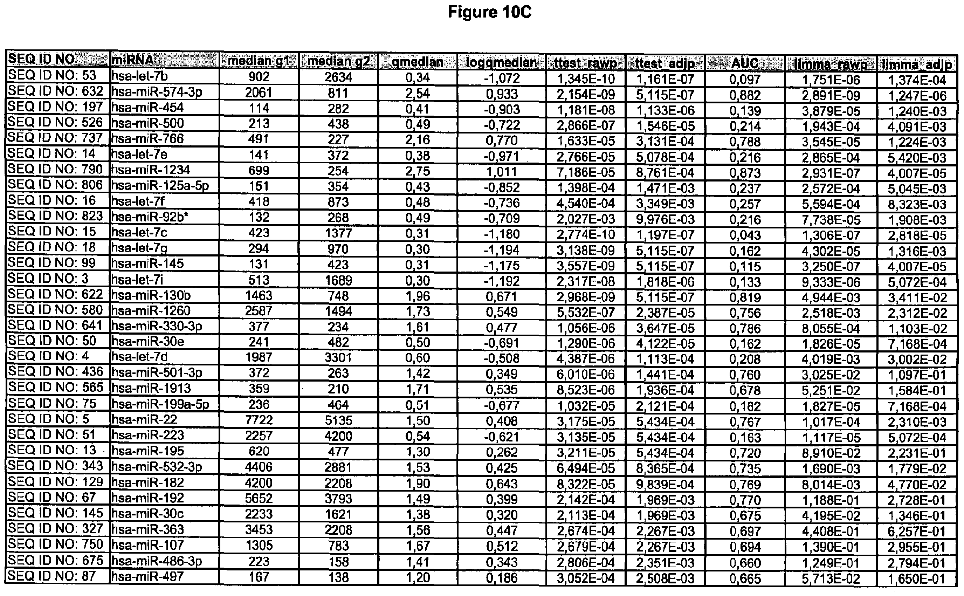

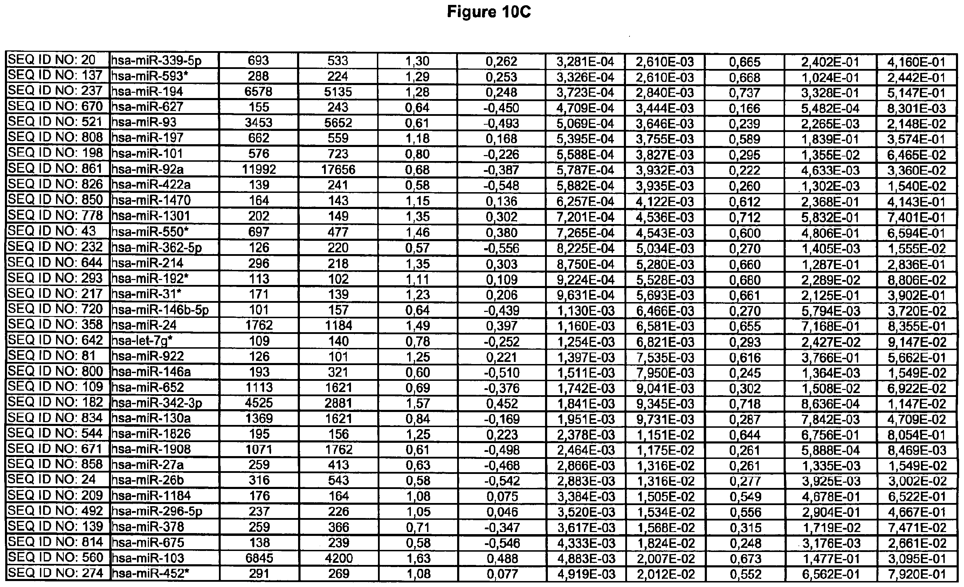

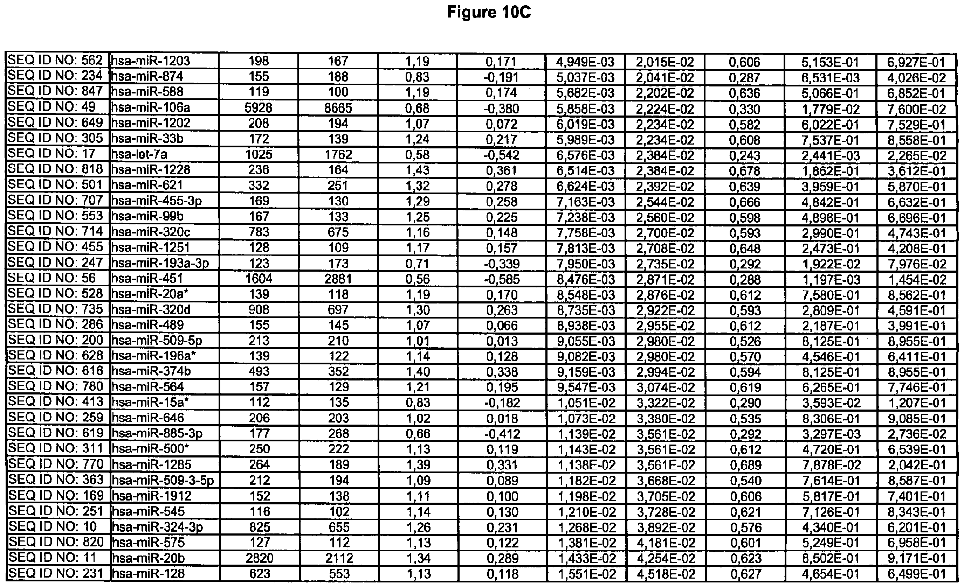

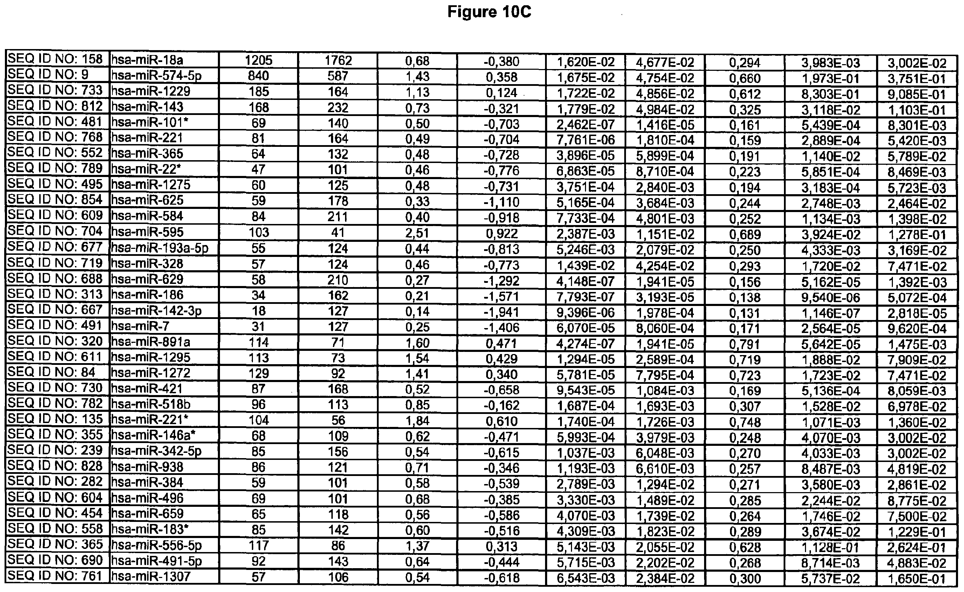

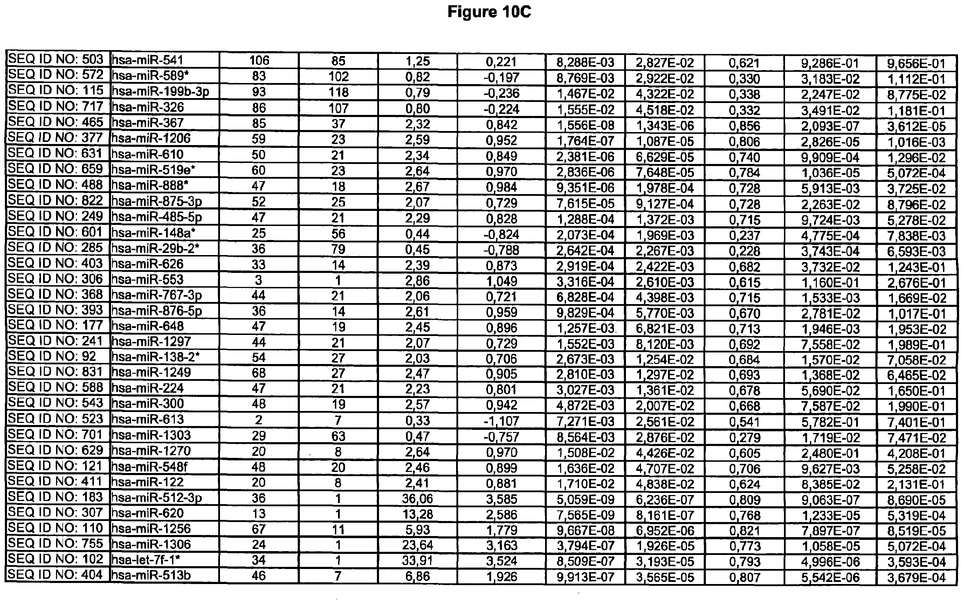

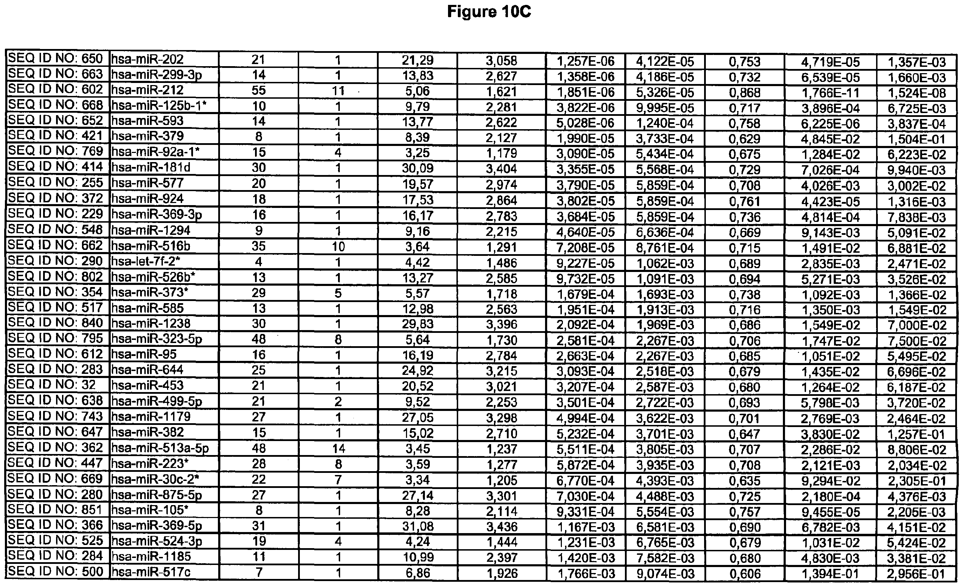

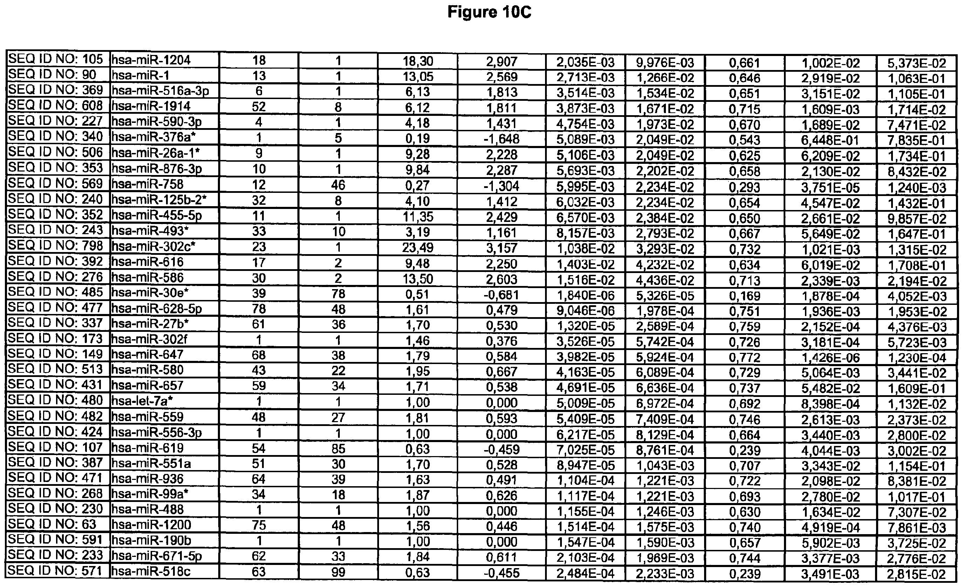

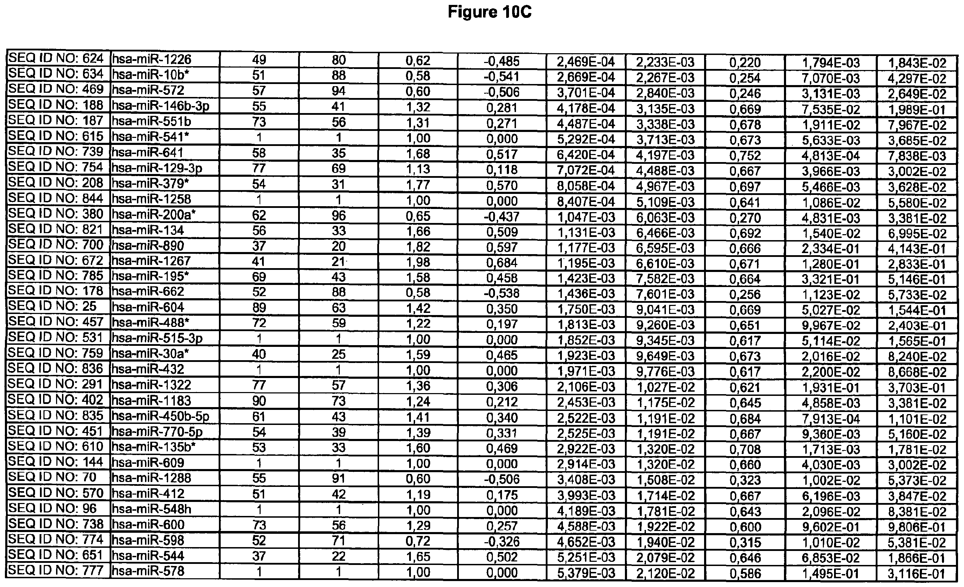

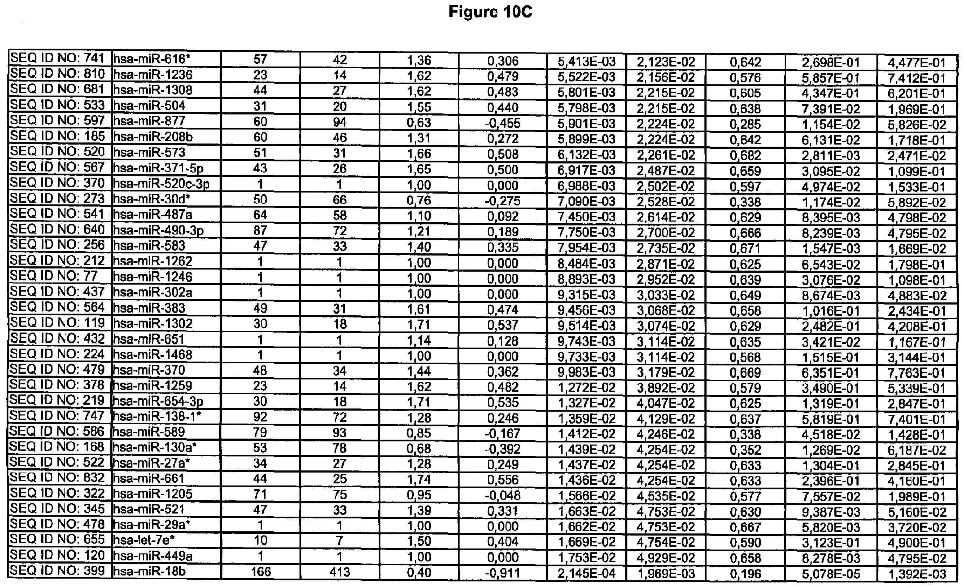

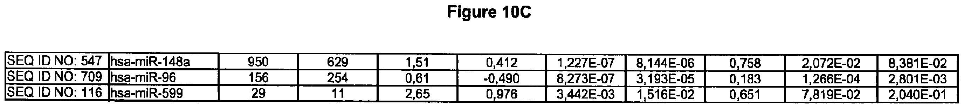

The inventors succeeded in developing a generally applicable approach to arrive at miRNA signatures that are correlated with a particular disease. The general work flow is depicted in Figure 9. In more detail, the following steps are accomplished:
1. miRNAs are extracted from a biological sample of a patient, preferably a blood or serum sample or a sample comprising erythrocytes, leukocytes or thrombocytes, using suitable kits / purification methods
2. The respective samples are measured using experimental techniques.
   These techniques include but are not restricted to:
   - Array based approaches
   - Real time quantitative polymerase chain reaction
   - Bead based assays (e.g. Luminex)
   - Sequencing
   - Next Generation Sequencing
   - Mass Spectroscopy
3. Mathematical approaches are applied to gather information on the value and the redundancy of single biomarkers. These methods include, but are not restricted to:
   - basic mathematic approaches (e.g. Fold Quotients, Signal to Noise ratios, Correlation)
   - statistical methods as hypothesis tests (e.g. t-test, Wilcoxon-Mann-Whitney test), the Area under the Receiver operator Characteristics Curve
   - Information Theory approaches, (e.g. the Mutual Information, Cross-entropy)
   - Probability theory (e.g. joint and conditional probabilities)
   - Combinations and modifications of the previously mentioned examples
4. The information collected in 3) are used to estimate for each biomarker the diagnostic content or value. Usually, however, this diagnostic value is too small to get a highly accurate diagnosis with accuracy rates, specificities and sensitivities beyond the 90% barrier.
   Please note that the diagnostic content for our miRNAs can be found in Figures 10A, 10B and 10C. This table includes the miRNAs with the sequences, the fold quotient, the mutual information and the significance value as computed by a t-test.
5. Thus statistical learning / machine learning / bioinformatics / computational approaches are applied to define subsets of biomarkers that are tailored for the detection of diseases. These techniques includes but are not restricted to
   - Wrapper subset selection techniques (e.g. forward step-wise, backward step-wise, combinatorial approaches, optimization approaches)
   - Filter subset selection methods (e.g. the methods mentioned in 3)
   - Principal Component Analysis
   - Combinations and modifications of such methods (e.g. hybrid approaches)
6. The diagnostic content of each detected set can be estimated by mathematical and/or computational techniques to define the diagnostic information content of subsets.
7. The subsets, detected in step 5, which may range from only a small number (at least two) to all measured biomarkers is then used to carry out a diagnosis. To this end, statistical learning / machine learning / bioinformatics / computational approaches are applied that include but are not restricted to any type of supervised or unsupervised analysis:
   - Classification techniques (e.g. naive Bayes, Linear Discriminant Analysis, Quadratic Discriminant Analysis Neural Nets, Tree based approaches, Support Vector Machines, Nearest Neighbour Approaches)
   - Regression techniques (e.g. linear Regression, Multiple Regression, logistic regression, probit regression, ordinal logistic regression ordinal Probit-Regression, Poisson Regression, negative binomial Regression, multinomial logistic Regression, truncated regression)
   - Clustering techniques (e.g. k-means clustering, hierarchical clustering, PCA)
   - Adaptations, extensions, and combinations of the previously mentioned approaches

The inventors surprisingly found out that the described approach yields in miRNA signatures that provide high diagnostic accuracy, specificity and sensitivity in the determination of multiple sclerosis or related disease states/clinical conditions.

According to a preferred embodiment of the invention, the disease to be determined is multiple sclerosis. Surprisingly, the inventors found out that miRNAs are differentially regulated in samples from MS patients as compared to health controls. A complete overview of all miRNAs that are found to be differentially regulated in blood samples of multiple sclerosis patients is provided in the tables shown in Figures 10A, 10B and 10C. In one embodiment, 193 miRNAs were found to be significantly deregulated in blood cells of MS patients as compared to controls (Fig. 10A). In a further embodiment - based on additional information - 165 miRNAs were found to be significantly deregulated in blood cells of MS patients as compared to controls (Fig. 10B). In a still further embodiment, 308 miRNAs were found to be significantly deregulated in blood cells of MS patients as compared to controls (Fig. 10C).

Preferably, the predetermined set of miRNAs for the diagnosis of multiple sclerosis comprises one or more nucleic acids selected from the deregulated miRNAs presented in the tables in Figures 10A, 10B or 10C. The predetermined set of miRNAs should preferably comprise at least 7, preferably at least 10, 15, 20 or 24 of the indicated nucleic acids. It is particularly preferred to include the 24, 20, 15, 10 or at least 7 of the first mentioned miRNAs according to their order in the tables in Figures 10A, 10B or 10C, preferably except hsa-miR-148a, hsa-miR-18b, hsa-miR-96, hsa-miR-96, hsa-miR-599, hsa-miR-493, hsa-miR-184, hsa-miR-193a.

Thus, preferably the predetermined set of miRNAs for the diagnosis of MS comprises one or more nucleic acids selected from the 24 most deregulated miRNAs hsa-miR-145, hsa-miR-186, hsa-miR-664, hsa-miR-584, hsa-miR-20b, hsa-miR-223, hsa-miR-422a, hsa-miR-142-3p, hsa-let-7c, hsa-miR-151-3p, hsa-miR-491-5p, hsa-miR-942, hsa-miR-361-3p, hsa-miR-22*, hsa-miR-140-5p, hsa-miR-216a, hsa-miR-1275, hsa-miR-367, hsa-miR-146a, hsa-miR-598, hsa-miR-613, hsa-miR-18a*, hsa-miR-302b, hsa-miR-501-5p. Preferably, the predetermined set of miRNAs comprises at least 7, preferably at least 10, 15, 20 or all of the above-indicated nucleic acids. Most preferably, the predetermined set of miRNAs comprises those miRNAs that were most significantly deregulated: hsa-miR-145, hsa-miR-186, hsa-miR-664, hsa-miR-584, hsa-miR-20b, hsa-miR-223, hsa-miR-422a, hsa-miR-142-3p, hsa-let-7c.

In another embodiment, the predetermined set of miRNAs for the diagnosis of MS comprises at least one preferred signature 1-84 as shown in Table 1. It should be noted that preferred diagnostic sets may also comprise one or more miRNAs of the miRNAs disclosed in Table 1 and any combination of the miRNAs together with one or more further diagnostically relevant miRNA from Figures 10A, 10B or 10C. Preferred predetermined sets of miRNA based on Table 1 comprise at least 3, 4, 5, 6, 7, 8, 9 or 10 miRNAs and up to 10, 15, or 20 or more miRNAs.

**Table 1**

| **Signature** | **Signature** | **SEQ-ID Nos** | **miRNA -identifiers** |
|---|---|---|---|
| 1 | A1 | SEQ ID NO: 53, SEQ ID NO: 632, SEQ ID NO: 526 | hsa-let-7b, hsa-miR-574-3p, hsa-miR-500 |
| 2 | A2 | SEQ ID NO: 526, SEQ ID NO: 790, SEQ ID NO: 806 | hsa-miR-500, hsa-miR-1234, hsa-miR-125a-5p |
| 3 | A3 | SEQ ID NO: 806, SEQ ID NO: 823, SEQ ID NO: 15 | hsa-miR-125a-5p, hsa-miR-92b*, hsa-let-7c |
| 4 | A4 | SEQ ID NO: 15, SEQ ID NO: 99, SEQ ID NO: 641 | hsa-let-7c, hsa-miR-145, hsa-miR-330-3p |
| 5 | A5 | SEQ ID NO: 641, SEQ ID NO: 50, SEQ ID NO: 75 | hsa-miR-330-3p, hsa-miR-30e, hsa-miR-199a-5p |
| 6 | A6 | SEQ ID NO: 75, SEQ ID NO: 51, SEQ ID NO: 13 | hsa-miR-199a-5p, hsa-miR-223, hsa-miR-195 |
| 7 | A7 | SEQ ID NO: 13, SEQ ID NO: 129, SEQ ID NO: 750 | hsa-miR-195, hsa-miR-182, hsa-miR-107 |
| 8 | A8 | SEQ ID NO: 750, SEQ ID NO: 87, SEQ ID NO: 137 | hsa-miR-107, hsa-miR-497, hsa-miR-593* |
| 9 | A9 | SEQ ID NO: 137, SEQ ID NO: 826, SEQ ID NO: 778 | hsa-miR-593*, hsa-miR-422a, hsa-miR-1301 |
| 10 | A10 | SEQ ID NO: 778, SEQ ID NO: 232, SEQ ID NO: 644 | hsa-miR-1301, hsa-miR-362-5p, hsa-miR-214 |
| 11 | A11 | SEQ ID NO: 644, SEQ ID NO: 217, SEQ ID NO: 358 | hsa-miR-214, hsa-miR-31*, hsa-miR-24 |
| 12 | A12 | SEQ ID NO: 358, SEQ ID NO: 800, SEQ ID NO: 671 | hsa-miR-24, hsa-miR-146a, hsa-miR-1908 |
| 13 | A13 | SEQ ID NO: 671, SEQ ID NO: 560, SEQ ID NO: 49 | hsa-miR-1908, hsa-miR-103, hsa-miR-106a |
| 14 | A14 | SEQ ID NO: 560, SEQ ID NO: 274, SEQ ID NO: 49 | hsa-miR-103, hsa-miR-452*, hsa-miR-106a |
| 15 | A15 | SEQ ID NO: 49, SEQ ID NO: 501, SEQ ID NO: 455 | hsa-miR-106a, hsa-miR-621, hsa-miR-1251 |
| 16 | A16 | SEQ ID NO: 53, SEQ ID NO: 632, SEQ ID NO: 526, SEQ ID NO: 790, SEQ ID NO: 806 | hsa-let-7b, hsa-miR-574-3p, hsa-miR-500, hsa-miR-1234, hsa-miR-125a-5p |
| 17 | A17 | SEQ ID NO: 526, SEQ ID NO: 790, SEQ ID NO: 806, SEQ ID NO: 823, SEQ ID NO: 15 | hsa-miR-500, hsa-miR-1234, hsa-miR-125a-5p, hsa-miR-92b*, hsa-let-7c |
| 18 | A18 | SEQ ID NO: 806, SEQ ID NO: 823, SEQ ID NO: 15, SEQ ID NO: 99, SEQ ID NO: 641 | hsa-miR-125a-5p, hsa-miR-92b*, hsa-let-7c, hsa-miR-145, hsa-miR-330-3p |
| 19 | A19 | SEQ ID NO: 823, SEQ ID NO: 15, SEQ ID NO: 99, SEQ ID NO: 641, SEQ ID NO: 50 | hsa-miR-92b*, hsa-let-7c, hsa-miR-145, hsa-miR-330-3p, hsa-miR-30e |
| 20 | A20 | SEQ ID NO: 99, SEQ ID NO: 641, SEQ ID NO: 50, SEQ ID NO: 75, SEQ ID NO: 51 | hsa-miR-145, hsa-miR-330-3p, hsa-miR-30e, hsa-miR-199a-5p, hsa-miR-223 |
| 21 | A21 | SEQ ID NO: 50, SEQ ID NO: 75, SEQ ID NO: 51, SEQ ID NO: 13, SEQ ID NO: 129 | hsa-miR-30e, hsa-miR-199a-5p, hsa-miR-223, hsa-miR-195, hsa-miR-182 |
| 22 | A22 | SEQ ID NO: 75, SEQ ID NO: 51, SEQ ID NO: 13, SEQ ID NO: 129, SEQ ID NO: 750, SEQ ID NO: 87 | hsa-miR-199a-5p, hsa-miR-223, hsa-miR-195, hsa-miR-182, hsa-miR-107, hsa-miR-497 |
| 23 | A23 | SEQ ID NO: 129, SEQ ID NO: 750, SEQ ID NO: 87, SEQ ID NO: 137, SEQ ID NO: 826, SEQ ID NO: 778 | hsa-miR-182, hsa-miR-107, hsa-miR-497, hsa-miR-593*, hsa-miR-422a, hsa-miR-1301 |
| 24 | A24 | SEQ ID NO: 137, SEQ ID NO: 826, SEQ ID NO: 778, SEQ ID NO: 232, SEQ ID NO: 644, SEQ ID NO: 217 | hsa-miR-593*, hsa-miR-422a, hsa-miR-1301, hsa-miR-362-5p, hsa-miR-214, hsa-miR-31* |
| 25 | A25 | SEQ ID NO: 232, SEQ ID NO: 644, SEQ ID NO: 217, SEQ ID NO: 358, SEQ ID NO: 800, SEQ ID NO: 671 | hsa-miR-362-5p, hsa-miR-214, hsa-miR-31*, hsa-miR-24, hsa-miR-146a, hsa-miR-1908 |
| 26 | A26 | SEQ ID NO: 358, SEQ ID NO: 800, SEQ ID NO: 671, SEQ ID NO: 814, SEQ ID NO: 560, SEQ ID NO: 274 | hsa-miR-24, hsa-miR-146a, hsa-miR-1908, hsa-miR-675, hsa-miR-103, hsa-miR-452* |
| 27 | A27 | SEQ ID NO: 814, SEQ ID NO: 560, SEQ ID NO: 274, SEQ ID NO: 49, SEQ ID NO: 501, SEQ ID NO: 455 | hsa-miR-675, hsa-miR-103, hsa-miR-452*, hsa-miR-106a, hsa-miR-621, hsa-miR-1251 |
| 28 | A28 | SEQ ID NO: 49, SEQ ID NO: 501, SEQ ID NO: 455, SEQ ID NO: 528, SEQ ID NO: 259, SEQ ID NO: 619 | hsa-miR-106a, hsa-miR-621, hsa-miR-1251, hsa-miR-20a*, hsa-miR-646, hsa-miR-885-3p |
| 29 | A29 | SEQ ID NO: 528, SEQ ID NO: 259, SEQ ID NO: 619, SEQ ID NO: 363, SEQ ID NO: 169, SEQ ID NO: 11 | hsa-miR-20a*, hsa-miR-646, hsa-miR-885-3p, hsa-miR-509-3-5p, hsa-miR-1912, hsa-miR-20b |
| 30 | A30 | SEQ ID NO: 363, SEQ ID NO: 169, SEQ ID NO: 11, SEQ ID NO: 9, SEQ ID NO: 768, SEQ ID NO: 552 | hsa-miR-509-3-5p, hsa-miR-1912, hsa-miR-20b, hsa-miR-574-5p, hsa-miR-221, hsa-miR-365 |
| 31 | A31 | SEQ ID NO: 53, SEQ ID NO: 632, SEQ ID NO: 526, SEQ ID NO: 790, SEQ ID NO: 806, SEQ ID NO: 823, SEQ ID NO: 15, SEQ ID NO: 99, SEQ ID NO: 641, SEQ ID NO: 50 | hsa-let-7b, hsa-miR-574-3p, hsa-miR-500, hsa-miR-1234, hsa-miR-125a-5p, hsa-miR-92b*, hsa-let-7c, hsa-miR-145, hsa-miR-330-3p, hsa-miR-30e |
| 32 | A32 | SEQ ID NO: 823, SEQ ID NO: 15, SEQ ID NO: 99, SEQ ID NO: 641, SEQ ID NO: 50, SEQ ID NO: 75, SEQ ID NO: 51, SEQ ID NO: 13, SEQ ID NO: 129, SEQ ID NO: 750, SEQ ID NO: 87 | hsa-miR-92b*, hsa-let-7c, hsa-miR-145, hsa-miR-330-3p, hsa-miR-30e, hsa-miR-199a-5p, hsa-miR-223, hsa-miR-195, hsa-miR-182, hsa-miR-107, hsa-miR-497 |
| 33 | A33 | SEQ ID NO: 51, SEQ ID NO: 13, SEQ ID NO: 129, SEQ ID NO: 750, SEQ ID NO: 87, SEQ ID NO: 137, SEQ ID NO: 826, SEQ ID NO: 778, SEQ ID NO: 232, SEQ ID NO: 644 | hsa-miR-223, hsa-miR-195, hsa-miR-182, hsa-miR-107, hsa-miR-497, hsa-miR-593*, hsa-miR-422a, hsa-miR-1301, hsa-miR-362-5p, hsa-miR-214 |
| 34 | A34 | SEQ ID NO: 137, SEQ ID NO: 826, SEQ ID NO: 778, SEQ ID NO: 232, SEQ ID NO: 644, SEQ ID NO: 217, SEQ ID NO: 358, SEQ ID NO: 800, SEQ ID NO: 671, SEQ ID NO: 814 | hsa-miR-593*, hsa-miR-422a, hsa-miR-1301, hsa-miR-362-5p, hsa-miR-214, hsa-miR-31*, hsa-miR-24, hsa-miR-146a, hsa-miR-1908, hsa-miR-675 |
| 35 | A35 | SEQ ID NO: 217, SEQ ID NO: 358, SEQ ID NO: 800, SEQ ID NO: 671, SEQ ID NO: 814, SEQ ID NO: 560, SEQ ID NO: 274, SEQ ID NO: 49, SEQ ID NO: 501, SEQ ID NO: 455 | hsa-miR-31*, hsa-miR-24, hsa-miR-146a, hsa-miR-1908, hsa-miR-675, hsa-miR-103, hsa-miR-452*, hsa-miR-106a, hsa-miR-621, hsa-miR-1251 |
| 36 | A36 | SEQ ID NO: 560, SEQ ID NO: 274, SEQ ID NO: 49, SEQ ID NO: 501, SEQ ID NO: 455, SEQ ID NO: 528, SEQ ID NO: 259, SEQ ID NO: 619, SEQ ID NO: 363, SEQ ID NO: 169 | hsa-miR-103, hsa-miR-452*, hsa-miR-106a, hsa-miR-621, hsa-miR-1251, hsa-miR-20a*, hsa-miR-646, hsa-miR-885-3p, hsa-miR-509-3-5p, hsa-miR-1912 |
| 37 | A37 | SEQ ID NO: 528, SEQ ID NO: 259, SEQ ID NO: 619, SEQ ID NO: 363, SEQ ID NO: 169, SEQ ID NO: 11, SEQ ID NO: 9, SEQ ID NO: 768, SEQ ID NO: 552, SEQ ID NO: 789 | hsa-miR-20a*, hsa-miR-646, hsa-miR-885-3p, hsa-miR-509-3-5p, hsa-miR-1912, hsa-miR-20b, hsa-miR-574-5p, hsa-miR-221, hsa-miR-365, hsa-miR-22* |
| 38 | A38 | SEQ ID NO: 11, SEQ ID NO: 9, SEQ ID NO: 768, SEQ ID NO: 552, SEQ ID NO: 789, SEQ ID NO: 495, SEQ ID NO: 854, SEQ ID NO: 609, SEQ ID NO: 719, SEQ ID NO: 688 | hsa-miR-20b, hsa-miR-574-5p, hsa-miR-221, hsa-miR-365, hsa-miR-22*, hsa-miR-1275, hsa-miR-625, hsa-miR-584, hsa-miR-328, hsa-miR-629 |
| 39 | A39 | SEQ ID NO: 495, SEQ ID NO: 854, SEQ ID NO: 609, SEQ ID NO: 719, SEQ ID NO: 688, SEQ ID NO: 313, SEQ ID NO: 667, SEQ ID NO: 491, SEQ ID NO: 320, SEQ ID NO: 84 | hsa-miR-1275, hsa-miR-625, hsa-miR-584, hsa-miR-328, hsa-miR-629, hsa-miR-186, hsa-miR-142-3p, hsa-miR-7, hsa-miR-891a, hsa-miR-1272 |
| 40 | A40 | SEQ ID NO: 53, SEQ ID NO: 526, SEQ ID NO: 806, SEQ ID NO: 15, SEQ ID NO: 641 | hsa-let-7b, hsa-miR-500, hsa-miR-125a-5p, hsa-let-7c, hsa-miR-330-3p |
| 41 | A41 | SEQ ID NO: 790, SEQ ID NO: 823, SEQ ID NO: 99, SEQ ID NO: 50, SEQ ID NO: 51 | hsa-miR-1234, hsa-miR-92b*, hsa-miR-145, hsa-miR-30e, hsa-miR-223 |
| 42 | A42 | SEQ ID NO: 99, SEQ ID NO: 50, SEQ ID NO: 51, SEQ ID NO: 129, SEQ ID NO: 87 | hsa-miR-145, hsa-miR-30e, hsa-miR-223, hsa-miR-182, hsa-miR-497 |
| 43 | B1 | SEQ ID NO: 53, SEQ ID NO: 632, SEQ ID NO: 197 | hsa-let-7b, hsa-miR-574-3p, hsa-miR-454 |
| 44 | B2 | SEQ ID NO: 197, SEQ ID NO: 526, SEQ ID NO: 737 | hsa-miR-454, hsa-miR-500, hsa-miR-766 |
| 45 | B3 | SEQ ID NO: 737, SEQ ID NO: 14, SEQ ID NO: 790 | hsa-miR-766, hsa-let-7e, hsa-miR-1234 |
| 46 | B4 | SEQ ID NO: 790, SEQ ID NO: 806, SEQ ID NO: 16 | hsa-miR-1234, hsa-miR-125a-5p, hsa-let-7f |
| 47 | B5 | SEQ ID NO: 16, SEQ ID NO: 823, SEQ ID NO: 15 | hsa-let-7f, hsa-miR-92b*, hsa-let-7c |
| 48 | B6 | SEQ ID NO: 15, SEQ ID NO: 18, SEQ ID NO: 99 | hsa-let-7c, hsa-let-7g, hsa-miR-145 |
| 49 | B7 | SEQ ID NO: 99, SEQ ID NO: 3, SEQ ID NO: 622 | hsa-miR-145, hsa-let-7i, hsa-miR-130b |
| 50 | B8 | SEQ ID NO: 622, SEQ ID NO: 580, SEQ ID NO: 641 | hsa-miR-130b, hsa-miR-1260, hsa-miR-330-3p |
| 51 | B9 | SEQ ID NO: 641, SEQ ID NO: 50, SEQ ID NO: 4 | hsa-miR-330-3p, hsa-miR-30e, hsa-let-7d |
| 52 | B10 | SEQ ID NO: 4, SEQ ID NO: 436, SEQ ID NO: 565 | hsa-let-7d, hsa-miR-501-3p, hsa-miR-1913 |
| 53 | B11 | SEQ ID NO: 565, SEQ ID NO: 75, SEQ ID NO: 5 | hsa-miR-1913, hsa-miR-199a-5p, hsa-miR-22 |
| 54 | B12 | SEQ ID NO: 5, SEQ ID NO: 51, SEQ ID NO: 13 | hsa-miR-22, hsa-miR-223, hsa-miR-195 |
| 55 | B13 | SEQ ID NO: 13, SEQ ID NO: 129, SEQ ID NO: 145 | hsa-miR-195, hsa-miR-182, hsa-miR-30c |
| 56 | B14 | SEQ ID NO: 129, SEQ ID NO: 67, SEQ ID NO: 145 | hsa-miR-182, hsa-miR-192, hsa-miR-30c |
| 57 | B15 | SEQ ID NO: 145, SEQ ID NO: 327, SEQ ID NO: 750 | hsa-miR-30c, hsa-miR-363, hsa-miR-107 |
| 58 | B16 | SEQ ID NO: 53, SEQ ID NO: 632, SEQ ID NO: 197, SEQ ID NO: 526, SEQ ID NO: 737 | hsa-tet-7b, hsa-miR-574-3p, hsa-miR-454, hsa-miR-500, hsa-miR-766 |
| 59 | B17 | SEQ ID NO: 197, SEQ ID NO: 526, SEQ ID NO: 737, SEQ ID NO: 14, SEQ ID NO: 790 | hsa-miR-454, hsa-miR-500, hsa-miR-766, hsa-let-7e, hsa-miR-1234 |
| 60 | B18 | SEQ ID NO: 737, SEQ ID NO: 14, SEQ ID NO: 790, SEQ ID NO: 806, SEQ ID NO: 16 | hsa-miR-766, hsa-let-7e, hsa-miR-1234, hsa-miR-125a-5p, hsa-let-7f |
| 61 | B19 | SEQ ID NO: 14, SEQ ID NO: 790, SEQ ID NO: 806, SEQ ID NO: 16, SEQ ID NO: 823 | hsa-let-7e, hsa-miR-1234, hsa-miR-125a-5p, hsa-let-7f, hsa-miR-92b* |
| 62 | B20 | SEQ ID NO: 806, SEQ ID NO: 16, SEQ ID NO: 823, SEQ ID NO: 15, SEQ ID NO: 18 | hsa-miR-125a-5p, hsa-let-7f, hsa-miR-92b*, hsa-let-7c, hsa-let-7g |
| 63 | B21 | SEQ ID NO: 823, SEQ ID NO: 15, SEQ ID NO: 18, SEQ ID NO: 99, SEQ ID NO: 3 | hsa-miR-92b*, hsa-let-7c, hsa-let-7g, hsa-miR-145, hsa-let-7i |
| 64 | B22 | SEQ ID NO: 15, SEQ ID NO: 18, SEQ ID NO: 99, SEQ ID NO: 3, SEQ ID NO: 622, SEQ ID NO: 580 | hsa-let-7c, hsa-let-7g, hsa-miR-145, hsa-let-7i, hsa-miR-130b, hsa-miR-1260 |
| 65 | B23 | SEQ ID NO: 3, SEQ ID NO: 622, SEQ ID NO: 580, SEQ ID NO: 641, SEQ ID NO: 50, SEQ ID NO: 4 | hsa-let-7i, hsa-miR-130b, hsa-miR-1260, hsa-miR-330-3p, hsa-miR-30e, hsa-let-7d |
| 66 | B24 | SEQ ID NO: 641, SEQ ID NO: 50, SEQ ID NO: 4, SEQ ID NO: 436, SEQ ID NO: 565, SEQ ID NO: 75 | hsa-miR-330-3p, hsa-miR-30e, hsa-let-7d, hsa-miR-501-3p, hsa-miR-1913, hsa-miR-199a-5p |
| 67 | B25 | SEQ ID NO: 436, SEQ ID NO: 565, SEQ ID NO: 75, SEQ ID NO: 5, SEQ ID NO: 51, SEQ ID NO: 13 | hsa-miR-501-3p, hsa-miR-1913, hsa-miR-199a-5p, hsa-miR-22, hsa-miR-223, hsa-miR-195 |
| 68 | B26 | SEQ ID NO: 5, SEQ ID NO: 51, SEQ ID NO: 13, SEQ ID NO: 343, SEQ ID NO: 129, SEQ ID NO: 67 | hsa-miR-22, hsa-miR-223, hsa-miR-195, hsa-miR-532-3p, hsa-miR-182, hsa-miR-192 |
| 69 | B27 | SEQ ID NO: 343, SEQ ID NO: 129, SEQ ID NO: 67, SEQ ID NO: 145, SEQ ID NO: 327, SEQ ID NO: 750 | hsa-miR-532-3p, hsa-miR-182, hsa-miR-192, hsa-miR-30c, hsa-miR-363, hsa-miR-107 |
| 70 | B28 | SEQ ID NO: 145, SEQ ID NO: 327, SEQ ID NO: 750, SEQ ID NO: 675, SEQ ID NO: 87, SEQ ID NO: 20 | hsa-miR-30c, hsa-miR-363, hsa-miR-107, hsa-miR-486-3p, hsa-miR-497, hsa-miR-339-5p |
| 71 | B29 | SEQ ID NO: 675, SEQ ID NO: 87, SEQ ID NO: 20, SEQ ID NO: 137, SEQ ID NO: 237, SEQ ID NO: 670 | hsa-miR-486-3p, hsa-miR-497, hsa-miR-339-5p, hsa-miR-593*, hsa-miR-194, hsa-miR-627 |
| 72 | B30 | SEQ ID NO: 137, SEQ ID NO: 237, SEQ ID NO: 670, SEQ ID NO: 521, SEQ ID NO: 808, SEQ ID NO: 198 | hsa-miR-593*, hsa-miR-194, hsa-miR-627, hsa-miR-93, hsa-miR-197, hsa-miR-101 |
| 73 | B31 | SEQ ID NO: 53, SEQ ID NO: 632, SEQ ID NO: 197, SEQ ID NO: 526, SEQ ID NO: 737, SEQ ID NO: 14, SEQ ID NO: 790, SEQ ID NO: 806, SEQ ID NO: 16, SEQ ID NO: 823 | hsa-let-7b, hsa-miR-574-3p, hsa-miR-454, hsa-miR-500, hsa-miR-766, hsa-let-7e, hsa-miR-1234, hsa-miR-125a-5p, hsa-let-7f, hsa-miR-92b* |
| 74 | B32 | SEQ ID NO: 14, SEQ ID NO: 790, SEQ ID NO: 806, SEQ ID NO: 16, SEQ ID NO: 823, SEQ ID NO: 15, SEQ ID NO: 18, SEQ ID NO: 99, SEQ ID NO: 3, SEQ ID NO: 622, SEQ ID NO: 580 | hsa-let-7e, hsa-miR-1234, hsa-miR-125a-5p, hsa-let-7f, hsa-miR-92b*, hsa-let-7c, hsa-let-7g, hsa-miR-145, hsa-let-7i, hsa-miR-130b, hsa-miR-1260 |
| 75 | B33 | SEQ ID NO: 18, SEQ ID NO: 99, SEQ ID NO: 3, SEQ ID NO: 622, SEQ ID NO: 580, SEQ ID NO: 641, SEQ ID NO: 50, SEQ ID NO: 4, SEQ ID NO: 436, SEQ ID NO: 565 | hsa-let-7g, hsa-miR-145, hsa-let-7i, hsa-miR-130b, hsa-miR-1260, hsa-miR-330-3p, hsa-miR-30e, hsa-let-7d, hsa-miR-501-3p, hsa-miR-1913 |
| 76 | B34 | SEQ ID NO: 641, SEQ ID NO: 50, SEQ ID NO: 4, SEQ ID NO: 436, SEQ ID NO: 565, SEQ ID NO: 75, SEQ ID NO: 5, SEQ ID NO: 51, SEQ ID NO: 13, SEQ ID NO: 343 | hsa-miR-330-3p, hsa-miR-30e, hsa-let-7d, hsa-miR-501-3p, hsa-miR-1913, hsa-miR-199a-5p, hsa-miR-22, hsa-miR-223, hsa-miR-195, hsa-miR-532-3p |
| 77 | B35 | SEQ ID NO: 75, SEQ ID NO: 5, SEQ ID NO: 51, SEQ ID NO: 13, SEQ ID NO: 343, SEQ ID NO: 129, SEQ ID NO: 67, SEQ ID NO: 145, SEQ ID NO: 327, SEQ ID NO: 750 | hsa-miR-199a-5p, hsa-miR-22, hsa-miR-223, hsa-miR-195, hsa-miR-532-3p, hsa-miR-182, hsa-miR-192, hsa-miR-30c, hsa-miR-363, hsa-miR-107 |
| 78 | B36 | SEQ ID NO: 129, SEQ ID NO: 67, SEQ ID NO: 145, SEQ ID NO: 327, SEQ ID NO: 750, SEQ ID NO: 675, SEQ ID NO: 87, SEQ ID NO: 20, SEQ ID NO: 137, SEQ ID NO: 237 | hsa-miR-182, hsa-miR-192, hsa-miR-30c, hsa-miR-363, hsa-miR-107, hsa-miR-48fi-3p, hsa-miR-497, hsa-miR-339-5p, hsa-miR-593*, hsa-miR-194 |
| 79 | B37 | SEQ ID NO: 675, SEQ ID NO: 87, SEQ ID NO: 20, SEQ ID NO: 137, SEQ ID NO: 237, SEQ ID NO: 670, SEQ ID NO: 521, SEQ ID NO: 808, SEQ ID NO: 198, SEQ ID NO: 861 | hsa-miR-486-3p, hsa-miR-497, hsa-miR-339-5p, hsa-miR-593*, hsa-miR-194, hsa-miR-627, hsa-miR-93, hsa-miR-197, hsa-miR-101, hsa-miR-92a |
| 80 | B38 | SEQ ID NO: 670, SEQ ID NO: 521, SEQ ID NO: 808, SEQ ID NO: 198, SEQ ID NO: 861, SEQ ID NO: 826, SEQ ID NO: 850, SEQ ID NO: 778, SEQ ID NO: 43, SEQ ID NO: 232 | hsa-miR-627, hsa-miR-93, hsa-miR-197, hsa-miR-101, hsa-miR-92a, hsa-miR-422a, hsa-miR-1470, hsa-miR-1301, hsa-miR-550*, hsa-miR-362-5p |
| 81 | B39 | SEQ ID NO: 826, SEQ ID NO: 850, SEQ ID NO: 778, SEQ ID NO: 43, SEQ ID NO: 232, SEQ ID NO: 644, SEQ ID NO: 293, SEQ ID NO: 217, SEQ ID NO: 720, SEQ ID NO: 358 | hsa-miR-422a, hsa-miR-1470, hsa-miR-1301, hsa-miR-550*, hsa-miR-362-5p, hsa-miR-214, hsa-miR-192*, hsa-miR-31*, hsa-miR-146b-5p, hsa-miR-24 |
| 82 | B40 | SEQ ID NO: 53, SEQ ID NO, 197, SEQ ID NO: 737, SEQ ID NO: 790, SEQ ID NO: 16 | hsa-let-7b, hsa-miR-454, hsa-miR-766, hsa-miR-1234, hsa-let-7f |
| 83 | B41 | SEQ ID NO: 526, SEQ ID NO: 14, SEQ ID NO: 806, SEQ ID NO: 823, SEQ ID NO: 18 | hsa-miR-500, hsa-let-7e, hsa-miR-125a-5p, hsa-miR-92b*, hsa-let-7g |
| 84 | B42 | SEQ ID NO: 806, SEQ ID NO: 823, SEQ ID NO: 18, SEQ ID NO: 3, SEQ ID NO: 580 | hsa-miR-125a-5p, hsa-miR-92b*, hsa-let-7g, hsa-let-7i, hsa-miR-1260 |

Another embodiment of the present invention is a kit for diagnosing a disease, comprising means for determining an expression profile of a predetermined set of miRNAs in a biological sample, in particular in a blood, plasma, and/or serum sample including whole blood, plasma, serum or fractions thereof, or in a sample comprising peripheral blood mononuclear cells, erythrocytes, leukocytes and/or thrombocytes. Preferably, one or more reference expression profiles or a relevant reference which may be a mathematical function or algorithm - which allows for the diagnosis of the disease - are also provided which show the expression profile of the same set of miRNAs in the same type of biological sample, in particular in a blood and/or serum sample, obtained from one or more healthy subjects. A comparison to said reference expression profile(s) or relevant reference incl. a mathematical function or algorithm allows for the diagnosis of the disease.

The kit is preferably a test kit for detecting a predetermined set of miRNAs in sample by nucleic acid hybridisation and optionally amplification such as PCR or RT-PCR. The kit preferably comprises probes and/or primers and enzymes and reagents, including also reagent for cDNA-synthesis from miRNAs prior to real-time PCR for detecting a predetermined set of miRNAs. Further, the kit may comprise enzymes and reagents, e.g. for cDNA synthesis from miRNAs prior to RT-PCR.

A preferred embodiment of the present invention is a kit for diagnosing multiple sclerosis, comprising means for determining the expression profile of one or more relevant miRNAs presented in the table in Figure 10A, 10B or 10C as described above, preferably one or more miRNAs selected from the group consisting of hsa-miR-145, hsa-miR-186, hsa-miR-664, hsa-miR-584, hsa-miR-20b, hsa-miR-223, hsa-miR-422a, hsa-miR-142-3p, hsa-let-7c, hsa-miR-151-3p, hsa-miR-491-5p, hsa-miR-942, hsa-miR-361-3p, hsa-miR-22*, hsa-miR-140-5p, hsa-miR-216a, hsa-miR-1275, hsa-miR-367, hsa-miR-146a, hsa-miR-598, hsa-miR-613, hsa-miR-18a*, hsa-miR-302b, hsa-miR-501-5p.

In a preferred embodiment the kit comprises means for determining at least seven, preferably at least 10, 15, 20 or all of the indicated miRNAs. It is particularly preferred to include means for determining the 24, 20, 15, 10 or at least 7 first mentioned miRNAs in the order of their diagnostic significance as represented by their order in the table in Figure 10A, 10B, or 10C. Further, the kit may comprise means for determining the expression profile of a predetermined set of miRNAs based on Table 1 as described above. The kit for diagnosing MS is particularly suitable for diagnosing MS in a blood, plasma, and/or serum sample or in a sample comprising peripheral blood mononuclear cells, erythrocytes, leukocytes and/or thrombocytes.

The means for determining a predetermined set of miRNAs may for example comprise a microarray comprising miRNA-specific oligonucleotide probes. In a preferred embodiment, the microarray comprises miRNA-specific oligonucleotide probes for one or more miRNAs oligonucleotide probes for one or more miRNAs. Depending on the intended use of the microarray in the diagnostic of a particular disease, probes for different miRNAs may be included.

A microarray intended for use in the diagnosis of multiple sclerosis preferably comprises miRNA specific oligonucleotide probes for one or more miRNAs presented in the table in Figures 10A, 10B or 10C as described above, preferably for one or more miRNAs selected from the group consisting of hsa-miR-145, hsa-miR-186, hsa-miR-664, hsa-miR-584, hsa-miR-20b, hsa-miR-223, hsa-miR-422a, hsa-miR-142-3p, hsa-let-7c, hsa-miR-151-3p, hsa-miR-491-5p, hsa-miR-942, hsa-miR-361-3p, hsa-miR-22*, hsa-miR-140-5p, hsa-miR-216a, hsa-miR-1275, hsa-miR-367, hsa-miR-146a, hsa-miR-598, hsa-miR-613, hsa-miR-18a*, hsa-miR-302b, hsa-miR-501-5p.

In a preferred embodiment the microarray comprises oligonucleotide probes for determining at least 7, preferably at least 10, 15, 20 or all of the indicated miRNAs. It is particularly preferred to include oligonucleotide probes for determining the most significant miRNAs, which is represented by their order in the table depicted in Figure 10A, 10B or 10C. In a further embodiment, the microarray comprises oligonucleotide probes for determining miRNA sets based on Table 1 as described above.

The microarray can comprise oligonucleotide probes obtained from known or predicted miRNA sequences. The array may contain different oligonucleotide probes for each miRNA, for example one containing the active mature sequence and another being specific for the precursor of the miRNA. The array may also contain controls such as one or more sequences differing from the human orthologs by only a few bases, which can serve as controls for hybridization stringency conditions. It is also possible to include viral miRNAs or putative miRNAs as predicted from bioinformatic tools. Further, it is possible to include appropriate controls for non-specific hybridization on the microarray.

The invention also relates to sets of oligo- or polynucleotides for diagnosing multiple sclerosis comprising the sequences of at least 7, preferably at least 10, 15, 20 or all of the indicated miRNAs, and/or the complement of such sequences. It is particularly preferred to include oligo- or polynucleotides for detecting the most significant miRNAs, which are represented by their order in the table depicted in Figure 10A, 10B or 10C. In a further embodiment, the set includes oligo- or polynucleotides for detecting of the miRNA sets based on Table 1 as described above. The oligo- or polynucleotides preferably have a length of 10, 15 or 20 and up to 30, 40, 50, 100 or more nucleotides. The term "oligo- or polynucleotides" includes single- or double-stranded molecules, RNA molecules, DNA molecules or nucleic acid analogs such as PNA or LNA.

Another embodiment of the present invention relates to a method for the assessment of a clinical condition related to multiple sclerosis of a patient.

Recent developments have shown that there is a tendency towards smaller sets of biomarkers for the detection of diseases. However, for single biomarkers and small biomarker sets, there is only a basic understanding whether these biomarkers are specific for only the single diseases or whether they occur in any other disease.

Therefore, the present inventors developed a novel class of diagnostic tests improving the current test scenarios. The inventors found out that a variety of diseases are correlated with a specific expression profile of miRNAs. In case a patient is affected by a particular disease, several miRNAs are present in larger amounts compared to a healthy normal control, whereas the amount of other miRNAs is decreased. Interestingly, the amount of some miRNAs is deregulated, i.e. increased or decreased, in more than one disease. The miRNA profile for a particular disease therefore shows conformity with the miRNA profile of other diseases in regard of individual miRNAs while other miRNAs show significant differences. If the expression profile of a large variety of miRNAs in a biological sample of a patient is measured, the comparison of the expression profile with a variety of reference expression profiles which are each characteristic for different diseases makes it possible to obtain information about the clinical condition of a certain patient and to determine, which disease(s) is/are present in said patient.

A further subject matter of the invention is a method for the assessment of a clinical condition related to multiple sclerosis of a patient comprising the steps
(a) providing a sample from the patient,
(b) determining a predetermined set of miRNAs in said sample to obtain a miRNA expression profile,
(c) comparing said miRNA expression profile with a plurality of miRNA reference expression profiles characteristic for different diseases, and
(d) assessing the clinical condition of the patient based on the comparison of step (c).

The inventors found out that the above method for the assessment of a clinical condition makes it possible to carry out an integrative diagnosis of a wide variety of diseases, particularly including multiple sclerosis. Comparing a miRNA profile obtained from a biological sample of a patient whose clinical condition is not known with a plurality of reference profiles characteristic for different diseases enables the diagnosis of a wide variety of diseases with high specificity and sensitivity.

A "biological sample" in terms of the invention means a sample of biological tissue or fluid as described hereinabove. Examples of biological samples are sections of tissues, blood, blood fractions, plasma, serum, urine or samples from other peripheral sources.

The set of miRNAs determined in step (d) preferably includes a large number of different miRNAs. It is particularly preferred to use at least 10, 20, 30, 50, preferably at least 100, 200, 500 or 1,000 miRNAs. Most preferably, all known miRNAs are included in the set of miRNAs determined in step (b) Such a complex set of miRNA-biomarkers enables a diagnosis with higher specificity and sensitivity compared to single biomarkers or sets of only a few dozens of such markers.

The determination of the set of miRNAs can be done as described herein above. Preferably, the determination is done on an experimental platform which shows a high degree of automation to minimize experimental variations, measure results time- and cost-efficiently, measures results highly reproduceably and be able for measuring more than one sample at once in order to ensure a high throughput.

Step (c) preferably includes a comparison of the miRNA profile measured for a patient with a large number of different reference profiles to provide information about the presence of as many different diseases as possible. The reference expression profiles may be laid down in a database, e.g. an internet database, a centralized or a decentralized database. The reference profiles do not necessarily have to include information about all miRNAs included in step (b), which are determined in the sample of the patient. It is, according to the invention, sufficient if the reference profile provides information on those miRNAs which are altered to a large extent compared to the condition of a healthy individual in case of the presence of a disease. Alternatively, the said relevant reference may be a mathematical function or algorithm.

Preferably, an miRNA reference profile or the relevant reference according to the invention provides information on miRNA expression characteristic for a particular disease in the same type of biological sample as used in step (b) for determining a predetermined set of miRNAs in a sample from a patient, This means that, if a patient with an unknown disease is to be classified with the analysis of a blood sample, the comparison is preferably made with miRNA reference expression profiles, which do also relate to the miRNA expression pattern in a blood sample.

The reference profiles or the relevant reference characteristic for particular diseases provide information on one or more miRNAs, which are, in case of the disease, highly deregulated, for example strongly increased or decreased, as compared to a healthy condition. It is not necessary for the reference profiles to provide information about all miRNAs included in the set of biomarkers determined in step (b). However, the more miRNAs are included in the reference profile or relevant reference, the more precise the diagnosis will be. If, for example, a reference profile for lung cancer is included, it is preferred to include the characteristic miRNAs for lung cancer. Equivalently, it is preferred to include into a reference profile for multiple sclerosis the characteristic miRNAs for multiple sclerosis as described hereinabove as well.

Another embodiment of this aspect of the invention is a kit for the assessment of a clinical condition related to multiple sclerosis of a patient comprising
(a) means for determining a predetermined set of miRNAs in a biological sample from a patient, and
(b) a plurality of miRNA reference expression profiles characteristic for different diseases or a mathematical function that allows for the diagnosis on the basis of the data derived from the miRNA expression profiles of a patient.

The set of miRNAs to be determined in a biological sample from a patient preferably includes a large number of different miRNAs. It is particularly preferred to include all known miRNAs in the set of miRNAs to be determined. In each case, said predetermined set of miRNAs should include those miRNAs for which information is provided in the reference profiles characteristic for particular diseases. It is understood that only in case the set of miRNAs determined in a biological sample from a patient comprises those miRNAs included in the reference profile/reference for a disease, a diagnosis regarding this particular disease can be provided or otherwise the diagnosis may be less informative.

The assessment of a clinical condition of a patient according to the invention is suitable for diagnosing any diseases which are correlated with a characteristic miRNA profile. Accordingly, the kit for the assessment of a clinical condition preferably includes reference profiles/references for a plurality of diseases that are correlated with a characteristic miRNA profile. It is understood that all miRNAs that are significantly deregulated in the disease states for which reference profiles are provided should be included in the set of miRNAs to be determined in a biological sample from a patient. If the kit for the assessment of a clinical condition of a patient should provide information regarding, e.g. lung cancer or multiple sclerosis, a reference profile should be available providing information about the significantly deregulated miRNAs compared to a normal or any other relevant control individual or any other relevant control individual(s). A kit for the assessment of a clinical condition shall provide information on the presence of multiple sclerosis, a reference profile characteristic for multiple sclerosis should be included. Said reference profile preferably includes information on those miRNAs that are most significantly deregulated in the case of MS. The relevant miRNAs are as disclosed hereinabove.

The invention will now be illustrated by the following figures and the nonlimiting experimental examples.

### Figures

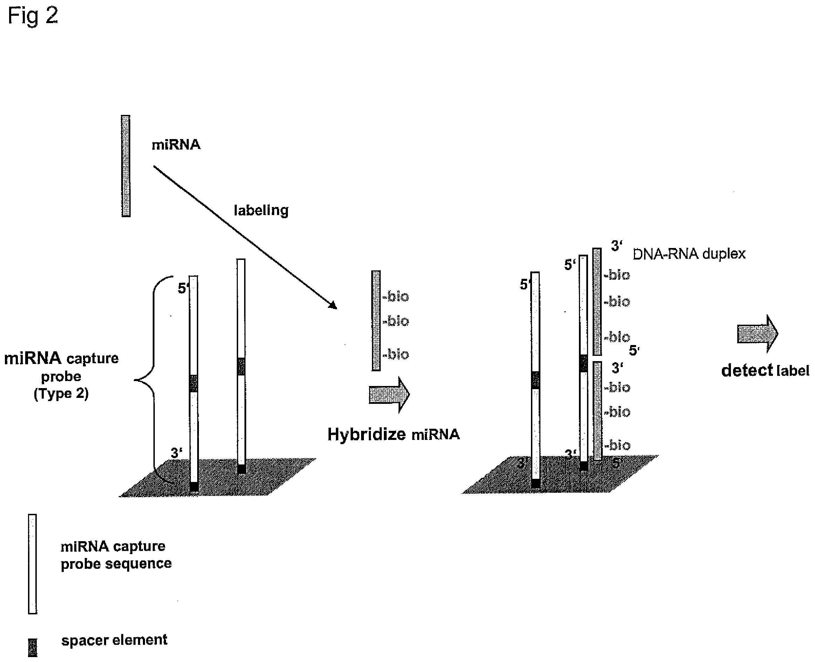

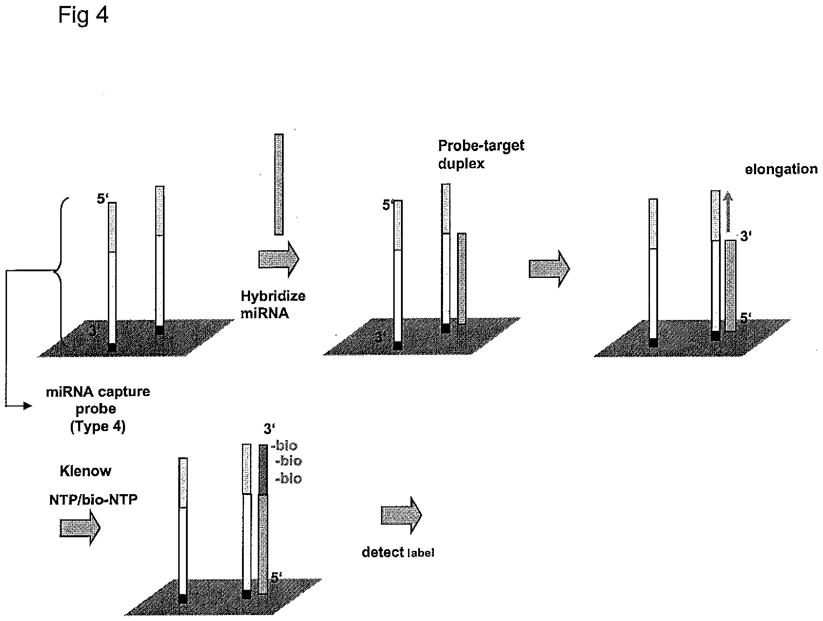

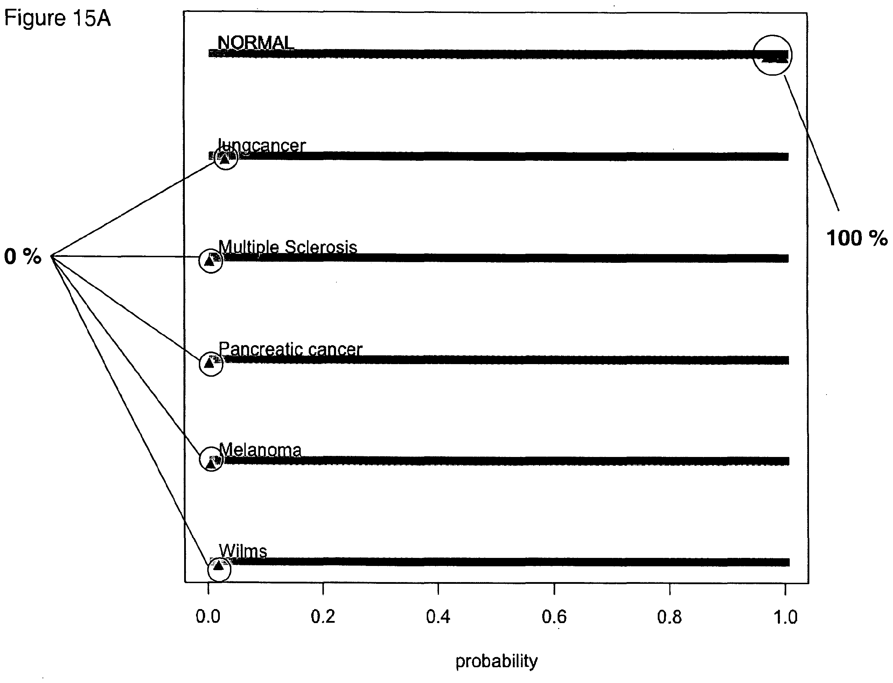

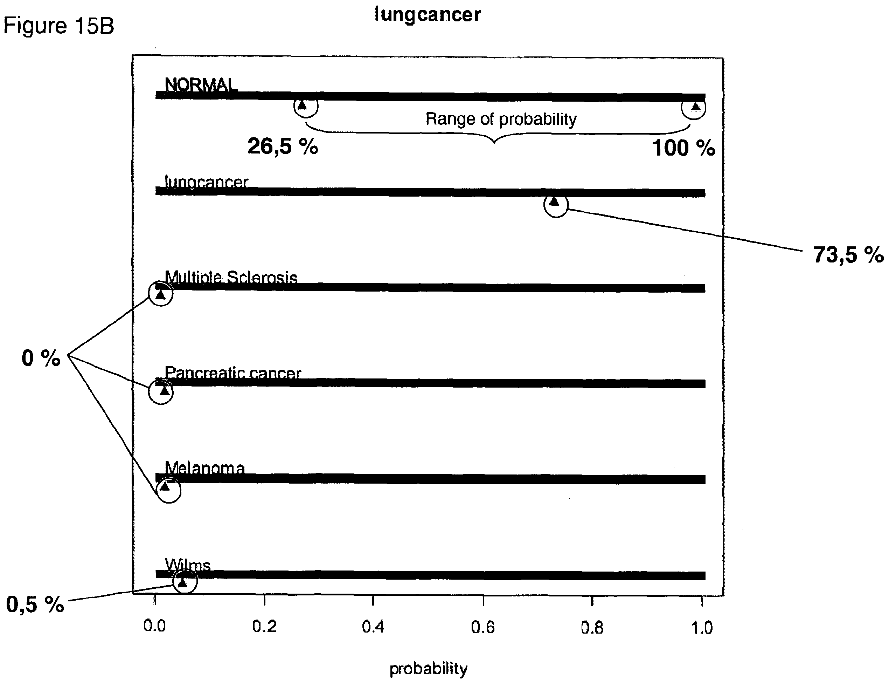

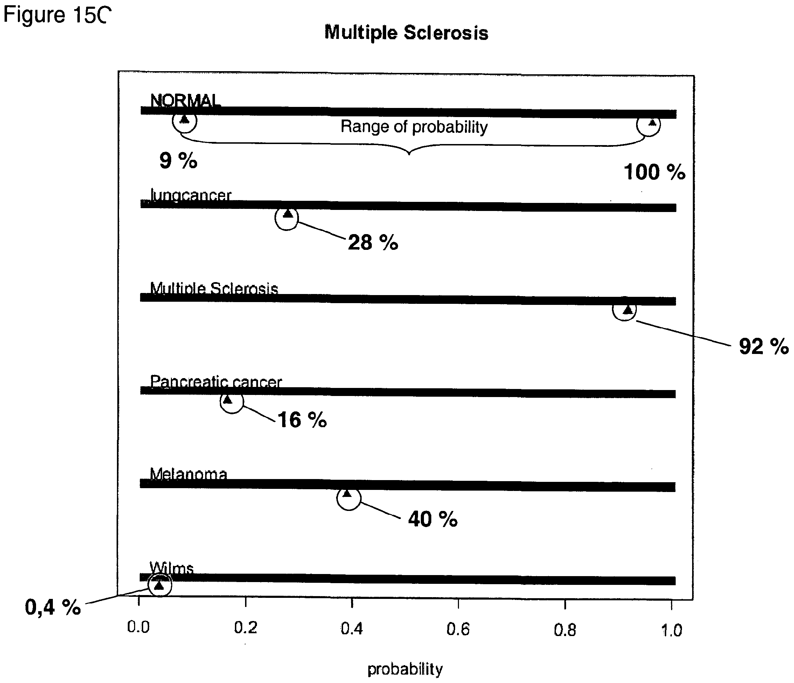

Figure 1:
   Scheme of a miRNA hybridization assay for use in the invention.
      - miRNA capture probes consist of 1 miRNA probe sequence stretch that is linked to support via 3'-end or alternatively by 5'-end (not depicted here)
      - the miRNA probe sequence stretches are complementary to miRNA target sequences
      - each miRNA capture probe can bind 1 miRNA target sequences
      - the miRNA target sequences are labeled prior to hybridisation (e.g, by biotin labeling)
Figure 2:
   Scheme of an miRNA tandem hybridization assay for use in the invention
      - miRNA capture probes consist of 2 DNA-based miRNA probe sequence stretches that are linked to each other by a spacer element
      - the miRNA probe sequence stretches are complementary to miRNA target sequences
      - each miRNA capture probe can bind 2 miRNA target sequences
      - the spacer sequence consists of 0 - 8 nucleotides the miRNA target sequences are labeled prior to hybridisation (e.g. by biotin labeling)
Figure 3:
   miRNA RAKE-Assay for use in the invention (PT Nelson et al., Nature Methods, 2004, 1(2), 1)
      - the miRNA capture probes consist of one miRNA probe sequence stretch (green) and one elongation element (orange)
      - probes are orientied 5'→3', presenting a free terminal 3'-OH
      - the miRNA probe sequence stretch (green) is complementary to miRNA target sequences (dark green)
      - the elongation sequences (orange) can be freely chosen and is typically between 1-12 nucleotides long, preferably a homomeric sequence
      - each miRNA capture probe can bind 1 miRNA target sequence
      - the miRNA target sequences are NOT labeled prior to hybridisation
      - Labeling occurs after hybridisation during elongation by polymerase extension reaction
         Biochip is not reusable due to exonuclease treatment
Figure 4:
   - miRNA MPEA-Assay for use in the invention (Vorwerk S. et al., Microfluidic-based enzymatic on-chip labeling of miRNAs, N. Biotechnol. 2008; 25(2-3):142-9. Epub 2008 Aug 20)
   - the miRNA capture probes consist of one miRNA probe sequence stretch (green) and one elongation element (orange)
   - probes are orientied 3'→5', presenting a free terminal 5'-OH the miRNA probe sequence stretch (green) is complementary to miRNA target sequences (dark green)
   - the elongation sequences (orange) can be freely chosen and is typically between 1-12 nucleotides long, preferably a homomeric sequence
   - each miRNA capture probe can bind 1 miRNA target sequences
   - the miRNA target sequences are NOT labeled prior to hybridisation
   - Labeling occurs after hybridisation during elongation by polymerase extension reaction
   - Biochip is reusable after removal of target / elongated target
Figure 5:
   miRNA capture probe design (design MPEA/RAKE)
   Depicted is the design of a capture probe for the exemplary miRNA human mature miRNA let-7a for use in the various types of hybridization assays shown in Figures 1-4. SP = spacer element; EL = elongation element
Figure 6:
   Spacer Element.
      Capture probes for use in e.g. a tandem hybridization assay as shown in Figure 2 may comprise a spacer element SP. The spacer element represents a nucleotide sequence with n = 0-12 nucleotides chosen on the basis of showing low complementarity to potential target sequences, therefore resulting in no to low degree of crosshybridization to target mixture. Preferably, n = 0, i.e. there is no spacer between the 2 miRNA probe sequence stretches.
Figure 7:
   Elongation element (Design Probe MPEA/RAKE)
      A capture probe, e.g. for use in a RAKE or MPEA assay as shown in Figures 3 and 4 may include an elongation element. The elongation element comprises a nucleotide sequence with N= 0-30 nucleotides chosen on the basis of showing low complementarity to potential target sequences, therefore resulting in no to low degree of crosshybridization to target mixture. Preferred is a homomeric sequence stretch -Nₙ- with n = 1-30, N = A or C, or T, or G. Especially preferred is a homomeric sequence stretch -Nn- with n = 1-12, N = A or C, or T, or G.
Figure 8:
   Pearson Correlation Coefficient depending on the number of elongated nucleotides in capture probes in an MPEA assay.
Figure 9:
   Diagram describing the general approach for determining miRNA signatures for use as biomarkers in disease diagnosis.
Figure 10A:
   Overview of all miRNAs that are found to be differentially regulated in blood samples of MS patients, grouped according to their diagnostic information represented by the respective area under the curve (AUC) value in receiver-operator characteristic curves. The first 193 entries represent miRNAs with t-test p-values < 0.05.
Figure 10B:
   Overview of all miRNAs that are found to be differentially regulated in blood samples of MS patients, grouped according to their diagnostic information represented by the t-test significance. The first 165 entries represent miRNAs with t-test p-values < 0.05. The grouping is based on additional information derived from further patients (compared to Fig. 10A).
Figure 10C:
   A further list of 308 entries representing miRNAs with t-test p-values < 0.05. The grouping is based on additional information derived from further patients (compared to Fig. 10A and 10B).
Figure 11:
   Histogram plots of the logarithm of fold quotions, the raw t-test p-values and the adjusted p-values. The histogram plots show in the upper part a histogram of logarithmized fold changes, detailing a manifold up-regulated miRNAs in Multiple Sclerosis compared to healthy subjects. The middle and lower part of the Figure describe raw significance values and adjusted significance values providing evidence for a wide variety of deregulated miRNAs that are well suited for MS detection.
Figure 12A and 12B:
   This Figure presents for two miRNAs, namely miR-145 and miR-186, the intensity values for all MS (left part) and control (right part) samples. Both miRNAs show a significant up-regulation in MS.
Figure 13:
   The Box-plots denote the accuracy, specificity and sensitivity of the diagnostic test of the invention. In comparison, random classification results are shown, providing evidence for a decreased classification accuracy of about 50% (corresponding to random guessing). Furthermore, the graphic shows that the true classification scenario is more stable while the random classifications entail high variances.
Figure 14:
   This graph illustrates a disease network containing nodes for each disease as blue-colored rhombs (lung cancer, multiple sclerosis, pancreatic cancer, melanoma and Wilm tumor). Additionally, it contains differentially colored and sized nodes, representing biomarker sets. The size of these nodes represents the number of biomarkers inside the set (additionally the number of biomarkers is given inside the corresponding circles). The color represents the information on the number of diseases that are significant for the biomarkers in the set. The nodes are connected to the respective diseases, e.g., each green colored node contains biomarkers, significant for two diseases and thus each green node is connected to two disease nodes. (blue = significant to one disease, rose = significant for 3 diseases, purple = significant to 5 diseases).
Figure 15:
   The bar graphs in Figures 15a), b) and c) depict the disease probability for a "normal" individual a), for an individual suffering from lung cancer b) and for an individual suffering from multiple sclerosis c).

### Examples

### Example 1: Multiple Sclerosis

### 1.1 Materials and Methods

### 1.1.1 Samples

Blood samples were obtained with patients' informed consent.

### 1.1.2 miRNA microarray screening

Blood of MS patients and volunteers without known disease was extracted in PAXgene Blood RNA tubes (BD, Franklin Lakes, New Jersey USA). For each blood donor, 5 ml of peripheral blood were obtained. Total RNA was extracted from blood cells using the miRNeasy Mini Kit (Qiagen GmbH, Hilden, Germany) and the RNA has been stored at -70°C. Samples were analyzed with the Geniom Realtime Analyzer (GRTA, febit GmbH, Heidelberg, Germany) using the Geniom Biochip miRNA homo sapiens. Each array contains 7 replicates of 866 miRNAs and miRNA star sequences as annotated in the Sanger mirBase 12.0 . Sample labelling with Biotine has been carried out by multifluidic-based enzymatic on-chip labelling of miRNAs (MPEA).

Following hybridization for 16 hours at 42°C the biochip was washed automatically and a program for signal enhancement was processed with the GRTA. The resulting detection pictures were evaluated using the Geniom Wizard Software. For each array, the median signal intensity was extracted from the raw data file such that for each miRNA seven intensity values have been calculated corresponding to each replicate copy of mirBase on the array. Following background correction, the seven replicate intensity values of each miRNA were summarized by their median value. To normalize the data across different arrays, quantile normalization was applied and all further analyses were carried out using the normalized and background subtracted intensity values.

### 1.1.3 Statistical analysis

After having verified the normal distribution of the measured data, a parametric t-test (unpaired, two-tailed) was carried out for each miRNA separately, to detect miRNAs that show a different behavior in different groups of blood donors. The resulting p-values were adjusted for multiple testing by Benjamini-Hochberg adjustment.

To find relations of the detected miRNAs to other diseases the Human miRNA Disease Database was used. In more detail, a bipartite network was built where nodes correspond either to a miRNA or to a diseases. Only edges between miRNA and diseases nodes are allowed, where an edge between miRNA *A* and disease B means that the miRNA *A* is differentially regulated in disease B. Since for MS no deregulated miRNAs are known the node "MultipleSclerosis" was added to this network and linked to all miRNAs that were significant in the analysis.

In addition to the single biomarker analysis and network analysis, classification of samples using miRNA patterns was carried out using Support Vector Machines (SVM,) as implemented in the R e1071 package. In detail, different kernel (linear, polynomial, sigmoid, radial basis function) Support Vector Machines were evaluated, where the cost parameter was sampled from 0.01 to 10 in decimal powers. The measured miRNA profiles were classified using 100 repetitions of standard 10-fold cross-validation. As a subset selection technique we applied a filter approach based on t-test. In detail, the s miRNAs with lowest p-values were computed on the training set in each fold of the cross validation, where s was sampled from 1 to 866. The respective subset was used to train the SVM and to carry out the prediction of the test samples. As result, the mean accuracy, specificity, and sensitivity were calculated together with the 95% Confidence Intervals (95% Cl) for each subset size. To check for overtraining permutation tests were applied. Here the class labels were sampled randomly and classifications were carried out using the permuted class labels. All statistical analyzes were performed using R .

### 1.2 Results

### 1.2.1 miRNA experiments

The expression of 866 miRNAs and miRNA star sequences was analyzed in blood cells of 22 patients with MS. As a control blood cells of 22 volunteers without known disease were used.

Following RNA isolation and the novel on-chip labeling technique, the miRNA expression profiles were measured by the Geniom Bioship miRNA homo sapiens in the GRTA (febit GmbH, Heidelberg). Following intensity value computation and quantile normalization of the miRNA profiles, a mean correlation value of 0.97 for technical replicates was determined by using purchased total RNA from Ambion (four heart and four liver replicates). For the biological replicates the different tumor samples were compared between each other and the different normal samples were compared between each other. The biological replicates showed a mean correlation of 0.87 and a variance of 0.009.

### 1.2.2 Ruling out the influence of age and gender

To cross-check that age and gender do not have an influence on our analysis, t-tests for the normal samples were computed. In the case of males versus females there were no statistically significant deregulated miRNA.

The most significant miRNA, hsa-miR-423, showed an adjusted significance level of 0.78.

To test for the influence of donor age the profiles obtained from samples obtained from the oldest versus youngest patients were compared by splitting the group in half based on age. Here, the most significant miRNA, miR-890, obtained an adjusted p-value of 0.87. As for gender, there were no deregulated miRNAs, thus providing evidence that age and gender do not have a substantial influence on the miRNA profiles.

Additionally, we checked the influence of a therapy. Patients were either treated with glatiramer acetate (n = 9), or interferon-β (n = 10), or did not get a therapy (n = 1). We compared the group of patients treated with glatiramer acetate to the group treated with interferon-β. As for the gender and the age we did not find any significant miRNA.

### 1.2.3 Single deregulated miRNAs

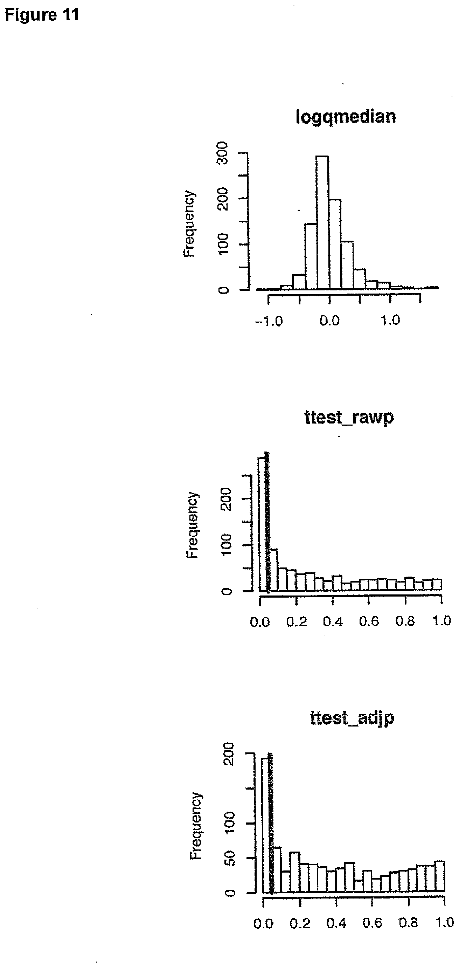

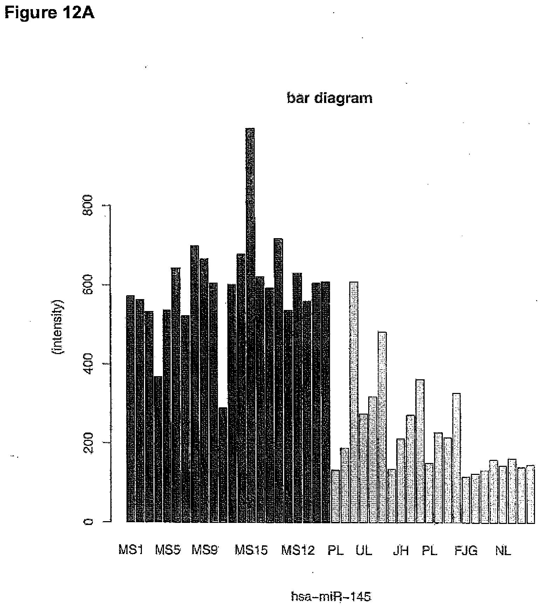

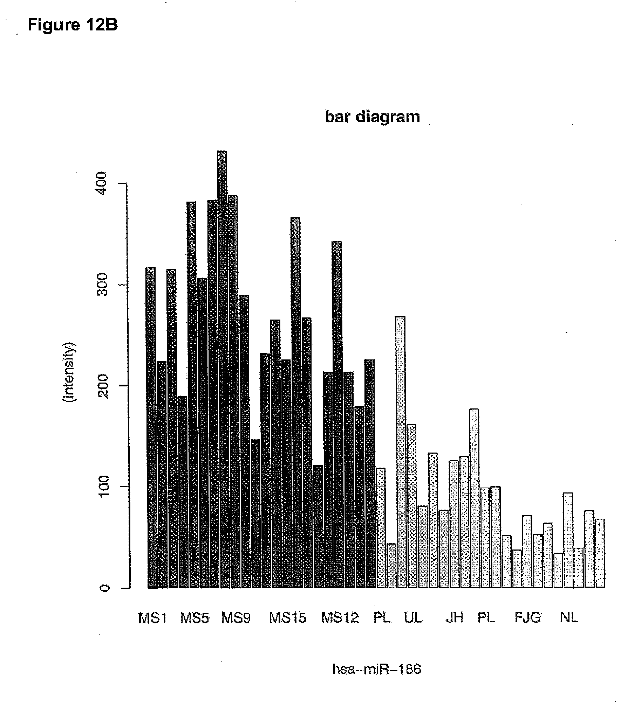

Hypothesis testing was applied to identify miRNAs deregulated in the blood cells of MS patients as compared to the blood cells of the controls. Following verification of an approximately normal distribution, two-tailed unpaired t-tests were performed for each miRNA. The respective p-values were adjusted for multiple testing by the Benjamini-Hochberg approach. In total 193 miRNAs significantly deregulated in blood cells of MS patients as compared to the controls were detected. Histogram plots of the logarithm of fold quotients, the raw t-test p-values and the adjusted p-values are presented in Figure 11. A complete list of deregulated miRNAs is given in the Table in Figure 10A. The miRNAs that were most significantly deregulated included hsa-miR-145 (5,25*10⁻⁹), hsa-miR-186 (3,42*10⁻⁷), hsa-miR-664 (1,20*10⁻⁵), hsa-miR-20b (1,98*10⁻⁵), hsa-miR-584 (1,98*10⁻⁵), hsa-miR-223 (2,14*10⁻⁵), hsa-miR-422a (2,87*10⁻⁵), hsa-miR-142-3p (3,01*10⁻⁵)and hsa-let-7c (7,68*10⁻⁵). For the two best miRNAs, hsa-miR-186 and hsa-miR-145, bar-plots showing the intensity values for all MS and control samples are presented in Figures 12a and 12b.

Notably, all the above-mentioned miRNAs showed a significant up-regulation in MS besides miR-20b. Table 2 shows the 24 most deregulated miRNAs. Of these 91.7% were up-regulated in MS while 8.3% were down-regulated, providing evidence for an overall up-regulation of miRNAs in MS.

Additionally for the best miRNAs receiver operator characteristic curves (ROC) and the area under the curve value (AUC) were computed. The higher the AUC, the better the miRNA biomarker is, where a maximal value of 1 for miRNA *A* would mean that the highest control reactivity would be lower than the lowest MS intensity of miRNA *A*. For the best miRNA hsa-miR-145 an AUC value of 0.96 was obtained and four of the 44 samples were wrong classified (2 Ms sera as controls, so-called False Negatives, and 2 controls classified as MS samples, so-called False Positives).

**Table 2: 24 most significant miRNAs for MS**

| **miRNA** | **median g1** | **median g2** | **qmedian** | **Log (qmedian)** | **raw Pval** | **adj. Pval** | **AUC** |
|---|---|---|---|---|---|---|---|
| hsa-miR-145 | 602,719 | 174,344 | 3,457 | 1,240 | 6,08E-12 | 5,25E-09 | 0,962 |
| hsa-miR-186 | 265,295 | 77,719 | 3,414 | 1,228 | 7,91E-10 | 3.42E-07 | 0,961 |
| hsa-miR-664 | 707,168 | 285,703 | 2,475 | 0,906 | 4,17E-08 | 1,20E-05 | 0,916 |
| hsa-miR-584 | 332,922 | 106,969 | 3,112 | 1,135 | 1,15E-07 | 1,98E-05 | 0,897 |
| hsa-miR-20b | 2689,207 | 5810,586 | 0,463 | -0,770 | 9,83E-08 | 1,98E-05 | 0,056 |
| hsa-miR-223 | 5118,574 | 2579,250 | 1,985 | 0,685 | 1,49E-07 | 2,14E-05 | 0,964 |
| hsa-miR-422a | 373,953 | 189,219 | 1,976 | 0,681 | 2,32E-07 | 2,87E-05 | 0,870 |
| hsa-miR-142-3p | 215,375 | 40,516 | 5,316 | 1,671 | 2,79E-07 | 3,01E-05 | 0,934 |
| hsa-let-7c | 1948,098 | 950,223 | 2,050 | 0,718 | 8,00E-07 | 7,68E-05 | 0,889 |
| hsa-miR-151-3p | 1021,363 | 571,344 | 1,788 | 0,581 | 1.81E-06 | 0,000156587 | 0,883 |
| hsa-miR-491-5p | 241,000 | 153,563 | 1,569 | 0,451 | 2,05E-06 | 0,000160884 | 0,876 |
| hsa-miR-942 | 112,969 | 38,094 | 2,966 | 1,087 | 5,09E-06 | 0,000366A.52 | 0,882 |
| hsa-miR-361-3p | 325,766 | 181,375 | 1,796 | 0,586 | 5,77E-06 | 0,000383235 | 0,852 |
| hsa-miR-22* | 178,938 | 103,844 | 1,723 | 0,544 | 6,24E-06 | 0,000385004 | 0,868 |
| hsa-miR-140-5p | 105,063 | 48,250 | 2,177 | 0,778 | 7,99E-06 | 0,000399262 | 0,874 |
| hsa-miR-216a | 202,219 | 315,828 | 0.640 | -0,446 | 8,24E-06 | 0,000399262 | 0,060 |
| hsa-miR-1275 | 210,203 | 116,969 | 1,797 | 0,586 | 7,04E-06 | 0,000399262 | 0,907 |
| hsa-miR-367 | 92,500 | 160,375 | 0,577 | -0,550 | 8,32E-06 | 0,000399262 | 0,138 |
| hsa-miR-146a | 470,359 | 271,342 | 1,733 | 0,550 | 9,61E-06 | 0,000437137 | 0,862 |
| hsa-miR-598 | 140,531 | 91,000 | 1,544 | 0,435 | 1,29E-05 | 0,000556416 | 0,841 |
| hsa-miR-613 | 60,781 | 19,000 | 3,199 | 1,163 | 1,67E-05 | 0,000687276 | 0,862 |
| hsa-miR-18a* | 490,891 | 233,672 | 2,101 | 0,742 | 2,02E-05 | 0,000794863 | 0,876 |
| hsa-miR-302b | 54,469 | 21,406 | 2,545 | 0,934 | 2,23E-05 | 0,000838901 | 0,855 |
| hsa-miR-501-5p | 139,938 | 79,563 | 1,759 | 0,565 | 2,60E-05 | 0,000936279 | 0,866 |

### 1.2.4 Relation to other diseases

Since there is no evidence for de-regulated miRNAs in MS patients in the literature, it was checked whether the detected 193 miRNAs are already related to other human diseases. To this end, the Human microRNA Disease Database (HMDD) was grasped. This comprehensive database contains for over 100 human diseases information on deregulated miRNAs. Altogether, over 2000 relations are included in the HMDD. To analyze the respective data, a bipartite graph was created were nodes are either miRNAs or human diseases, and edges between a miRNA and a disease mean that the respective miRNA is deregulated in the respective disease.

Thereby, a network containing 452 nodes was created, 137 belonging to diseases and 315 to miRNAs. The network also contained 1617 unique edges (some relations between miRNAs and diseases have been published in multiple papers). As mentioned previously, MS is not included as disease in this network. Thus, the network was modified as followings: a disease node "MultipleSclerosis" was added and edges between this node and all significant miRNAs were created. Additionally, all disease nodes that are not linked to any MS miRNA and all miRNAs belonging only to removed disease nodes were removed. The novel network thus contains only those miRNAs that are significant in MS and other diseases and those that are significant in MS, only. This shrunken network contained 77 disease nodes together with the 193 significant miRNAs. Remarkably, only 43 of the 193 (22%) miRNAs were related to a disease other than MS while the remaining 78% miRNAs were only connected to MS. Of these 146 miRNAs, 36 were so-called star sequences.

Altogether, these results provide strong evidence that the detected complex miRNA profile is not disease specific but rather specific for MS.

### 1.2.5 Evaluating complex fingerprints

As discussed in Section 1.2.3, the best miRNA suffices to classify 20 of 22 MS samples and 20 of 22 control samples correctly. This obviously corresponds to a high specificity, sensitivity and accuracy of 90.8%. However, these results are not validated by a re-sampling technique as bootstrapping or cross-validation and are based only on a single marker.

In order to improve the already high classification accuracy and the statistical reliability the predictive power of multiple miRNAs was combined by using statistical learning techniques. In detail, Support Vector Machines with different kernels (linear, polynomial, sigmoid, radial basis function) were applied to the data and a hypothesis test based subset selection was carried out as described in Material and Methods. To gain statistical significance 100 repetitions of 10-fold cross validation were carried out. Likewise, 100 repetitions for the permutation tests were computed where samples with randomly assigned class labels were investigated.

The best results were obtained with radial basis function Support Vector Machines and a subset of 24 miRNAs (see Table 2). These miRNAs allowed for the discrimination between blood samples of MS patients and blood samples of controls with an accuracy of 95.5% a specificity of 95.5%, and a sensitivity of 95.5%.

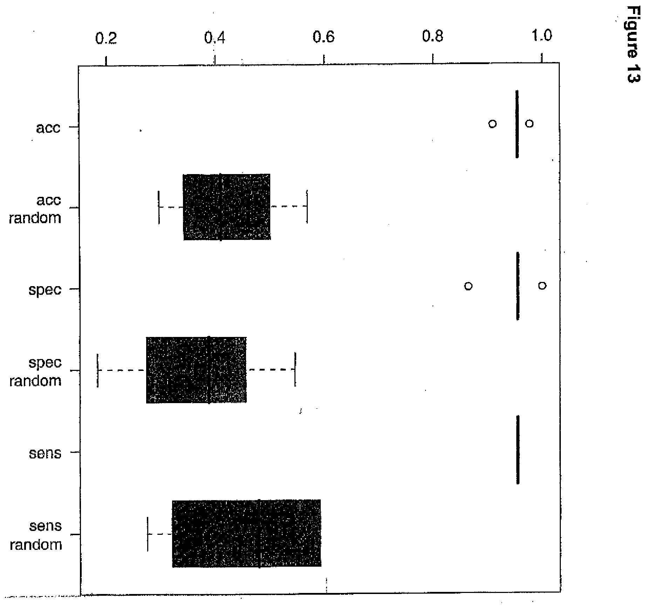

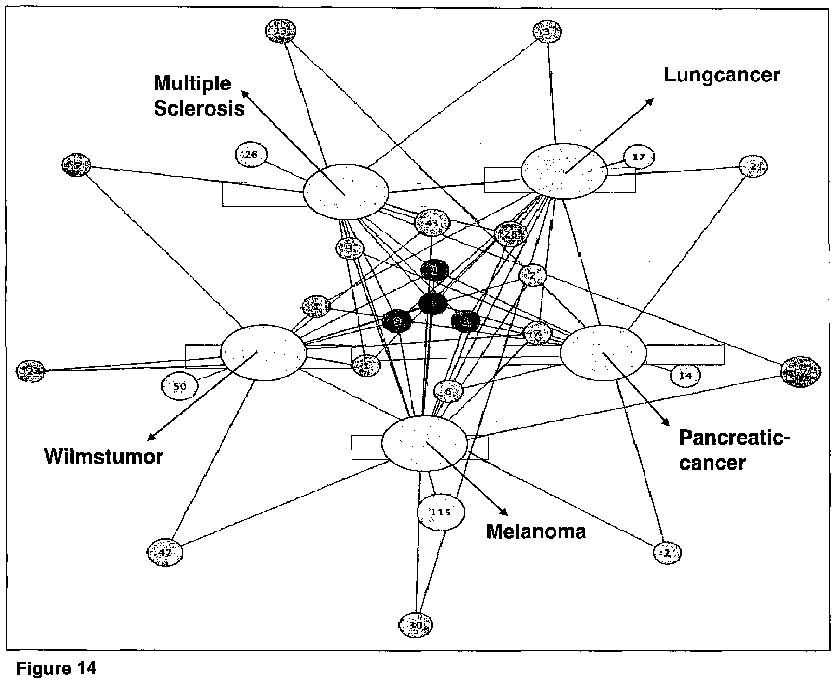

The permutation tests showed significantly decreased accuracy, specificity, and sensitivity rates, as detailed in Figure 13. These results show that the obtained results are not due to an overfit of the statistical model on the miRNA fingerprints.

Additionally, it was checked whether the relevant miRNAs were linked to one of over 100 other diseases as annotated in the HMDD. Remarkable over 80% of the respective miRNAs have not been linked to other diseases, so far.

### Example 2: Molecular clinical thermometer

For the molecular clinical thermometer, an arbitrary machine learning (feature extraction/ classification / regression/ clustering) technique can be applied. The workflow does not depend on the applied method that can be seen as a black box.

First, a sophisticated large set of samples for the diseases to be investigated has to be measured using a larger amount of biomarkers. This set, consisting of a p x n matrix where n is the number of samples and p the number of biomarkers, is commonly denoted as training data set.

Now, a combination of feature extraction and supervised learning techniques (the process can be also carried out with slight modifications using unsupervised techniques) is applied to generate a statistical model, which describes the training data well. Here, it is essential to control the model complexity in order to avoid so-called overtraining of the statistical models. Although, in general, multi-class cases can be carried out, we focus on two class comparisons, i.e., normal versus cancer 1, normal versus cancer 2, cancer 1 versus cancer 2.

Given the trained models and a new biomarker profile, the statistical model can be used to compute the probability for each class and this new sample. Only one example are support vector machines, where the distance of a sample to the seperating hyperplane can be used to estimate the class probability via a regression approach. The specificity and sensitivity can be trade-off by shifting the probability threshold (which usually should be 0.5 or 50%).

The probabilities in the previously described step can be used to generate so-called disease probability plots (DPP). These plots contain for each class and a single sample the probabilities to belong to a certain class. In detail, each class is described by a colored line of length 100 (representing a percentage range), where the lower rate is colored green (representing small probabilities) and the higher range red (higher probabilities). For each class, an arrow marks the probability for the patient and the respective disease. For class "normal" the minimal and maximal probability to be normal are shown.

The DPPs thus allow for visualizing the complex statistical evaluation in a simple and well interpretable way.

### References

Alvarez-GarCia, I. and E. A. Miska (2005). "MicroRNA functions in animal development and human disease." Development 132(21): 4653-62.
Benjamini, Y. and Y. Hochberg (1995). "Controlling the false discovery rate: A practical and powerful approach to multiple testing." J R Statist Soc B 57: 289-300.
Bolstad, B. M., R. A. Irizarry, et al. (2003). "A comparison of normalization methods for high density oligonucleotide array data based on variance and bias." Bioinformatics 19(2): 185-93.
Calin, G. A. and C. M. Croce (2006). "MicroRNA-cancer connection: the beginning of a new tale." Cancer Res 66(15): 7390-4.
Calin, G. A. and C. M. Croce (2006). "MicroRNA signatures in human cancers." Nat Rev Cancer 6(11): 857-66.
Chen, X., Y. Ba, et al. (2008). "Characterization of microRNAs in serum: a novel class of biomarkers for diagnosis of cancer and other diseases." Cell Res 18(10): 997-1006.
Crawford, M., E. Brawner, et al. (2008). "MicroRNA-126 inhibits invasion in non-small cell lung carcinoma cell lines." Biochem Biophys Res Commun 373(4): 607-12.
Esquela-Kerscher, A. and F. J. Slack (2006). "Oncomirs - microRNAs with a role in cancer." Nat Rev Cancer 6(4): 259-69.
Feitelson, M. A. and J. Lee (2007). "Hepatitis B virus integration, fragile sites, and hepatocarcinogenesis." Cancer Lett 252(2): 157-70.
Gilad, S., E. Meiri, et al. (2008). "Serum microRNAs are promising novel biomarkers." PLoS ONE 3(9): e3148.
Grifffihs-Jones, S., R. J. Grocock, et al. (2006). "miRBase: microRNA sequences, targets and gene nomenclature." Nucleic Acids Res 34(Database issue): D140-4.
Griffiths-Jones, S., S. Moxon, et al. (2005). "Rfam: annotating non-coding RNAs in complete genomes." Nucleic Acids Res 33(Database issue): D121-4.
Griffiths-Jones, S., H. K. Saini, et al. (2008). "miRBase: tools for microRNA genomics." Nucleic Acids Res 36(Database issue): D154-8.
Guo, L., Z. X. Huang, et al. (2008). "Differential Expression Profiles of microRNAs in NIH3T3 Cells in Response to UVB Irradiation." Photochem Photobiol.
Harris, T. A., M. Yamakuchi, et al. (2008). "MicroRNA-126 regulates endothelial expression of vascular cell adhesion molecule 1." Proc Natl Acad Sci USA 105(5): 1516-21.
Hayashita, Y., H. Osada, et al. (2005). "A polycistronic microRNA cluster, miR-17-92, is overexpressed in human lung cancers and enhances cell proliferation." Cancer Res 65(21): 9628-32.
He, L., J. M. Thomson, et al. (2005). "A microRNA polycistron as a potential human oncogene." Nature 435(7043): 828-33.
Henschke, C. I. and D. F. Yankelevitz (2008). "CT screening for lung cancer: update 2007." Oncologist 13(1): 65-78.
Hochberg, Y. (1988). "A sharper bonferroni procedure for multiple tests of significance." Biometrica 75: 185-193.
Ichimi, T., H. Enokida, et al. (2009). "Identification of novel microRNA targets based on microRNA signatures in bladder cancer." Int J Cancer.
Jemal, A., R. Siegel, et al. (2008). "Cancer statistics, 2008." CA Cancer J Clin 58(2): 71-96.
Johnson, S. M., H. Grosshans, et al. (2005). "RAS is regulated by the let-7 microRNA family." Cell 120(5): 635-47.
Keller, A., N. Ludwig, et al. (2006). "A minimally invasive multiple marker approach allows highly efficient detection of meningioma tumors." BMC Bioinformatics 7: 539.
Lee, R. C., R. L. Feinbaum, et al. (1993). "The C. elegans heterochronic gene lin-4 encodes small RNAs with antisense complementarity to lin-14." Cell 75(5): 843-54.
Lu, J., G. Getz, et al. (2005). "MicroRNA expression profiles classify human cancers." Nature 435(7043): 834-8.
Mann, H. and F. Wilcoxon (1947). "On a test whether one of two random variables is stochastically larger than the other." Ann Mat Stat 18: 50-60.
Sassen, S., E. A. Miska, et al. (2008). "MicroRNA: implications for cancer." Virchows Arch 452(1): 1-10.
Scott, W. J., J. Howington, et al. (2007). "Treatment of non-small cell lung cancer stage I and stage II: ACCP evidence-based clinical practice guidelines (2nd edition)." Chest 132(3 Suppl): 234S-242S.
Shannon, C. (1984). "A mathematical theory of communication." The Bell System Technical Journal 27: 623-656.
Stahlhut Espinosa, C. E. and F. J. Slack (2006). "The role of microRNAs in cancer." Yale J Biol Med 79(3-4): 131-40.
Takamizawa, J., H. Konishi, et al. (2004). "Reduced expression of the let-7 microRNAs in human lung cancers in association with shortened postoperative survival." Cancer Res 64(11): 3753-6.
Team, R. D. C. (2008). R: A Language and Environment for Statistical Computing.. Vienna, Austria, R Foundation for Statistical Computing.
Tong, A. W. (2006). "Small RNAs and non-small cell lung cancer." Curr Mol Med 6(3): 339-49.
Vapnik, V. (2000). The Nature of Statistical Learning Theory., Springer.
Volinia, S., G. A. Calin, et al. (2006). "A microRNA expression signature of human solid tumors defines cancer gene targets." Proc Natl Acad Sci U S A 103(7): 2257-61.
Vorwerk, S., K. Ganter, et al. (2008). "Microfluidic-based enzymatic on-chip labeling of miRNAs." N Biotechnol 25(2-3):142-9.
Wilcoxon, F. (1945). "Individual comparisons by ranking methods." Biometric Bull 1 : 80-83.
Williams, A. E. (2008). "Functional aspects of animal microRNAs." Cell Mol Life Sci 65(4): 545-62.
Yanaihara, N., N. Caplen, et al. (2006). "Unique microRNA molecular profiles in lung cancer diagnosis and prognosis." Cancer Cell 9(3): 189-98.
Zhang, B., X. Pan, et al. (2007). "microRNAs as oncogenes and tumor suppressors." Dev Biol 302(1): 1-12.
Zhang, B., Q. Wang, et al. (2007). "MicroRNAs and their regulatory roles in animals and plants." J Cell Physiol 210(2): 279-89.

## Claims

1. A method of diagnosing multiple sclerosis, comprising the steps
(a) determining an expression profile of a predetermined set of miRNAs in a blood sample from a patient; said predetermined set of miRNAs comprising hsa-miR-145
(b) comparing said expression profile to a reference expression profile,
wherein the comparison of said determined expression profile to said reference expression profile allows for the diagnosis of multiple sclerosis.

2. The method of claim 1, wherein the blood sample is selected from whole blood, blood cells, plasma and/or serum, or samples comprising erythrocytes, leukocytes or thrombocytes.

3. The method of any one of claims 1 to 2, wherein said predetermined set of miRNAs comprises one or more miRNAs known to be differentially expressed in the disease state to be diagnosed.

4. The method of any one of claims 1 to 3, wherein the determination of an expression profile in step (a) comprises nucleic acid hybridization, nucleic acid amplification, polymerase extension, sequencing, mass spectroscopy or any combinations thereof.

5. The method of any one of claims 1 to 4, wherein the predetermined set of miRNAs comprises a signature selected from the group of
A4 : hsa-let-7c, hsa-miR-145, and hsa-miR-330-3p;
A19: hsa-miR-92b*, hsa-let-7c, hsa-miR-145, hsa-miR-330-3p, and hsa-miR-30e;
A41: hsa-miR-1234, hsa-miR-92b*, hsa-miR-145, hsa-miR-30e, and hsa-miR-223;
B6: hsa-let-7c, hsa-let-7g, and hsa-miR-145;
B7: hsa-miR-145, hsa-let-7i, and hsa-miR-130b;
A18 : hsa-miR-125a-5p, hsa-miR-92b*, hsa-let-7c, hsa-miR-145, and hsa-miR-330-3p;
A20: hsa-miR-145, hsa-miR-330-3p, hsa-miR-30e, hsa-miR-199a-5p, and hsa-miR-223;
A31 : hsa-let-7b, hsa-miR-574-3p, hsa-miR-500, hsa-miR-1234, hsa-miR-125a-5p, hsa-miR-92b*, hsa-let-7c, hsa-miR-145, hsa-miR-330-3p, and hsa-miR-30e;
A32: hsa-miR-92b*, hsa-let-7c, hsa-miR-145, hsa-miR-330-3p, hsa-miR-30e, hsa-miR-199a-5p, hsa-miR-223, hsa-miR-195, hsa-miR-182, hsa-miR-107, and hsa-miR-497;
A42: hsa-miR-145, hsa-miR-30e, hsa-miR-223, hsa-miR-182, and hsa-miR-497;
B21 : hsa-miR-92b*, hsa-let-7c, hsa-let-7g, hsa-miR-145, and hsa-let-7i;
B22: hsa-let-7c, hsa-let-7g, hsa-miR-145, hsa-let-7i, hsa-miR-130b, and hsa-miR-1260;
B32 : hsa-let-7e, hsa-miR-1234, hsa-miR-125a-5p, hsa-let-7f, hsa-miR-92b*, hsa-let-7c, hsa-let-7g, hsa-miR-145, hsa-let-7i, hsa-miR-130b, and hsa-miR-1260;
B33: hsa-let-7g, hsa-miR-145, hsa-let-7i, hsa-miR-130b, hsa-miR-1260, hsa-miR-330-3p, hsa-miR-30e, hsa-let-7d, hsa-miR-501-3p, and hsa-miR-1913.

6. The method of any one of claims 1 to 4, wherein said predetermined set of miRNAs comprises the miRNAs hsa-miR-145, hsa-miR-186, hsa-miR-664, hsa-miR-584, hsa-miR-20b, hsa-miR-223 and hsa-miR-422a.

7. The method of any one of claims 1 to 4, wherein said predetermined set of miRNAs comprises the miRNAs hsa-miR-145, hsa-miR-186, hsa-miR-664, hsa-miR-584, hsa-miR-20b, hsa-miR-223, hsa-miR-422a, hsa-miR-142-3p, hsa-let-7c, hsa-miR-151-3p.

8. Use of a kit for diagnosing multiple sclerosis, the kit comprising
(a) means for determining an expression profile of a predetermined set of miRNAs in a blood sample from a patient, said predetermined set of miRNAs comprising hsa-miR-145, and
(b) a reference expression profile of said predetermined set of miRNAs in a blood sample from a healthy subject,
wherein said blood sample is selected from whole blood, blood cells, plasma, and/or serum or samples comprising erythrocytes, leukocytes or thrombocytes, and wherein the predetermined set of miRNAs is selected from the group consisting of miRNAs as defined in any one of claims 5 to 7.

9. The use of claim 8, wherein the kit comprises means for performing a nucleic acid detection based on hybridisation and optionally amplification such as PCR, RT-PCR or qRT-PCR.

10. Use of a set of oligo- or polynucleotides comprising the sequences of a set of miRNAs as defined in any one of claims 1 to 7, and/or the complement thereof for diagnosing multiple sclerosis.

## Patentansprüche

1. Verfahren zur Diagnose von Multipler Sklerose umfassend die Schritte:
(a) Ermitteln eines Expressionsprofils eines vorbestimmten Sets von miRNAs in einer Blutprobe eines Patienten, wobei das vorbestimmte Set von miRNAs hsa-miR-145 umfasst,
(b) Vergleichen des Expressionsprofils mit einem Referenzexpressionsprofil, wobei der Vergleich des ermittelten Expressionsprofils mit dem Referenzexpressionsprofil die Diagnose von Multipler Sklerose erlaubt.

2. Verfahren nach Anspruch 1, wobei die Blutprobe aus Vollblut, Blutzellen, Plasma und/oder Serum oder aus Proben, die Erythrozyten, Leukozyten oder Thrombozyten umfassen, ausgewählt ist.

3. Verfahren nach einem der Ansprüche 1 bis 2, wobei das vorbestimmte Set von miRNAs eine oder mehrere miRNAs umfasst, die dafür bekannt sind, dass sie differentiell in dem zu diagnostizierenden Krankheitszustand exprimiert sind.

4. Verfahren nach einem der Ansprüche 1 bis 3, wobei die Ermittlung eines Expressionsprofils in Schritt (a) Nukleinsäurehybridisierung, Nukleinsäureamplifikation, Polymeraseverlängerung, Sequenzierung, Massenspektrometrie oder jeder Kombination davon umfasst.

5. Verfahren nach einem der Ansprüche 1 bis 4, wobei das vorbestimmte Set von miRNAs eine Signatur ausgewählt aus der Gruppe:
A4: hsa-let-7c, hsa-miR-145 und hsa-miR-330-3p;
A19: hsa-miR-92b*, hsa-let-7c, hsa-miR-145, hsa-miR-330-3p und hsa-miR-30e;
A41: hsa-miR-1234, hsa-miR-92b*, hsa-miR-145, hsa-miR-30e und hsa-miR-223;
B6: hsa-let-7c, hsa-let-7g und hsa-miR-145;
B7: hsa-miR-145, hsa-let-7i und hsa-miR-130b;
A18: hsa-miR-125a-5p, hsa-miR-92b*, hsa-let-7c, hsa-miR-145 und hsa-miR-330-3p;
A20: hsa-miR-145, hsa-miR-330-3p, hsa-miR-30e, hsa-miR-199a-5p und hsa-miR-223;
A31: hsa-let-7b, hsa-miR-574-3p, hsa-miR-500, hsa-miR-1234, hsa-miR-125a-5p, hsa-miR-92b*, hsa-let-7c, hsa-miR-145, hsa-miR-330-3p und hsa-miR-30e;
A32: hsa-miR-92b*, hsa-let-7c, hsa-miR-145, hsa-miR-330-3p, hsa-miR-30e, hsa-miR-199a-Sp, hsa-miR-223, hsa-miR-195, hsa-miR-182, hsa-miR-107 und hsa-miR-497;
A42 : hsa-miR-145, hsa-miR-30e, hsa-miR-223, hsa-miR-182 und hsa-miR-497;
B21: hsa-miR-92b*, hsa-let-7c, hsa-let-7g, hsa-miR-145 und hsa-let-7i;
B22: hsa-let-7c, hsa-let-7g, hsa-miR-145, hsa-let-7i, hsa-miR-130b und hsa-miR-1260;
B32: hsa-let-7e, hsa-miR-1234, hsa-miR-125a-5p, hsa-let-7f, hsa-miR-92b*, hsa-let-7c, hsa-let-7g, hsa-miR-145, hsa-let-7i, hsa-miR-130b und hsa-miR-1260;
B33: hsa-let-7g, hsa-miR-145, hsa-let-7i, hsa-miR-130b, hsa-miR-1260, hsa-miR-330-3p, hsa-miR-30e, hsa-let-7d, hsa-miR-501-3p und hsa-miR-1913 umfasst.

6. Verfahren nach einem der Ansprüche 1 bis 4, wobei das vorbestimmte Set von miRNAs die miRNAs hsa-miR-145, hsa-miR-186, hsa-miR-664, hsa-miR-584, hsa-miR-20b, hsa-miR-223 und hsa-miR-422a umfasst.

7. Verfahren nach einem der Ansprüche 1 bis 4, wobei das vorbestimmte Set von miRNAs die miRNAs hsa-miR-145, hsa-miR-186, hsa-miR-664, hsa-miR-584, hsa-miR-20b, hsa-miR-223, hsa-miR-422a, hsa-miR-142-3p, hsa-let-7c und hsa-miR-151-3p umfasst.

8. Verwendung eines Kits zur Diagnose von Multipler Sklerose, wobei das Kit umfasst
(a) Mittel zur Ermittlung eines Expressionsprofils eines vorbestimmten Sets von miRNAs in einer Blutprobe eines Patienten, wobei das vorbestimmte Set von miRNAs hsa-miR-145 umfasst und
(b) ein Referenzexpressionsprofil eines vorbestimmten Sets von miRNAs in einer Blutprobe eines gesunden Subjekts,
wobei die Blutprobe ausgewählt ist aus Vollblut, Blutzellen, Plasma und/oder Serum oder aus Proben, die Erythrozyten, Leukozyten oder Thrombozyten umfassen und
wobei das vorbestimmte Set von miRNAs aus der Gruppe bestehend aus miRNAs, wie sie in einem der Ansprüche 5 bis 7 definiert sind, ausgewählt ist.

9. Verwendung nach Anspruch 8, wobei das Kit Mittel zur Durchführung einer Nukleinsäuredetektion basierend auf Hybridisierung und optional Amplifikation, wie PCR, RT-PCR oder qRT-PCR, umfasst.

10. Verwendung eines Sets von Oligo- oder Polynukleotiden umfassend die Sequenzen eines Sets von miRNAs, wie sie in einem der Ansprüche 1 bis 7 definiert sind, und/oder die Komplemente davon, zur Diagnose von Multipler Sklerose.

## Revendications

1. Procédé de diagnostic de la sclérose en plaques, comprenant les étapes de
(a) détermination d'un profil d'expression d'un ensemble prédéfini de miARN dans un échantillon de sang d'un patient ; ledit ensemble prédéfini de miARN comprenant hsa-miR-145
(b) comparaison dudit profil d'expression à un profil d'expression de référence,
ladite comparaison dudit profil d'expression déterminé audit profil d'expression de référence permettant le diagnostic de la sclérose en plaques.

2. Procédé selon la revendication 1, ledit échantillon de sang étant choisi parmi le sang entier, les cellules sanguines, le plasma et/ou le sérum, ou lesdits échantillons comprenant des érythrocytes, des leucocytes ou des thrombocytes.

3. Procédé selon l'une quelconque des revendications 1 à 2, ledit ensemble prédéfini de miARN comprenant un ou plusieurs miARN connus pour être exprimés de façon différentielle dans l'état pathologique à diagnostiquer.

4. Procédé selon l'une quelconque des revendications 1 à 3, ladite détermination d'un profil d'expression dans l'étape (a) comprenant l'hybridation d'acides nucléiques, l'amplification d'acides nucléiques, l'extension par polymérase, le séquençage, la spectroscopie de masse ou toutes les combinaisons de celles-ci.

5. Procédé selon l'une quelconque des revendications 1 à 4, ledit ensemble prédéfini de miARN comprenant une signature choisie dans le groupe constitué par
A4 : hsa-let-7c, hsa-miR-145 et hsa-miR-330-3p ;
A19 : hsa-miR-92b*, hsa-let-7c, hsa-miR-145, hsa-miR-330-3p et hsa-miR-30e ;
A41 : hsa-miR-1234, hsa-miR-92b*, hsa-miR-145, hsa-miR-30e et hsa-miR-223 ;
B6 : hsa-let-7c, hsa-let-7g et hsa-miR-145 ;
B7 : hsa-miR-145, hsa-let-7i et hsa-miR-130b ;
A18 : hsa-miR-125a-5p, hsa-miR-92b*, hsa-let-7c, hsa-miR-145 et hsa-miR-330-3p ;
A20 : hsa-miR-145, hsa-miR-330-3p, hsa-miR-30e, hsa-miR-199a-5p et hsa-miR-223 ;
A31 : hsa-let-7b, hsa-miR-574-3p, hsa-miR-500, hsa-miR-1234, hsa-miR-125a-5p, hsa-miR-92b*, hsa-let-7c, hsa-miR-145, hsa-miR-330-3p et hsa-miR-30e ;
A32: hsa-miR-92b*, hsa-let-7c, hsa-miR-145, hsa-miR-330-3p, hsa-miR-30e, hsa-miR-199a-5p, hsa-miR-223, hsa-miR-195, hsa-miR-182, hsa-miR-107 et hsa-miR-497 ;
A42 : hsa-miR-145, hsa-miR-30e, hsa-miR-223, hsa-miR-182 et hsa-miR-497 ;
B21 : hsa-miR-92b*, hsa-let-7c, hsa-let-7g, hsa-miR-145 et hsa-let-7i ;
B22 : hsa-let-7c, hsa-let-7g, hsa-miR-145, hsa-let-7i, hsa-miR-130b et hsa-miR-1260 ;
B32: hsa-let-7e, hsa-miR-1234, hsa-miR-125a-5p, hsa-let-7f, hsa-miR-92b*, hsa-let-7c, hsa-let-7g, hsa-miR-145, hsa-let-7i, hsa-miR-130b et hsa-miR-1260 ;
B33 : hsa-let-7g, hsa-miR-145, hsa-let-7i, hsa-miR-130b, hsa-miR-1260, hsa-miR-330-3p, hsa-miR-30e, hsa-let-7d, hsa-miR-501-3p et hsa-miR-1913.

6. Procédé selon l'une quelconque des revendications 1 à 4, ledit ensemble prédéfini de miARN comprenant les miARN hsa-miR-145, hsa-miR-186, hsa-miR-664, hsa-miR-584, hsa-miR-20b, hsa-miR-223 et hsa-miR-422a.

7. Procédé selon l'une quelconque des revendications 1 à 4, ledit ensemble prédéfini de miARN comprenant les miARN hsa-miR-145, hsa-miR-186, hsa-miR-664, hsa-miR-584, hsa-miR-20b, hsa-miR-223, hsa-miR-422a, hsa-miR-142-3p, hsa-let-7c et hsa-miR-151-3p.

8. Utilisation d'un kit permettant le diagnostic de la sclérose en plaques, ledit kit comprenant
(a) des moyens permettant de déterminer un profil d'expression d'un ensemble prédéfini de miARN dans un échantillon de sang d'un patient, ledit ensemble prédéfini de miARN comprenant hsa-miR-145, et
(b) un profil d'expression de référence dudit ensemble prédéfini de miARN dans un échantillon de sang d'un patient en bonne santé,
ledit échantillon de sang étant choisi parmi le sang entier, les cellules sanguines, le plasma et/ou le sérum ou lesdits échantillons comprenant des érythrocytes, des leucocytes ou des thrombocytes, et ledit ensemble prédéfini de miARN étant choisi dans le groupe constitué par les miARN tels que définis dans l'une quelconque des revendications 5 à 7.

9. Utilisation selon la revendication 8, ledit kit comprenant des moyens permettant d'effectuer une détection d'acides nucléiques basée sur une hybridation et éventuellement une amplification telle que la PCR, la RT-PCR ou la qRT-PCR.

10. Utilisation d'un ensemble d'oligo- ou poly-nucléotides comprenant les séquences d'un ensemble de miARN tel que défini dans l'une quelconque des revendications 1 à 7, et/ou le complément de celles-ci permettant le diagnostic de la sclérose en plaques.


### SEQUENCE LISTING

<110> febit holding GmbH
<120> Complex miRNA sets as novel biomarkers for pancreatic diseases
<130> 45828 miRNA fingerprints in the diagnosis of diseases
<160>866
<170> PatentIn version 3.5
<210> 1
   <211> 22
   <212> RNA
   <213> Homo sapiens
   <400> 1
   ucguaccgugaguaauaaugcg 22
<210> 2
   <211> 23
   <212> RNA
   <213> Homo sapiens
   <400> 2
   ugaggggcagagagcgagacuuu 23
<210> 3
   <211> 22
   <212> RNA
   <213> Homo sapiens
   <400> 3
   ugagguaguaguuugugcuguu 22
<210> 4
   <211> 22
   <212> RNA
   <213> Homo sapiens
   <400> 4
   agagguaguagguugcauaguu 22
<210> 5
   <211> 22
   <212> RNA
   <213> Homo sapiens
   <400> 5
   aagcugccaguugaagaacugu 22
<210> 6
   <211> 22
   <212> RNA
   <213> Homo sapiens
   <400> 6
   uagcagcacauaaugguuugug 22
<210> 7
   <211> 22
   <212> RNA
   <213> Homo sapiens
   <400> 7
   ugagguaguaaguuguauuguu 22
<210> 8
   <211> 23
   <212> RNA
   <213> Homo sapiens
   <400> 8
   ugugcaaaucuaugcaaaacuga 23
<210> 9
   <211> 23
   <212> RNA
   <213> Homo sapiens
   <400> 9
   ugagugugugugugugagugugu 23
<210> 10
   <211> 20
   <212> RNA
   <213> Homo sapiens
   <400> 10
   acugccccaggugcugcugg 20
<210> 11
   <211> 23
   <212> RNA
   <213> Homo sapiens
   <400> 11
   caaagugcucauagugcagguag 23
<210> 12
   <211> 22
   <212> RNA
   <213> Homo sapiens
   <400> 12
   cauugcacuugucucggucuga 22
<210> 13
   <211> 21
   <212> RNA
   <213> Homo sapiens
   <400> 13
   uagcagcacagaaauauuggc 21
<210> 14
   <211> 22
   <212> RNA
   <213> Homo sapiens
   <400> 14
   ugagguaggagguuguauaguu 22
<210> 15
   <211> 22
   <212> RNA
   <213> Homo sapiens
   <400> 15
   ugagguaguagguuguaugguu 22
<210> 16
   <211> 22
   <212> RNA
   <213> Homo sapiens
   <400> 16
   ugagguaguagauuguauaguu 22
<210> 17
   <211> 22
   <212> RNA
   <213> Homo sapiens
   <400> 17
   ugagguaguagguuguauaguu 22
<210> 18
   <211> 22
   <212> RNA
   <213> Homo sapiens
   <400> 18
   ugagguaguaguuuguacaguu 22
<210> 19
   <211> 21
   <212> RNA
   <213> Homo sapiens
   <400> 19
   uaccacaggguagaaccacgg 21
<210> 20
   <211> 23
   <212> RNA
   <213> Homo sapiens
   <400> 20
   ucccuguccuccaggagcucacg 23
<210> 21
   <211> 22
   <212> RNA
   <213> Homo sapiens
   <400> 21
   uuaucagaaucuccagggguac 22
<210> 22
   <211> 22
   <212> RNA
   <213> Homo sapiens
   <400> 22
   ucuacaaaggaaagcgcuuucu 22
<210> 23
   <211> 23
   <212> RNA
   <213> Homo sapiens
   <400> 23
   acugcccuaagugcuccuucugg 23
<210> 24
   <211> 21
   <212> RNA
   <213> Homo sapiens
   <400> 24
   uucaaguaauucaggauaggu 21
<210> 25
   <211> 19
   <212> RNA
   <213> Homo sapiens
   <400> 25
   aggcugcggaauucaggac 19
<210> 26
   <211> 23
   <212> RNA
   <213> Homo sapiens
   <400> 26
   agcucggucugaggccccucagu 23
<210> 27
   <211> 22
   <212> RNA
   <213> Homo sapiens
   <400> 27
   acugcugagcuagcacuucccg 22
<210> 28
   <211> 22
   <212> RNA
   <213> Homo sapiens
   <400> 28
   uagcaccaucugaaaucgguua 22
<210> 29
   <211> 27
   <212> RNA
   <213> Homo sapiens
   <400> 29
   accuucuuguauaagcacugugcuaaa 27
<210> 30
   <211> 22
   <212> RNA
   <213> Homo sapiens
   <400> 30
   cugugcgugugacagcggcuga 22
<210> 31
   <211> 23
   <212> RNA
   <213> Homo sapiens
   <400> 31
   ugugcaaauccaugcaaaacuga 23
<210> 32
   <211> 23
   <212> RNA
   <213> Homo sapiens
   <400> 32
   agguuguccguggugaguucgca 23
<210> 33
   <211> 21
   <212> RNA
   <213> Homo sapiens
   <400> 33
   cauuauuacuuuugguacgcg 21
<210> 34
   <211> 21
   <212> RNA
   <213> Homo sapiens
   <400> 34
   cucccacaugcaggguuugca 21
<210> 35
   <211> 22
   <212> RNA
   <213> Homo sapiens
   <400> 35
   uaguaccaguaccuuguguuca 22
<210> 36
   <211> 22
   <212> RNA
   <213> Homo sapiens
   <400> 36
   gggagccaggaaguauugaugu 22
<210> 37
   <211> 23
   <212> RNA
   <213> Homo sapiens
   <400> 37
   aaugacacgaucacucccguuga 23
<210> 38
   <211> 23
   <212> RNA
   <213> Homo sapiens
   <400> 38
   ugagcgccucgacgacagagccg 23
<210> 39
   <211> 23
   <212> RNA
   <213> Homo sapiens
   <400> 39
   ugucacucggcucggcccacuac 23
<210> 40
   <211> 22
   <212> RNA
   <213> Homo sapiens
   <400> 40
   cggguggaucacgaugcaauuu 22
<210> 41
   <211> 22
   <212> RNA
   <213> Homo sapiens
   <400> 41
   cgaaucauuauuugcugcucua 22
<210> 42
   <211> 22
   <212> RNA
   <213> Homo sapiens
   <400> 42
   ugaccgauuucuccugguguuc 22
<210> 43
   <211> 22
   <212> RNA
   <213> Homo sapiens
   <400> 43
   ugucuuacucccucaggcacau 22
<210> 44
   <211> 22
   <212> RNA
   <213> Homo sapiens
   <400> 44
   aaucacuaaccacacggccagg 22
<210> 45
   <211> 23
   <212> RNA
   <213> Homo sapiens
   <400> 45
   uaaagugcuuauagugcagguag 23
<210> 46
   <211> 22
   <212> RNA
   <213> Homo sapiens
   <400> 46
   uuauaauacaaccugauaagug 22
<210> 47
   <211> 22
   <212> RNA
   <213> Homo sapiens
   <400> 47
   ggauuccuggaaauacuguucu 22
<210> 48
   <211> 23
   <212> RNA
   <213> Homo sapiens
   <400> 48
   uaagugcuuccauguuuuaguag 23
<210> 49
   <211> 23
   <212> RNA
   <213> Homo sapiens
   <400> 49
   aaaagugcuuacagugcagguag 23
<210> 50
   <211> 22
   <212> RNA
   <213> Homo sapiens
   <400> 50
   uguaaacauccuugacuggaag 22
<210> 51
   <211> 22
   <212> RNA
   <213> Homo sapiens
   <400> 51
   ugucaguuugucaaauacccca 22
<210> 52
   <211> 22
   <212> RNA
   <213> Homo sapiens
   <400> 52
   cuggacugagccgugcuacugg 22
<210> 53
   <211> 22
   <212> RNA
   <213> Homo sapiens
   <400> 53
   ugagguaguagguugugugguu 22
<210> 54
   <211> 22
   <212> RNA
   <213> Homo sapiens
   <400> 54
   ugugacagauugauaacugaaa 22
<210> 55
   <211> 18
   <212> RNA
   <213> Homo sapiens
   <400> 55
   ugcuuccuuucagagggu 18
<210> 56
   <211> 22
   <212> RNA
   <213> Homo sapiens
   <400> 56
   aaaccguuaccauuacugaguu 22
<210> 57
   <211> 22
   <212> RNA
   <213> Homo sapiens
   <400> 57
   aaagugcaucuuuuuagaggau 22
<210> 58
   <211> 26
   <212> RNA
   <213> Homo sapiens
   <400> 58
   aaguaguugguuuguaugagaugguu 26
<210> 59
   <211> 23
   <212> RNA
   <213> Homo sapiens
   <400> 59
   gacacgggcgacagcugcggccc 23
<210> 60
   <211> 23
   <212> RNA
   <213> Homo sapiens
   <400> 60
   ucccccaggugugauucugauuu 23
<210> 61
   <211> 22
   <212> RNA
   <213> Homo sapiens
   <400> 61
   aguuuugcauaguugcacuaca 22
<210> 62
   <211> 22
   <212> RNA
   <213> Homo sapiens
   <400> 62
   aucaugaugggcuccucggugu 22
<210> 63
   <211> 22
   <212> RNA
   <213> Homo sapiens
   <400> 63
   cuccugagccauucugagccuc 22
<210> 64
   <211> 22
   <212> RNA
   <213> Homo sapiens
   <400> 64
   aaaaugguucccuuuagagugu 22
<210> 65
   <211> 22
   <212> RNA
   <213> Homo sapiens
   <400> 65
   aagugcuuccuuuuagaggguu 22
<210> 66
   <211> 22
   <212> RNA
   <213> Homo sapiens
   <400> 66
   cucuagagggaagcgcuuucug 22
<210> 67
   <211> 21
   <212> RNA
   <213> Homo sapiens
   <400> 67
   cugaccuaugaauugacagcc 21
<210> 68
   <211> 21
   <212> RNA
   <213> Homo sapiens
   <400> 68
   aagugaucuaaaggccuacau 21
<210> 69
   <211> 21
   <212> RNA
   <213> Homo sapiens
   <400> 69
   ucgaggagcucacagucuagu 21
<210> 70
   <211> 21
   <212> RNA
   <213> Homo sapiens
   <400> 70
   uggacugcccugaucuggaga 21
<210> 71
   <211> 23
   <212> RNA
   <213> Homo sapiens
   <400> 71
   uagcagcgggaacaguucugcag 23
<210> 72
   <211> 19
   <212> RNA
   <213> Homo sapiens
   <400> 72
   agguugacauacguuuccc 19
<210> 73
   <211> 22
   <212> RNA
   <213> Homo sapiens
   <400> 73
   gguggcccggccgugccugagg 22
<210> 74
   <211> 22
   <212> RNA
   <213> Homo sapiens
   <400> 74
   cuauacgaccugcugccuuucu 22
<210> 75
   <211> 23
   <212> RNA
   <213> Homo sapiens
   <400> 75
   cccaguguucagacuaccuguuc 23
<210> 76
   <211> 17
   <212> RNA
   <213> Homo sapiens
   <400> 76
   ucucgcuggggccucca 17
<210> 77
   <211> 19
   <212> RNA
   <213> Homo sapiens
   <400> 77
   aauggauuuuuggagcagg 19
<210> 78
   <211> 22
   <212> RNA
   <213> Homo sapiens
   <400> 78
   aacaauauccuggugcugagug 22
<210> 79
   <211> 21
   <212> RNA
   <213> Homo sapiens
   <400> 79
   auguaugugugcaugugcaug 21
<210> 80
   <211> 19
   <212> RNA
   <213> Homo sapiens
   <400> 80
   auggauaaggcuuuggcuu 19
<210> 81
   <211> 23
   <212> RNA
   <213> Homo sapiens
   <400> 81
   gcagcagagaauaggacuacguc 23
<210> 82
   <211> 22
   <212> RNA
   <213> Homo sapiens
   <400> 82
   uggagagaaaggcaguuccuga 22
<210> 83
   <211> 23
   <212> RNA
   <213> Homo sapiens
   <400> 83
   gcgaggaccccucggggucugac 23
<210> 84
   <211> 26
   <212> RNA
   <213> Homo sapiens
   <400> 84
   gaugaugauggcagcaaauucugaaa 26
<210> 85
   <211> 22
   <212> RNA
   <213> Homo sapiens
   <400> 85
   uucuggaauucugugugaggga 22
<210> 86
   <211> 22
   <212> RNA
   <213> Homo sapiens
   <400> 86
   uuuuucauuauugcuccugacc 22
<210> 87
   <211> 21
   <212> RNA
   <213> Homo sapiens
   <400> 87
   cagcagcacacugugguuugu 21
<210> 88
   <211> 18
   <212> RNA
   <213> Homo sapiens
   <400> 88
   ucagcuggcccucauuuc 18
<210> 89
   <211> 22
   <212> RNA
   <213> Homo sapiens
   <400> 89
   uagcagcacguaaauauuggcg 22
<210> 90
   <211> 22
   <212> RNA
   <213> Homo sapiens
   <400> 90
   uggaauguaaagaaguauguau 22
<210> 91
   <211> 24
   <212> RNA
   <213> Homo sapiens
   <400> 91
   uggcccugacugaagaccagcagu 24
<210> 92
   <211> 22
   <212> RNA
   <213> Homo sapiens
   <400> 92
   gcuauuucacgacaccaggguu 22
<210> 93
   <211> 23
   <212> RNA
   <213> Homo sapiens
   <400> 93
   acuccauuuguuuugaugaugga 23
<210> 94
   <211> 22
   <212> RNA
   <213> Homo sapiens
   <400> 94
   caaaaaccacaguuucuuuugc 22
<210> 95
   <211> 22
   <212> RNA
   <213> Homo sapiens
   <400> 95
   caaaguuuaagauccuugaagu 22
<210> 96
   <211> 22
   <212> RNA
   <213> Homo sapiens
   <400> 96
   aaaaguaaucgcgguuuuuguc 22
<210> 97
   <211> 21
   <212> RNA
   <213> Homo sapiens
   <400> 97
   gccccugggccuauccuagaa 21
<210> 98
   <211> 22
   <212> RNA
   <213> Homo sapiens
   <400> 98
   gcccaaaggugaauuuuuuggg 22
<210> 99
   <211> 23
   <212> RNA
   <213> Homo sapiens
   <400> 99
   guccaguuuucccaggaaucccu 23
<210> 100
   <211> 23
   <212> RNA
   <213> Homo sapiens
   <400> 100
   caaagugcuuacagugcagguag 23
<210> 101
   <211> 22
   <212> RNA
   <213> Homo sapiens
   <400> 101
   uguaaacauccuacacucagcu 22
<210> 102
   <211> 22
   <212> RNA
   <213> Homo sapiens
   <400> 102
   cuauacaaucuauugccuuccc 22
<210> 103
   <211> 22
   <212> RNA
   <213> Homo sapiens
   <400> 103
   uuuucaacucuaaugggagaga 22
<210> 104
   <211> 21
   <212> RNA
   <213> Homo sapiens
   <400> 104
   cuuuuugcggucugggcuugc 21
<210> 105
   <211> 21
   <212> RNA
   <213> Homo sapiens
   <400> 105
   ucguggccuggucuccauuau 21
<210> 106
   <211> 22
   <212> RNA
   <213> Homo sapiens
   <400> 106
   ccgcacuguggguacuugcugc 22
<210> 107
   <211> 24
   <212> RNA
   <213> Homo sapiens
   <400> 107
   gaccuggacauguuugugcccagu 24
<210> 108
   <211> 22
   <212> RNA
   <213> Homo sapiens
   <400> 108
   caaucagcaaguauacugcccu 22
<210> 109
   <211> 21
   <212> RNA
   <213> Homo sapiens
   <400> 109
   aauggcgccacuaggguugug 21
<210> 110
   <211> 22
   <212> RNA
   <213> Homo sapiens
   <400> 110
   aggcauugacuucucacuagcu 22
<210> 111
   <211> 22
   <212> RNA
   <213> Homo sapiens
   <400> 111
   acuguaguaugggcacuuccag 22
<210> 112
   <211> 21
   <212> RNA
   <213> Homo sapiens
   <400> 112
   caaaacgugaggcgcugcuau 21
<210> 113
   <211> 22
   <212> RNA
   <213> Homo sapiens
   <400> 113
   aucgugcaucccuuuagagugu 22
<210> 114
   <211> 22
   <212> RNA
   <213> Homo sapiens
   <400> 114
   ucuauacagacccuggcuuuuc 22
<210> 115
   <211> 22
   <212> RNA
   <213> Homo sapiens
   <400> 115
   acaguagucugcacauugguua 22
<210> 116
   <211> 20
   <212> RNA
   <213> Homo sapiens
   <400> 116
   guugugucaguuuaucaaac 20
<210> 117
   <211> 21
   <212> RNA
   <213> Homo sapiens
   <400> 117
   uaguagaccguauagcguacg 21
<210> 118
   <211> 21
   <212> RNA
   <213> Homo sapiens
   <400> 118
   aucacauugccagggauuacc 21
<210> 119
   <211> 21
   <212> RNA
   <213> Homo sapiens
   <400> 119
   uugggacauacuuaugcuaaa 21
<210> 120
   <211> 22
   <212> RNA
   <213> Homo sapiens
   <400> 120
   uggcaguguauuguuagcuggu 22
<210> 121
   <211> 19
   <212> RNA
   <213> Homo sapiens
   <400> 121
   aaaaacuguaauuacuuuu 19
<210> 122
   <211> 22
   <212> RNA
   <213> Homo sapiens
   <400> 122
   ugugucacucgaugaccacugu 22
<210> 123
   <211> 22
   <212> RNA
   <213> Homo sapiens
   <400> 123
   cacacacugcaauuacuuuugc 22
<210> 124
   <211> 22
   <212> RNA
   <213> Homo sapiens
   <400> 124
   acccgucccguucguccccgga 22
<210> 125
   <211> 21
   <212> RNA
   <213> Homo sapiens
   <400> 125
   uccugcgcgucccagaugccc 21
<210> 126
   <211> 23
   <212> RNA
   <213> Homo sapiens
   <400> 126
   ugaguaccgccaugucuguuggg 23
<210> 127
   <211> 23
   <212> RNA
   <213> Homo sapiens
   <400> 127
   ccuaguagguguccaguaagugu 23
<210> 128
   <211> 23
   <212> RNA
   <213> Homo sapiens
   <400> 128
   agguuacccgagcaacuuugcau 23
<210> 129
   <211> 24
   <212> RNA
   <213> Homo sapiens
   <400> 129
   uuuggcaaugguagaacucacacu 24
<210> 130
   <211> 25
   <212> RNA
   <213> Homo sapiens
   <400> 130
   ggcggagggaaguagguccguuggu 25
<210> 131
   <211> 21
   <212> RNA
   <213> Homo sapiens
   <400> 131
   augaccuaugaauugacagac 21
<210> 132
   <211> 22
   <212> RNA
   <213> Homo sapiens
   <400> 132
   gugugcggaaaugcuucugcua 22
<210> 133
   <211> 22
   <212> RNA
   <213> Homo sapiens
   <400> 133
   uguaaacauccccgacuggaag 22
<210> 134
   <211> 22
   <212> RNA
   <213> Homo sapiens
   <400> 134
   cuccugacuccagguccugugu 22
<210> 135
   <211> 22
   <212> RNA
   <213> Homo sapiens
   <400> 135
   accuggcauacaauguagauuu 22
<210> 136
   <211> 22
   <212> RNA
   <213> Homo sapiens
   <400> 136
   caaucacuaacuccacugccau 22
<210> 137
   <211> 25
   <212> RNA
   <213> Homo sapiens
   <400> 137
   aggcaccagccaggcauugcucagc 25
<210> 138
   <211> 21
   <212> RNA
   <213> Homo sapiens
   <400> 138
   aacaggugacugguuagacaa 21
<210> 139
   <211> 21
   <212> RNA
   <213> Homo sapiens
   <400> 139
   acuggacuuggagucagaagg 21
<210> 140
   <211> 22
   <212> RNA
   <213> Homo sapiens
   <400> 140
   ggugcagugcugcaucucuggu 22
<210> 141
   <211> 23
   <212> RNA
   <213> Homo sapiens
   <400> 141
   ccucagggcuguagaacagggcu 23
<210> 142
   <211> 21
   <212> RNA
   <213> Homo sapiens
   <400> 142
   gcuaguccugacucagccagu 21
<210> 143
   <211> 21
   <212> RNA
   <213> Homo sapiens
   <400> 143
   agaccuggcccagaccucagc 21
<210> 144
   <211> 20
   <212> RNA
   <213> Homo sapiens
   <400> 144
   aggguguuucucucaucucu 20
<210> 145
   <211> 23
   <212> RNA
   <213> Homo sapiens
   <400> 145
   uguaaacauccuacacucucagc 23
<210> 146
   <211> 22
   <212> RNA
   <213> Homo sapiens
   <400> 146
   aaggagcucacagucuauugag 22
<210> 147
   <211> 21
   <212> RNA
   <213> Homo sapiens
   <400> 147
   aucacauugccagggauuucc 21
<210> 148
   <211> 19
   <212> RNA
   <213> Homo sapiens
   <400> 148
   ucuaggcugguacugcuga 19
<210> 149
   <211> 21
   <212> RNA
   <213> Homo sapiens
   <400> 149
   guggcugcacucacuuccuuc 21
<210> 150
   <211> 22
   <212> RNA
   <213> Homo sapiens
   <400> 150
   acuuuaacauggaagugcuuuc 22
<210> 151
   <211> 21
   <212> RNA
   <213> Homo sapiens
   <400> 151
   guucaaauccagaucuauaac 21
<210> 152
   <211> 23
   <212> RNA
   <213> Homo sapiens
   <400> 152
   uggaguccaggaaucugcauuuu 23
<210> 153
   <211> 24
   <212> RNA
   <213> Homo sapiens
   <400> 153
   ccagacagaauucuaugcacuuuc 24
<210> 154
   <211> 23
   <212> RNA
   <213> Homo sapiens
   <400> 154
   uaaauuucaccuuucugagaagg 23
<210> 155
   <211> 24
   <212> RNA
   <213> Homo sapiens
   <400> 155
   uggggagcugaggcucugggggug 24
<210> 156
   <211> 23
   <212> RNA
   <213> Homo sapiens
   <400> 156
   uagcaccauuugaaaucaguguu 23
<210> 157
   <211> 20
   <212> RNA
   <213> Homo sapiens
   <400> 157
   accaggaggcugaggccccu 20
<210> 158
   <211> 23
   <212> RNA
   <213> Homo sapiens
   <400> 158
   uaaggugcaucuagugcagauag 23
<210> 159
   <211> 19
   <212> RNA
   <213> Homo sapiens
   <400> 159
   gugaggacucgggaggugg 19
<210> 160
   <211> 22
   <212> RNA
   <213> Homo sapiens
   <400> 160
   caaauucguaucuaggggaaua 22
<210> 161
   <211> 22
   <212> RNA
   <213> Homo sapiens
   <400> 161
   accaucgaccguugauuguacc 22
<210> 162
   <211> 22
   <212> RNA
   <213> Homo sapiens
   <400> 162
   caugguucugucaagcaccgcg 22
<210> 163
   <211> 23
   <212> RNA
   <213> Homo sapiens
   <400> 163
   aagugccgccaucuuuugagugu 23
<210> 164
   <211> 22
   <212> RNA
   <213> Homo sapiens
   <400> 164
   aucacacaaaggcaacuuuugu 22
<210> 165
   <211> 22
   <212> RNA
   <213> Homo sapiens
   <400> 165
   cagugguuuuacccuaugguag 22
<210> 166
   <211> 23
   <212> RNA
   <213> Homo sapiens
   <400> 166
   cagugcaauaguauugucaaagc 23
<210> 167
   <211> 22
   <212> RNA
   <213> Homo sapiens
   <400> 167
   uacguagauauauauguauuuu 22
<210> 168
   <211> 22
   <212> RNA
   <213> Homo sapiens
   <400> 168
   uucacauugugcuacugucugc 22
<210> 169
   <211> 22
   <212> RNA
   <213> Homo sapiens
   <400> 169
   uacccagagcaugcagugugaa 22
<210> 170
   <211> 22
   <212> RNA
   <213> Homo sapiens
   <400> 170
   aacuggcccucaaagucccgcu 22
<210> 171
   <211> 22
   <212> RNA
   <213> Homo sapiens
   <400> 171
   ugccugucuacacuugcugugc 22
<210> 172
   <211> 22
   <212> RNA
   <213> Homo sapiens
   <400> 172
   aaaucucugcaggcaaauguga 22
<210> 173
   <211> 17
   <212> RNA
   <213> Homo sapiens
   <400> 173
   uaauugcuuccauguuu 17
<210> 174
   <211> 22
   <212> RNA
   <213> Homo sapiens
   <400> 174
   cucuagagggaagcgcuuucug 22
<210> 175
   <211> 22
   <212> RNA
   <213> Homo sapiens
   <400> 175
   aaaaguaauugcggucuuuggu 22
<210> 176
   <211> 20
   <212> RNA
   <213> Homo sapiens
   <400> 176
   auguauaaauguauacacac 20
<210> 177
   <211> 19
   <212> RNA
   <213> Homo sapiens
   <400> 177
   aagugugcagggcacuggu 19
<210> 178
   <211> 21
   <212> RNA
   <213> Homo sapiens
   <400> 178
   ucccacguuguggcccagcag 21
<210> 179
   <211> 21
   <212> RNA
   <213> Homo sapiens
   <400> 179
   agcuacaucuggcuacugggu 21
<210> 180
   <211> 22
   <212> RNA
   <213> Homo sapiens
   <400> 180
   ugcuggaucagugguucgaguc 22
<210> 181
   <211> 22
   <212> RNA
   <213> Homo sapiens
   <400> 181
   ugcaacuuaccugagucauuga 22
<210> 182
   <211> 23
   <212> RNA
   <213> Homo sapiens
   <400> 182
   ucucacacagaaaucgcacccgu 23
<210> 183
   <211> 22
   <212> RNA
   <213> Homo sapiens
   <400> 183
   aagugcugucauagcugagguc 22
<210> 184
   <211> 23
   <212> RNA
   <213> Homo sapiens
   <400> 184
   aucccuugcaggggcuguugggu 23
<210> 185
   <211> 22
   <212> RNA
   <213> Homo sapiens
   <400> 185
   auaagacgaacaaaagguuugu 22
<210> 186
   <211> 22
   <212> RNA
   <213> Homo sapiens
   <400> 186
   ccaguauuaacugugcugcuga 22
<210> 187
   <211> 21
   <212> RNA
   <213> Homo sapiens
   <400> 187
   gcgacccauacuugguuucag 21
<210> 188
   <211> 22
   <212> RNA
   <213> Homo sapiens
   <400> 188
   ugcccuguggacucaguucugg 22
<210> 189
   <211> 21
   <212> RNA
   <213> Homo sapiens
   <400> 189
   aaagugcuuccuuuuagaggg 21
<210> 190
   <211> 22
   <212> RNA
   <213> Homo sapiens
   <400> 190
   aggcaguguauuguuagcuggc 22
<210> 191
   <211> 24
   <212> RNA
   <213> Homo sapiens
   <400> 191
   acaaagugcuucccuuuagagugu 24
<210> 192
   <211> 22
   <212> RNA
   <213> Homo sapiens
   <400> 192
   ugccuacugagcugaaacacag 22
<210> 193
   <211> 21
   <212> RNA
   <213> Homo sapiens
   <400> 193
   gaaagcgcuucucuuuagagg 21
<210> 194
   <211> 22
   <212> RNA
   <213> Homo sapiens
   <400> 194
   aaaccuguguuguucaagaguc 22
<210> 195
   <211> 22
   <212> RNA
   <213> Homo sapiens
   <400> 195
   uauugcacauuacuaaguugca 22
<210> 196
   <211> 21
   <212> RNA
   <213> Homo sapiens
   <400> 196
   cuagacugaagcuccuugagg 21
<210> 197
   <211> 23
   <212> RNA
   <213> Homo sapiens
   <400> 197
   uagugcaauauugcuuauagggu 23
<210> 198
   <211> 21
   <212> RNA
   <213> Homo sapiens
   <400> 198
   uacaguacugugauaacugaa 21
<210> 199
   <211> 23
   <212> RNA
   <213> Homo sapiens
   <400> 199
   aguuuugcagguuugcauccagc 23
<210> 200
   <211> 21
   <212> RNA
   <213> Homo sapiens
   <400> 200
   uacugcagacaguggcaauca 21
<210> 201
   <211> 20
   <212> RNA
   <213> Homo sapiens
   <400> 201
   uacaguauagaugauguacu 20
<210> 202
   <211> 23
   <212> RNA
   <213> Homo sapiens
   <400> 202
   uacuccagagggcgucacucaug 23
<210> 203
   <211> 21
   <212> RNA
   <213> Homo sapiens
   <400> 203
   aguuaaugaauccuggaaagu 21
<210> 204
   <211> 23
   <212> RNA
   <213> Homo sapiens
   <400> 204
   ugugcuugcucgucccgcccgca 23
<210> 205
   <211> 22
   <212> RNA
   <213> Homo sapiens
   <400> 205
   auccgcgcucugacucucugcc 22
<210> 206
   <211> 23
   <212> RNA
   <213> Homo sapiens
   <400> 206
   ugucugcccgcaugccugccucu 23
<210> 207
   <211> 21
   <212> RNA
   <213> Homo sapiens
   <400> 207
   uaaggcacccuucugaguaga 21
<210> 208
   <211> 22
   <212> RNA
   <213> Homo sapiens
   <400> 208
   uauguaacaugguccacuaacu 22
<210> 209
   <211> 23
   <212> RNA
   <213> Homo sapiens
   <400> 209
   ccugcagcgacuugauggcuucc 23
<210> 210
   <211> 23
   <212> RNA
   <213> Homo sapiens
   <400> 210
   uucauuugguauaaaccgcgauu 23
<210> 211
   <211> 22
   <212> RNA
   <213> Homo sapiens
   <400> 211
   uggguuccuggcaugcugauuu 22
<210> 212
   <211> 22
   <212> RNA
   <213> Homo sapiens
   <400> 212
   augggugaauuuguagaaggau 22
<210> 213
   <211> 22
   <212> RNA
   <213> Homo sapiens
   <400> 213
   uugcauagucacaaaagugauc 22
<210> 214
   <211> 21
   <212> RNA
   <213> Homo sapiens
   <400> 214
   aaagugcuuccuuuuugaggg 21
<210> 215
   <211> 19
   <212> RNA
   <213> Homo sapiens
   <400> 215
   gugucugcuuccuguggga 19
<210> 216
   <211> 22
   <212> RNA
   <213> Homo sapiens
   <400> 216
   cugcaauguaagcacuucuuac 22
<210> 217
   <211> 22
   <212> RNA
   <213> Homo sapiens
   <400> 217
   ugcuaugccaacauauugccau 22
<210> 218
   <211> 22
   <212> RNA
   <213> Homo sapiens
   <400> 218
   cagugccucggcagugcagccc 22
<210> 219
   <211> 22
   <212> RNA
   <213> Homo sapiens
   <400> 219
   uaugucugcugaccaucaccuu 22
<210> 220
   <211> 22
   <212> RNA
   <213> Homo sapiens
   <400> 220
   caagcucgugucuguggguccg 22
<210> 221
   <211> 22
   <212> RNA
   <213> Homo sapiens
   <400> 221
   uaguacugugcauaucaucuau 22
<210> 222
   <211> 23
   <212> RNA
   <213> Homo sapiens
   <400> 222
   uauggcuuuucauuccuauguga 23
<210> 223
   <211> 22
   <212> RNA
   <213> Homo sapiens
   <400> 223
   uagaguuacacccugggaguua 22
<210> 224
   <211> 21
   <212> RNA
   <213> Homo sapiens
   <400> 224
   cuccguuugccuguuucgcug 21
<210> 225
   <211> 22
   <212> RNA
   <213> Homo sapiens
   <400> 225
   cuuagcagguuguauuaucauu 22
<210> 226
   <211> 21
   <212> RNA
   <213> Homo sapiens
   <400> 226
   auugacacuucugugaguaga 21
<210> 227
   <211> 21
   <212> RNA
   <213> Homo sapiens
   <400> 227
   uaauuuuauguauaagcuagu 21
<210> 228
   <211> 21
   <212> RNA
   <213> Homo sapiens
   <400> 228
   aaacuacugaaaaucaaagau 21
<210> 229
   <211> 21
   <212> RNA
   <213> Homo sapiens
   <400> 229
   aauaauacaugguugaucuuu 21
<210> 230
   <211> 21
   <212> RNA
   <213> Homo sapiens
   <400> 230
   uugaaaggcuauuucuugguc 21
<210> 231
   <211> 21
   <212> RNA
   <213> Homo sapiens
   <400> 231
   ucacagugaaccggucucuuu 21
<210> 232
   <211> 24
   <212> RNA
   <213> Homo sapiens
   <400> 232
   aauccuuggaaccuaggugugagu 24
<210> 233
   <211> 23
   <212> RNA
   <213> Homo sapiens
   <400> 233
   aggaagcccuggaggggcuggag 23
<210> 234
   <211> 22
   <212> RNA
   <213> Homo sapiens
   <400> 234
   cugcccuggcccgagggaccga 22
<210> 235
   <211> 20
   <212> RNA
   <213> Homo sapiens
   <400> 235
   caccaggcauuguggucucc 20
<210> 236
   <211> 25
   <212> RNA
   <213> Homo sapiens
   <400> 236
   ugggaacggguuccggcagacgcug 25
<210> 237
   <211> 22
   <212> RNA
   <213> Homo sapiens
   <400> 237
   uguaacagcaacuccaugugga 22
<210> 238
   <211> 22
   <212> RNA
   <213> Homo sapiens
   <400> 238
   uagcagcacaucaugguuuaca 22
<210> 239
   <211> 21
   <212> RNA
   <213> Homo sapiens
   <400> 239
   aggggugcuaucugugauuga 21
<210> 240
   <211> 22
   <212> RNA
   <213> Homo sapiens
   <400> 240
   ucacaagucaggcucuugggac 22
<210> 241
   <211> 17
   <212> RNA
   <213> Homo sapiens
   <400> 241
   uucaaguaauucaggug 17
<210> 242
   <211> 22
   <212> RNA
   <213> Homo sapiens
   <400> 242
   ugugcgcagggagaccucuccc 22
<210> 243
   <211> 22
   <212> RNA
   <213> Homo sapiens
   <400> 243
   uuguacaugguaggcuuucauu 22
<210> 244
   <211> 23
   <212> RNA
   <213> Homo sapiens
   <400> 244
   ucaaaugcucagacuccuguggu 23
<210> 245
   <211> 22
   <212> RNA
   <213> Homo sapiens
   <400> 245
   uaacacugucugguaaagaugg 22
<210> 246
   <211> 22
   <212> RNA
   <213> Homo sapiens
   <400> 246
   aaccaucgaccguugaguggac 22
<210> 247
   <211> 22
   <212> RNA
   <213> Homo sapiens
   <400> 247
   aacuggccuacaaagucccagu 22
<210> 248
   <211> 23
   <212> RNA
   <213> Homo sapiens
   <400> 248
   uaagugcuuccauguuucagugg 23
<210> 249
   <211> 22
   <212> RNA
   <213> Homo sapiens
   <400> 249
   agaggcuggccgugaugaauuc 22
<210> 250
   <211> 22
   <212> RNA
   <213> Homo sapiens
   <400> 250
   aacaucacagcaagucugugcu 22
<210> 251
   <211> 22
   <212> RNA
   <213> Homo sapiens
   <400> 251
   ucagcaaacauuuauugugugc 22
<210> 252
   <211> 22
   <212> RNA
   <213> Homo sapiens
   <400> 252
   aaaaguaauugugguuuuggcc 22
<210> 253
   <211> 21
   <212> RNA
   <213> Homo sapiens
   <400> 253
   ugacaacuauggaugagcucu 21
<210> 254
   <211> 22
   <212> RNA
   <213> Homo sapiens
   <400> 254
   auucuaauuucuccacgucuuu 22
<210> 255
   <211> 21
   <212> RNA
   <213> Homo sapiens
   <400> 255
   uagauaaaauauugguaccug 21
<210> 256
   <211> 21
   <212> RNA
   <213> Homo sapiens
   <400> 256
   caaagaggaaggucccauuac 21
<210> 257
   <211> 21
   <212> RNA
   <213> Homo sapiens
   <400> 257
   uuuccauaggugaugagucac 21
<210> 258
   <211> 21
   <212> RNA
   <213> Homo sapiens
   <400> 258
   cacaagguauugguauuaccu 21
<210> 259
   <211> 19
   <212> RNA
   <213> Homo sapiens
   <400> 259
   aagcagcugccucugaggc 19
<210> 260
   <211> 22
   <212> RNA
   <213> Homo sapiens
   <400> 260
   auaauacaugguuaaccucuuu 22
<210> 261
   <211> 22
   <212> RNA
   <213> Homo sapiens
   <400> 261
   uccauuacacuacccugccucu 22
<210> 262
   <211> 22
   <212> RNA
   <213> Homo sapiens
   <400> 262
   ccaguggggcugcuguuaucug 22
<210> 263
   <211> 22
   <212> RNA
   <213> Homo sapiens
   <400> 263
   ugguuuaccgucccacauacau 22
<210> 264
   <211> 22
   <212> RNA
   <213> Homo sapiens
   <400> 264
   cuccuauaugaugccuuucuuc 22
<210> 265
   <211> 22
   <212> RNA
   <213> Homo sapiens
   <400> 265
   ugaaggucuacugugugccagg 22
<210> 266
   <211> 22
   <212> RNA
   <213> Homo sapiens
   <400> 266
   caaaccacacugugguguuaga 22
<210> 267
   <211> 22
   <212> RNA
   <213> Homo sapiens
   <400> 267
   aaagugcauccuuuuagagugu 22
<210> 268
   <211> 22
   <212> RNA
   <213> Homo sapiens
   <400> 268
   caagcucgcuucuaugggucug 22
<210> 269
   <211> 17
   <212> RNA
   <213> Homo sapiens
   <400> 269
   ucccaccgcugccaccc 17
<210> 270
   <211> 22
   <212> RNA
   <213> Homo sapiens
   <400> 270
   cucuagagggaagcgcuuucug 22
<210> 271
   <211> 22
   <212> RNA
   <213> Homo sapiens
   <400> 271
   gguccagaggggagauagguuc 22
<210> 272
   <211> 22
   <212> RNA
   <213> Homo sapiens
   <400> 272
   ugucuacuacuggagacacugg 22
<210> 273
   <211> 22
   <212> RNA
   <213> Homo sapiens
   <400> 273
   cuuucagucagauguuugcugc 22
<210> 274
   <211> 22
   <212> RNA
   <213> Homo sapiens
   <400> 274
   cucaucugcaaagaaguaagug 22
<210> 275
   <211> 22
   <212> RNA
   <213> Homo sapiens
   <400> 275
   caagaaccucaguugcuuuugu 22
<210> 276
   <211> 22
   <212> RNA
   <213> Homo sapiens
   <400> 276
   uaugcauuguauuuuuaggucc 22
<210> 277
   <211> 22
   <212> RNA
   <213> Homo sapiens
   <400> 277
   uauugcacucgucccggccucc 22
<210> 278
   <211> 22
   <212> RNA
   <213> Homo sapiens
   <400> 278
   ucgugcaucccuuuagaguguu 22
<210> 279
   <211> 22
   <212> RNA
   <213> Homo sapiens
   <400> 279
   caaaacuggcaauuacuuuugc 22
<210> 280
   <211> 22
   <212> RNA
   <213> Homo sapiens
   <400> 280
   uauaccucaguuuuaucaggug 22
<210> 281
   <211> 22
   <212> RNA
   <213> Homo sapiens
   <400> 281
   caggucgucuugcagggcuucu 22
<210> 282
   <211> 20
   <212> RNA
   <213> Homo sapiens
   <400> 282
   auuccuagaaauuguucaua 20
<210> 283
   <211> 19
   <212> RNA
   <213> Homo sapiens
   <400> 283
   aguguggcuuucuuagagc 19
<210> 284
   <211> 21
   <212> RNA
   <213> Homo sapiens
   <400> 284
   agaggauacccuuuguauguu 21
<210> 285
   <211> 22
   <212> RNA
   <213> Homo sapiens
   <400> 285
   cugguuucacaugguggcuuag 22
<210> 286
   <211> 22
   <212> RNA
   <213> Homo sapiens
   <400> 286
   gugacaucacauauacggcagc 22
<210> 287
   <211> 19
   <212> RNA
   <213> Homo sapiens
   <400> 287
   gggcgccugugaucccaac 19
<210> 288
   <211> 23
   <212> RNA
   <213> Homo sapiens
   <400> 288
   cggcccgggcugcugcuguuccu 23
<210> 289
   <211> 22
   <212> RNA
   <213> Homo sapiens
   <400> 289
   cacuagauugugagcuccugga 22
<210> 290
   <211> 22
   <212> RNA
   <213> Homo sapiens
   <400> 290
   cuauacagucuacugucuuucc 22
<210> 291
   <211> 19
   <212> RNA
   <213> Homo sapiens
   <400> 291
   gaugaugcugcugaugcug 19
<210> 292
   <211> 18
   <212> RNA
   <213> Homo sapiens
   <400> 292
   ugaggcaguagauugaau 18
<210> 293
   <211> 22
   <212> RNA
   <213> Homo sapiens
   <400> 293
   cugccaauuccauaggucacag 22
<210> 294
   <211> 17
   <212> RNA
   <213> Homo sapiens
   <400> 294
   uaagugcuuccaugcuu 17
<210> 295
   <211> 22
   <212> RNA
   <213> Homo sapiens
   <400> 295
   uauguaacacgguccacuaacc 22
<210> 296
   <211> 22
   <212> RNA
   <213> Homo sapiens
   <400> 296
   cagcagcaauucauguuuugaa 22
<210> 297
   <211> 22
   <212> RNA
   <213> Homo sapiens
   <400> 297
   uaacugguugaacaacugaacc 22
<210> 298
   <211> 22
   <212> RNA
   <213> Homo sapiens
   <400> 298
   guucucccaacguaagcccagc 22
<210> 299
   <211> 22
   <212> RNA
   <213> Homo sapiens
   <400> 299
   cuuaugcaagauucccuucuac 22
<210> 300
   <211> 22
   <212> RNA
   <213> Homo sapiens
   <400> 300
   aaagugcauccuuuuagagguu 22
<210> 301
   <211> 21
   <212> RNA
   <213> Homo sapiens
   <400> 301
   uaggacacauggucuacuucu 21
<210> 302
   <211> 22
   <212> RNA
   <213> Homo sapiens
   <400> 302
   cugaagcucagagggcucugau 22
<210> 303
   <211> 21
   <212> RNA
   <213> Homo sapiens
   <400> 303
   ugcaggaccaagaugagcccu 21
<210> 304
   <211> 22
   <212> RNA
   <213> Homo sapiens
   <400> 304
   accguggcuuucgauuguuacu 22
<210> 305
   <211> 20
   <212> RNA
   <213> Homo sapiens
   <400> 305
   gugcauugcuguugcauugc 20
<210> 306
   <211> 21
   <212> RNA
   <213> Homo sapiens
   <400> 306
   aaaacggugagauuuuguuuu 21
<210> 307
   <211> 20
   <212> RNA
   <213> Homo sapiens
   <400> 307
   auggagauagauauagaaau 20
<210> 308
   <211> 23
   <212> RNA
   <213> Homo sapiens
   <400> 308
   aaggagcuuacaaucuagcuggg 23
<210> 309
   <211> 22
   <212> RNA
   <213> Homo sapiens
   <400> 309
   cacuggcuccuuucuggguaga 22
<210> 310
   <211> 22
   <212> RNA
   <213> Homo sapiens
   <400> 310
   acaaagugcuucccuuuagagu 22
<210> 311
   <211> 22
   <212> RNA
   <213> Homo sapiens
   <400> 311
   augcaccugggcaaggauucug 22
<210> 312
   <211> 22
   <212> RNA
   <213> Homo sapiens
   <400> 312
   gaaaucaagcgugggugagacc 22
<210> 313
   <211> 22
   <212> RNA
   <213> Homo sapiens
   <400> 313
   caaagaauucuccuuuugggcu 22
<210> 314
   <211> 19
   <212> RNA
   <213> Homo sapiens
   <400> 314
   ugagcugcuguaccaaaau 19
<210> 315
   <211> 22
   <212> RNA
   <213> Homo sapiens
   <400> 315
   uucaaguaauccaggauaggcu 22
<210> 316
   <211> 22
   <212> RNA
   <213> Homo sapiens
   <400> 316
   augguacccuggcauacugagu 22
<210> 317
   <211> 22
   <212> RNA
   <213> Homo sapiens
   <400> 317
   uucccuuugucauccuucgccu 22
<210> 318
   <211> 22
   <212> RNA
   <213> Homo sapiens
   <400> 318
   uuugaggcuacagugagaugug 22
<210> 319
   <211> 21
   <212> RNA
   <213> Homo sapiens
   <400> 319
   ccaccaccgugucugacacuu 21
<210> 320
   <211> 22
   <212> RNA
   <213> Homo sapiens
   <400> 320
   ugcaacgaaccugagccacuga 22
<210> 321
   <211> 21
   <212> RNA
   <213> Homo sapiens
   <400> 321
   agagaagaagaucagccugca 21
<210> 322
   <211> 20
   <212> RNA
   <213> Homo sapiens
   <400> 322
   ucugcaggguuugcuuugag 20
<210> 323
   <211> 23
   <212> RNA
   <213> Homo sapiens
   <400> 323
   uuauugcuuaagaauacgcguag 23
<210> 324
   <211> 22
   <212> RNA
   <213> Homo sapiens
   <400> 324
   aaucauacacgguugaccuauu 22
<210> 325
   <211> 21
   <212> RNA
   <213> Homo sapiens
   <400> 325
   aggguaagcugaaccucugau 21
<210> 326
   <211> 22
   <212> RNA
   <213> Homo sapiens
   <400> 326
   gugaacgggcgccaucccgagg 22
<210> 327
   <211> 22
   <212> RNA
   <213> Homo sapiens
   <400> 327
   aauugcacgguauccaucugua 22
<210> 328
   <211> 22
   <212> RNA
   <213> Homo sapiens
   <400> 328
   aaaaccgucuaguuacaguugu 22
<210> 329
   <211> 22
   <212> RNA
   <213> Homo sapiens
   <400> 329
   agaguugagucuggacgucccg 22
<210> 330
   <211> 21
   <212> RNA
   <213> Homo sapiens
   <400> 330
   ccacaccguaucugacacuuu 21
<210> 331
   <211> 22
   <212> RNA
   <213> Homo sapiens
   <400> 331
   cucaguagccaguguagauccu 22
<210> 332
   <211> 21
   <212> RNA
   <213> Homo sapiens
   <400> 332
   cacauuacacggucgaccucu 21
<210> 333
   <211> 22
   <212> RNA
   <213> Homo sapiens
   <400> 333
   aucauagaggaaaauccauguu 22
<210> 334
   <211> 20
   <212> RNA
   <213> Homo sapiens
   <400> 334
   ccauggaucuccaggugggu 20
<210> 335
   <211> 23
   <212> RNA
   <213> Homo sapiens
   <400> 335
   gaacgcgcuucccuauagagggu 23
<210> 336
   <211> 23
   <212> RNA
   <213> Homo sapiens
   <400> 336
   acuuaaacguggauguacuugcu 23
<210> 337
   <211> 22
   <212> RNA
   <213> Homo sapiens
   <400> 337
   agagcuuagcugauuggugaac 22
<210> 338
   <211> 20
   <212> RNA
   <213> Homo sapiens
   <400> 338
   agaccauggguucucauugu 20
<210> 339
   <211> 21
   <212> RNA
   <213> Homo sapiens
   <400> 339
   uacucaaaaagcugucaguca 21
<210> 340
   <211> 22
   <212> RNA
   <213> Homo sapiens
   <400> 340
   guagauucuccuucuaugagua 22
<210> 341
   <211> 23
   <212> RNA
   <213> Homo sapiens
   <400> 341
   aaacucuacuuguccuucugagu 23
<210> 342
   <211> 23
   <212> RNA
   <213> Homo sapiens
   <400> 342
   gagggucuugggagggaugugac 23
<210> 343
   <211> 22
   <212> RNA
   <213> Homo sapiens
   <400> 343
   ccucccacacccaaggcuugca 22
<210> 344
   <211> 23
   <212> RNA
   <213> Homo sapiens
   <400> 344
   aacauucauugcugucggugggu 23
<210> 345
   <211> 22
   <212> RNA
   <213> Homo sapiens
   <400> 345
   aacgcacuucccuuuagagugu 22
<210> 346
   <211> 22
   <212> RNA
   <213> Homo sapiens
   <400> 346
   ucaguaaauguuuauuagauga 22
<210> 347
   <211> 22
   <212> RNA
   <213> Homo sapiens
   <400> 347
   auaaagcuagauaaccgaaagu 22
<210> 348
   <211> 20
   <212> RNA
   <213> Homo sapiens
   <400> 348
   ggggagcuguggaagcagua 20
<210> 349
   <211> 21
   <212> RNA
   <213> Homo sapiens
   <400> 349
   ugaguuggccaucugagugag 21
<210> 350
   <211> 23
   <212> RNA
   <213> Homo sapiens
   <400> 350
   acuugggcacugaaacaaugucc 23
<210> 351
   <211> 22
   <212> RNA
   <213> Homo sapiens
   <400> 351
   uaauacugccugguaaugauga 22
<210> 352
   <211> 22
   <212> RNA
   <213> Homo sapiens
   <400> 352
   uaugugccuuuggacuacaucg 22
<210> 353
   <211> 22
   <212> RNA
   <213> Homo sapiens
   <400> 353
   uggugguuuacaaaguaauuca 22
<210> 354
   <211> 22
   <212> RNA
   <213> Homo sapiens
   <400> 354
   acucaaaaugggggcgcuuucc 22
<210> 355
   <211> 22
   <212> RNA
   <213> Homo sapiens
   <400> 355
   ccucugaaauucaguucuucag 22
<210> 356
   <211> 22
   <212> RNA
   <213> Homo sapiens
   <400> 356
   aacgccauuaucacacuaaaua 22
<210> 357
   <211> 22
   <212> RNA
   <213> Homo sapiens
   <400> 357
   uugggaucauuuugcauccaua 22
<210> 358
   <211> 22
   <212> RNA
   <213> Homo sapiens
   <400> 358
   uggcucaguucagcaggaacag 22
<210> 359
   <211> 22
   <212> RNA
   <213> Homo sapiens
   <400> 359
   ucaggcucaguccccucccgau 22
<210> 360
   <211> 23
   <212> RNA
   <213> Homo sapiens
   <400> 360
   ucauagcccuguacaaugcugcu 23
<210> 361
   <211> 22
   <212> RNA
   <213> Homo sapiens
   <400> 361
   uauguaauaugguccacaucuu 22
<210> 362
   <211> 18
   <212> RNA
   <213> Homo sapiens
   <400> 362
   uucacagggaggugucau 18
<210> 363
   <211> 22
   <212> RNA
   <213> Homo sapiens
   <400> 363
   uacugcagacguggcaaucaug 22
<210> 364
   <211> 21
   <212> RNA
   <213> Homo sapiens
   <400> 364
   gcaggaacuugugagucuccu 21
<210> 365
   <211> 22
   <212> RNA
   <213> Homo sapiens
   <400> 365
   gaugagcucauuguaauaugag 22
<210> 366
   <211> 22
   <212> RNA
   <213> Homo sapiens
   <400> 366
   agaucgaccguguuauauucgc 22
<210> 367
   <211> 21
   <212> RNA
   <213> Homo sapiens
   <400> 367
   guguugaaacaaucucuacug 21
<210> 368
   <211> 23
   <212> RNA
   <213> Homo sapiens
   <400> 368
   ucugcucauaccccaugguuucu 23
<210> 369
   <211> 18
   <212> RNA
   <213> Homo sapiens
   <400> 369
   ugcuuccuuucagagggu 18
<210> 370
   <211> 22
   <212> RNA
   <213> Homo sapiens
   <400> 370
   aaagugcuuccuuuuagagggu 22
<210> 371
   <211> 22
   <212> RNA
   <213> Homo sapiens
   <400> 371
   caacuagacugugagcuucuag 22
<210> 372
   <211> 20
   <212> RNA
   <213> Homo sapiens
   <400> 372
   agagucuugugaugucuugc 20
<210> 373
   <211> 20
   <212> RNA
   <213> Homo sapiens
   <400> 373
   cuacaaagggaagcccuuuc 20
<210> 374
   <211> 23
   <212> RNA
   <213> Homo sapiens
   <400> 374
   cacucagccuugagggcacuuuc 23
<210> 375
   <211> 22
   <212> RNA
   <213> Homo sapiens
   <400> 375
   cuuaucagauuguauuguaauu 22
<210> 376
   <211> 25
   <212> RNA
   <213> Homo sapiens
   <400> 376
   cuagugagggacagaaccaggauuc 25
<210> 377
   <211> 21
   <212> RNA
   <213> Homo sapiens
   <400> 377
   uguucauguagauguuuaagc 21
<210> 378
   <211> 21
   <212> RNA
   <213> Homo sapiens
   <400> 378
   auauaugaugacuuagcuuuu 21
<210> 379
   <211> 21
   <212> RNA
   <213> Homo sapiens
   <400> 379
   cuccagagggaugcacuuucu 21
<210> 380
   <211> 22
   <212> RNA
   <213> Homo sapiens
   <400> 380
   caucuuaccggacagugcugga 22
<210> 381
   <211> 22
   <212> RNA
   <213> Homo sapiens
   <400> 381
   uggguggucuggagauuugugc 22
<210> 382
   <211> 23
   <212> RNA
   <213> Homo sapiens
   <400> 382
   aaagugcugcgacauuugagcgu 23
<210> 383
   <211> 22
   <212> RNA
   <213> Homo sapiens
   <400> 383
   aaaaguaauugcgaguuuuacc 22
<210> 384
   <211> 22
   <212> RNA
   <213> Homo sapiens
   <400> 384
   aaaaguacuugcggauuuugcu 22
<210> 385
   <211> 18
   <212> RNA
   <213> Homo sapiens
   <400> 385
   uugagaaggaggcugcug 18
<210> 386
   <211> 23
   <212> RNA
   <213> Homo sapiens
   <400> 386
   caagucuuauuugagcaccuguu 23
<210> 387
   <211> 21
   <212> RNA
   <213> Homo sapiens
   <400> 387
   gcgacccacucuugguuucca 21
<210> 388
   <211> 22
   <212> RNA
   <213> Homo sapiens
   <400> 388
   uagguaguuuccuguuguuggg 22
<210> 389
   <211> 22
   <212> RNA
   <213> Homo sapiens
   <400> 389
   caauuuagugugugugauauuu 22
<210> 390
   <211> 21
   <212> RNA
   <213> Homo sapiens
   <400> 390
   gugcauuguaguugcauugca 21
<210> 391
   <211> 22
   <212> RNA
   <213> Homo sapiens
   <400> 391
   aaaaguaauugugguuuuugcc 22
<210> 392
   <211> 22
   <212> RNA
   <213> Homo sapiens
   <400> 392
   agucauuggaggguuugagcag 22
<210> 393
   <211> 22
   <212> RNA
   <213> Homo sapiens
   <400> 393
   uggauuucuuugugaaucacca 22
<210> 394
   <211> 23
   <212> RNA
   <213> Homo sapiens
   <400> 394
   ugauuguagccuuuuggaguaga 23
<210> 395
   <211> 22
   <212> RNA
   <213> Homo sapiens
   <400> 395
   ccuauucuugauuacuuguuuc 22
<210> 396
   <211> 22
   <212> RNA
   <213> Homo sapiens
   <400> 396
   ucgugucuuguguugcagccgg 22
<210> 397
   <211> 22
   <212> RNA
   <213> Homo sapiens
   <400> 397
   acaguagucugcacauugguua 22
<210> 398
   <211> 22
   <212> RNA
   <213> Homo sapiens
   <400> 398
   aaucaugugcagugccaauaug 22
<210> 399
   <211> 23
   <212> RNA
   <213> Homo sapiens
   <400> 399
   uaaggugcaucuagugcaguuag 23
<210> 400
   <211> 21
   <212> RNA
   <213> Homo sapiens
   <400> 400
   cuggauggcuccuccaugucu 21
<210> 401
   <211> 22
   <212> RNA
   <213> Homo sapiens
   <400> 401
   ugauugguacgucuguggguag 22
<210> 402
   <211> 27
   <212> RNA
   <213> Homo sapiens
   <400> 402
   cacuguaggugauggugagagugggca 27
<210> 403
   <211> 19
   <212> RNA
   <213> Homo sapiens
   <400> 403
   agcugucugaaaaugucuu 19
<210> 404
   <211> 22
   <212> RNA
   <213> Homo sapiens
   <400> 404
   uucacaaggaggugucauuuau 22
<210> 405
   <211> 22
   <212> RNA
   <213> Homo sapiens
   <400> 405
   agacuucccauuugaagguggc 22
<210> 406
   <211> 23
   <212> RNA
   <213> Homo sapiens
   <400> 406
   ucuuugguuaucuagcuguauga 23
<210> 407
   <211> 22
   <212> RNA
   <213> Homo sapiens
   <400> 407
   aagugccuccuuuuagaguguu 22
<210> 408
   <211> 22
   <212> RNA
   <213> Homo sapiens
   <400> 408
   uucccuuugucauccuaugccu 22
<210> 409
   <211> 22
   <212> RNA
   <213> Homo sapiens
   <400> 409
   uagcaccauuugaaaucgguua 22
<210> 410
   <211> 18
   <212> RNA
   <213> Homo sapiens
   <400> 410
   cgggcguggugguggggg 18
<210> 411
   <211> 22
   <212> RNA
   <213> Homo sapiens
   <400> 411
   uggagugugacaaugguguuug 22
<210> 412
   <211> 22
   <212> RNA
   <213> Homo sapiens
   <400> 412
   caacaaaucccagucuaccuaa 22
<210> 413
   <211> 22
   <212> RNA
   <213> Homo sapiens
   <400> 413
   caggccauauugugcugccuca 22
<210> 414
   <211> 23
   <212> RNA
   <213> Homo sapiens
   <400> 414
   aacauucauuguugucggugggu 23
<210> 415
   <211> 21
   <212> RNA
   <213> Homo sapiens
   <400> 415
   ugauuguccaaacgcaauucu 21
<210> 416
   <211> 23
   <212> RNA
   <213> Homo sapiens
   <400> 416
   uaagugcuuccauguuugagugu 23
<210> 417
   <211> 22
   <212> RNA
   <213> Homo sapiens
   <400> 417
   uggcagugucuuagcugguugu 22
<210> 418
   <211> 21
   <212> RNA
   <213> Homo sapiens
   <400> 418
   aauauaacacagauggccugu 21
<210> 419
   <211> 22
   <212> RNA
   <213> Homo sapiens
   <400> 419
   caauguuuccacagugcaucac 22
<210> 420
   <211> 22
   <212> RNA
   <213> Homo sapiens
   <400> 420
   aaugcaccugggcaaggauuca 22
<210> 421
   <211> 21
   <212> RNA
   <213> Homo sapiens
   <400> 421
   ugguagacuauggaacguagg 21
<210> 422
   <211> 23
   <212> RNA
   <213> Homo sapiens
   <400> 422
   uuucaagccagggggcguuuuuc 23
<210> 423
   <211> 22
   <212> RNA
   <213> Homo sapiens
   <400> 423
   cucuagagggaagcacuuucug 22
<210> 424
   <211> 22
   <212> RNA
   <213> Homo sapiens
   <400> 424
   auauuaccauuagcucaucuuu 22
<210> 425
   <211> 21
   <212> RNA
   <213> Homo sapiens
   <400> 425
   auccuugcuaucugggugcua 21
<210> 426
   <211> 21
   <212> RNA
   <213> Homo sapiens
   <400> 426
   aggcaagaugcuggcauagcu 21
<210> 427
   <211> 22
   <212> RNA
   <213> Homo sapiens
   <400> 427
   aacccguagauccgaacuugug 22
<210> 428
   <211> 22
   <212> RNA
   <213> Homo sapiens
   <400> 428
   gaggguuggguggaggcucucc 22
<210> 429
   <211> 22
   <212> RNA
   <213> Homo sapiens
   <400> 429
   ggggguccccggugcucggauc 22
<210> 430
   <211> 21
   <212> RNA
   <213> Homo sapiens
   <400> 430
   caacaccagucgaugggcugu 21
<210> 431
   <211> 23
   <212> RNA
   <213> Homo sapiens
   <400> 431
   ggcagguucucacccucucuagg 23
<210> 432
   <211> 22
   <212> RNA
   <213> Homo sapiens
   <400> 432
   uuuaggauaagcuugacuuuug 22
<210> 433
   <211> 21
   <212> RNA
   <213> Homo sapiens
   <400> 433
   uggaggagaaggaaggugaug 21
<210> 434
   <211> 21
   <212> RNA
   <213> Homo sapiens
   <400> 434
   caaagguauuugugguuuuug 21
<210> 435
   <211> 22
   <212> RNA
   <213> Homo sapiens
   <400> 435
   agaauuguggcuggacaucugu 22
<210> 436
   <211> 22
   <212> RNA
   <213> Homo sapiens
   <400> 436
   aaugcacccgggcaaggauucu 22
<210> 437
   <211> 23
   <212> RNA
   <213> Homo sapiens
   <400> 437
   uaagugcuuccauguuuugguga 23
<210> 438
   <211> 22
   <212> RNA
   <213> Homo sapiens
   <400> 438
   uuccuaugcauauacuucuuug 22
<210> 439
   <211> 22
   <212> RNA
   <213> Homo sapiens
   <400> 439
   uggaauguaaggaagugugugg 22
<210> 440
   <211> 22
   <212> RNA
   <213> Homo sapiens
   <400> 440
   aaagugcuucucuuuggugggu 22
<210> 441
   <211> 22
   <212> RNA
   <213> Homo sapiens
   <400> 441
   aaaaguaauugcggauuuugcc 22
<210> 442
   <211> 21
   <212> RNA
   <213> Homo sapiens
   <400> 442
   gugucuuuugcucugcaguca 21
<210> 443
   <211> 22
   <212> RNA
   <213> Homo sapiens
   <400> 443
   uguaaacauccucgacuggaag 22
<210> 444
   <211> 21
   <212> RNA
   <213> Homo sapiens
   <400> 444
   ccccaccuccucucuccucag 21
<210> 445
   <211> 22
   <212> RNA
   <213> Homo sapiens
   <400> 445
   gaaggcgcuucccuuuagagcg 22
<210> 446
   <211> 22
   <212> RNA
   <213> Homo sapiens
   <400> 446
   guggguacggcccagugggggg 22
<210> 447
   <211> 22
   <212> RNA
   <213> Homo sapiens
   <400> 447
   cguguauuugacaagcugaguu 22
<210> 448
   <211> 22
   <212> RNA
   <213> Homo sapiens
   <400> 448
   uccgagccugggucucccucuu 22
<210> 449
   <211> 22
   <212> RNA
   <213> Homo sapiens
   <400> 449
   cgaaaacagcaauuaccuuugc 22
<210> 450
   <211> 22
   <212> RNA
   <213> Homo sapiens
   <400> 450
   aaaagcuggguugagagggcga 22
<210> 451
   <211> 23
   <212> RNA
   <213> Homo sapiens
   <400> 451
   uccaguaccacgugucagggcca 23
<210> 452
   <211> 23
   <212> RNA
   <213> Homo sapiens
   <400> 452
   uuacaguuguucaaccaguuacu 23
<210> 453
   <211> 22
   <212> RNA
   <213> Homo sapiens
   <400> 453
   gagcuuauucauaaaagugcag 22
<210> 454
   <211> 22
   <212> RNA
   <213> Homo sapiens
   <400> 454
   cuugguucagggagggucccca 22
<210> 455
   <211> 21
   <212> RNA
   <213> Homo sapiens
   <400> 455
   acucuagcugccaaaggcgcu 21
<210> 456
   <211> 23
   <212> RNA
   <213> Homo sapiens
   <400> 456
   uauucauuuauccccagccuaca 23
<210> 457
   <211> 21
   <212> RNA
   <213> Homo sapiens
   <400> 457
   cccagauaauggcacucucaa 21
<210> 458
   <211> 22
   <212> RNA
   <213> Homo sapiens
   <400> 458
   aaaacuguaauuacuuuuguac 22
<210> 459
   <211> 23
   <212> RNA
   <213> Homo sapiens
   <400> 459
   caguaacaaagauucauccuugu 23
<210> 460
   <211> 23
   <212> RNA
   <213> Homo sapiens
   <400> 460
   ucggggaucaucaugucacgaga 23
<210> 461
   <211> 22
   <212> RNA
   <213> Homo sapiens
   <400> 461
   ugauauguuugauauauuaggu 22
<210> 462
   <211> 22
   <212> RNA
   <213> Homo sapiens
   <400> 462
   augguuccgucaagcaccaugg 22
<210> 463
   <211> 22
   <212> RNA
   <213> Homo sapiens
   <400> 463
   acuguugcuaauaugcaacucu 22
<210> 464
   <211> 22
   <212> RNA
   <213> Homo sapiens
   <400> 464
   uuuugcgauguguuccuaauau 22
<210> 465
   <211> 22
   <212> RNA
   <213> Homo sapiens
   <400> 465
   aauugcacuuuagcaaugguga 22
<210> 466
   <211> 20
   <212> RNA
   <213> Homo sapiens
   <400> 466
   uaaggcacgcggugaaugcc 20
<210> 467
   <211> 23
   <212> RNA
   <213> Homo sapiens
   <400> 467
   ugcaccaugguugucugagcaug 23
<210> 468
   <211> 23
   <212> RNA
   <213> Homo sapiens
   <400> 468
   uaauacugccggguaaugaugga 23
<210> 469
   <211> 20
   <212> RNA
   <213> Homo sapiens
   <400> 469
   guccgcucggcgguggccca 20
<210> 470
   <211> 22
   <212> RNA
   <213> Homo sapiens
   <400> 470
   cucuagagggaagcacuuucug 22
<210> 471
   <211> 22
   <212> RNA
   <213> Homo sapiens
   <400> 471
   acaguagagggaggaaucgcag 22
<210> 472
   <211> 22
   <212> RNA
   <213> Homo sapiens
   <400> 472
   caaaaguaauuguggauuuugu 22
<210> 473
   <211> 22
   <212> RNA
   <213> Homo sapiens
   <400> 473
   uagcuuaucagacugauguuga 22
<210> 474
   <211> 21
   <212> RNA
   <213> Homo sapiens
   <400> 474
   ugguucuagacuugccaacua 21
<210> 475
   <211> 23
   <212> RNA
   <213> Homo sapiens
   <400> 475
   aggcaguguaguuagcugauugc 23
<210> 476
   <211> 22
   <212> RNA
   <213> Homo sapiens
   <400> 476
   uaauacugucugguaaaaccgu 22
<210> 477
   <211> 22
   <212> RNA
   <213> Homo sapiens
   <400> 477
   augcugacauauuuacuagagg 22
<210> 478
   <211> 22
   <212> RNA
   <213> Homo sapiens
   <400> 478
   acugauuucuuuugguguucag 22
<210> 479
   <211> 22
   <212> RNA
   <213> Homo sapiens
   <400> 479
   gccugcugggguggaaccuggu 22
<210> 480
   <211> 21
   <212> RNA
   <213> Homo sapiens
   <400> 480
   cuauacaaucuacugucuuuc 21
<210> 481
   <211> 22
   <212> RNA
   <213> Homo sapiens
   <400> 481
   caguuaucacagugcugaugcu 22
<210> 482
   <211> 21
   <212> RNA
   <213> Homo sapiens
   <400> 482
   uaaaguaaauaugcaccaaaa 21
<210> 483
   <211> 23
   <212> RNA
   <213> Homo sapiens
   <400> 483
   uacugcaucaggaacugauugga 23
<210> 484
   <211> 22
   <212> RNA
   <213> Homo sapiens
   <400> 484
   cucuagagggaagcgcuuucug 22
<210> 485
   <211> 22
   <212> RNA
   <213> Homo sapiens
   <400> 485
   cuuucagucggauguuuacagc 22
<210> 486
   <211> 20
   <212> RNA
   <213> Homo sapiens
   <400> 486
   guguguggaaaugcuucugc 20
<210> 487
   <211> 22
   <212> RNA
   <213> Homo sapiens
   <400> 487
   aaucguacagggucauccacuu 22
<210> 488
   <211> 22
   <212> RNA
   <213> Homo sapiens
   <400> 488
   gacugacaccucuuugggugaa 22
<210> 489
   <211> 22
   <212> RNA
   <213> Homo sapiens
   <400> 489
   uccuucauuccaccggagucug 22
<210> 490
   <211> 21
   <212> RNA
   <213> Homo sapiens
   <400> 490
   agugaaugauggguucugacc 21
<210> 491
   <211> 23
   <212> RNA
   <213> Homo sapiens
   <400> 491
   uggaagacuagugauuuuguugu 23
<210> 492
   <211> 21
   <212> RNA
   <213> Homo sapiens
   <400> 492
   agggcccccccucaauccugu 21
<210> 493
   <211> 23
   <212> RNA
   <213> Homo sapiens
   <400> 493
   aggaugagcaaagaaaguagauu 23
<210> 494
   <211> 22
   <212> RNA
   <213> Homo sapiens
   <400> 494
   ugguugaccauagaacaugcgc 22
<210> 495
   <211> 17
   <212> RNA
   <213> Homo sapiens
   <400> 495
   gugggggagaggcuguc 17
<210> 496
   <211> 22
   <212> RNA
   <213> Homo sapiens
   <400> 496
   ucucugggccugugucuuaggc 22
<210> 497
   <211> 22
   <212> RNA
   <213> Homo sapiens
   <400> 497
   aacuggaucaauuauaggagug 22
<210> 498
   <211> 22
   <212> RNA
   <213> Homo sapiens
   <400> 498
   caucaucgucucaaaugagucu 22
<210> 499
   <211> 22
   <212> RNA
   <213> Homo sapiens
   <400> 499
   caucuuccaguacaguguugga 22
<210> 500
   <211> 22
   <212> RNA
   <213> Homo sapiens
   <400> 500
   aucgugcauccuuuuagagugu 22
<210> 501
   <211> 21
   <212> RNA
   <213> Homo sapiens
   <400> 501
   ggcuagcaacagcgcuuaccu 21
<210> 502
   <211> 22
   <212> RNA
   <213> Homo sapiens
   <400> 502
   accuugccuugcugcccgggcc 22
<210> 503
   <211> 22
   <212> RNA
   <213> Homo sapiens
   <400> 503
   uggugggcacagaaucuggacu 22
<210> 504
   <211> 22
   <212> RNA
   <213> Homo sapiens
   <400> 504
   aaacauucgcggugcacuucuu 22
<210> 505
   <211> 22
   <212> RNA
   <213> Homo sapiens
   <400> 505
   ucuucucuguuuuggccaugug 22
<210> 506
   <211> 22
   <212> RNA
   <213> Homo sapiens
   <400> 506
   ccuauucuugguuacuugcacg 22
<210> 507
   <211> 23
   <212> RNA
   <213> Homo sapiens
   <400> 507
   aguauguucuuccaggacagaac 23
<210> 508
   <211> 22
   <212> RNA
   <213> Homo sapiens
   <400> 508
   uggacggagaacugauaagggu 22
<210> 509
   <211> 21
   <212> RNA
   <213> Homo sapiens
   <400> 509
   aucauagaggaaaauccacgu 21
<210> 510
   <211> 22
   <212> RNA
   <213> Homo sapiens
   <400> 510
   cguguucacagcggaccuugau 22
<210> 511
   <211> 24
   <212> RNA
   <213> Homo sapiens
   <400> 511
   agccuggaagcuggagccugcagu 24
<210> 512
   <211> 21
   <212> RNA
   <213> Homo sapiens
   <400> 512
   uggcagggaggcugggagggg 21
<210> 513
   <211> 22
   <212> RNA
   <213> Homo sapiens
   <400> 513
   uugagaaugaugaaucauuagg 22
<210> 514
   <211> 22
   <212> RNA
   <213> Homo sapiens
   <400> 514
   cuauacaaccuacugccuuccc 22
<210> 515
   <211> 22
   <212> RNA
   <213> Homo sapiens
   <400> 515
   ggagaaauuauccuuggugugu 22
<210> 516
   <211> 22
   <212> RNA
   <213> Homo sapiens
   <400> 516
   aaagugcuucccuuuggacugu 22
<210> 517
   <211> 19
   <212> RNA
   <213> Homo sapiens
   <400> 517
   ugggcguaucuguaugcua 19
<210> 518
   <211> 20
   <212> RNA
   <213> Homo sapiens
   <400> 518
   cuguaugcccucaccgcuca 20
<210> 519
   <211> 21
   <212> RNA
   <213> Homo sapiens
   <400> 519
   cugacuguugccguccuccag 21
<210> 520
   <211> 24
   <212> RNA
   <213> Homo sapiens
   <400> 520
   cugaagugauguguaacugaucag 24
<210> 521
   <211> 23
   <212> RNA
   <213> Homo sapiens
   <400> 521
   caaagugcuguucgugcagguag 23
<210> 522
   <211> 22
   <212> RNA
   <213> Homo sapiens
   <400> 522
   agggcuuagcugcuugugagca 22
<210> 523
   <211> 20
   <212> RNA
   <213> Homo sapiens
   <400> 523
   aggaauguuccuucuuugcc 20
<210> 524
   <211> 22
   <212> RNA
   <213> Homo sapiens
   <400> 524
   acacagggcuguugugaagacu 22
<210> 525
   <211> 21
   <212> RNA
   <213> Homo sapiens
   <400> 525
   gaaggcgcuucccuuuggagu 21
<210> 526
   <211> 23
   <212> RNA
   <213> Homo sapiens
   <400> 526
   uaauccuugcuaccugggugaga 23
<210> 527
   <211> 24
   <212> RNA
   <213> Homo sapiens
   <400> 527
   agccugauuaaacacaugcucuga 24
<210> 528
   <211> 22
   <212> RNA
   <213> Homo sapiens
   <400> 528
   acugcauuaugagcacuuaaag 22
<210> 529
   <211> 22
   <212> RNA
   <213> Homo sapiens
   <400> 529
   ggaggggucccgcacugggagg 22
<210> 530
   <211> 22
   <212> RNA
   <213> Homo sapiens
   <400> 530
   aucgggaaugucguguccgccc 22
<210> 531
   <211> 22
   <212> RNA
   <213> Homo sapiens
   <400> 531
   gagugccuucuuuuggagcguu 22
<210> 532
   <211> 22
   <212> RNA
   <213> Homo sapiens
   <400> 532
   agagguugcccuuggugaauuc 22
<210> 533
   <211> 22
   <212> RNA
   <213> Homo sapiens
   <400> 533
   agacccuggucugcacucuauc 22
<210> 534
   <211> 22
   <212> RNA
   <213> Homo sapiens
   <400> 534
   caaaaaucucaauuacuuuugc 22
<210> 535
   <211> 20
   <212> RNA
   <213> Homo sapiens
   <400> 535
   uaaagagcccuguggagaca 20
<210> 536
   <211> 23
   <212> RNA
   <213> Homo sapiens
   <400> 536
   agcugguguugugaaucaggccg 23
<210> 537
   <211> 21
   <212> RNA
   <213> Homo sapiens
   <400> 537
   ugucuugcaggccgucaugca 21
<210> 538
   <211> 22
   <212> RNA
   <213> Homo sapiens
   <400> 538
   ugaaacauacacgggaaaccuc 22
<210> 539
   <211> 22
   <212> RNA
   <213> Homo sapiens
   <400> 539
   uugcauauguaggaugucccau 22
<210> 540
   <211> 23
   <212> RNA
   <213> Homo sapiens
   <400> 540
   cuaauaguaucuaccacaauaaa 23
<210> 541
   <211> 22
   <212> RNA
   <213> Homo sapiens
   <400> 541
   aaucauacagggacauccaguu 22
<210> 542
   <211> 23
   <212> RNA
   <213> Homo sapiens
   <400> 542
   ucuggcuccgugucuucacuccc 23
<210> 543
   <211> 22
   <212> RNA
   <213> Homo sapiens
   <400> 543
   uauacaagggcagacucucucu 22
<210> 544
   <211> 27
   <212> RNA
   <213> Homo sapiens
   <400> 544
   auugaucaucgacacuucgaacgcaau 27
<210> 545
   <211> 22
   <212> RNA
   <213> Homo sapiens
   <400> 545
   ucggauccgucugagcuuggcu 22
<210> 546
   <211> 22
   <212> RNA
   <213> Homo sapiens
   <400> 546
   uccuguacugagcugccccgag 22
<210> 547
   <211> 22
   <212> RNA
   <213> Homo sapiens
   <400> 547
   ucagugcacuacagaacuuugu 22
<210> 548
   <211> 22
   <212> RNA
   <213> Homo sapiens
   <400> 548
   ugugagguuggcauuguugucu 22
<210> 549
   <211> 22
   <212> RNA
   <213> Homo sapiens
   <400> 549
   aaaaguauuugcggguuuuguc 22
<210> 550
   <211> 21
   <212> RNA
   <213> Homo sapiens
   <400> 550
   cauaaaguagaaagcacuacu 21
<210> 551
   <211> 21
   <212> RNA
   <213> Homo sapiens
   <400> 551
   uuaauaucggacaaccauugu 21
<210> 552
   <211> 22
   <212> RNA
   <213> Homo sapiens
   <400> 552
   uaaugccccuaaaaauccuuau 22
<210> 553
   <211> 22
   <212> RNA
   <213> Homo sapiens
   <400> 553
   cacccguagaaccgaccuugcg 22
<210> 554
   <211> 22
   <212> RNA
   <213> Homo sapiens
   <400> 554
   caucuuacugggcagcauugga 22
<210> 555
   <211> 22
   <212> RNA
   <213> Homo sapiens
   <400> 555
   uaacacugucugguaacgaugu 22
<210> 556
   <211> 21
   <212> RNA
   <213> Homo sapiens
   <400> 556
   aaagcgcuucccuucagagug 21
<210> 557
   <211> 25
   <212> RNA
   <213> Homo sapiens
   <400> 557
   gcugggcagggcuucugagcuccuu 25
<210> 558
   <211> 22
   <212> RNA
   <213> Homo sapiens
   <400> 558
   gugaauuaccgaagggccauaa 22
<210> 559
   <211> 22
   <212> RNA
   <213> Homo sapiens
   <400> 559
   ucagugcaucacagaacuuugu 22
<210> 560
   <211> 23
   <212> RNA
   <213> Homo sapiens
   <400> 560
   agcagcauuguacagggcuauga 23
<210> 561
   <211> 22
   <212> RNA
   <213> Homo sapiens
   <400> 561
   ccaaaacugcaguuacuuuugc 22
<210> 562
   <211> 21
   <212> RNA
   <213> Homo sapiens
   <400> 562
   cccggagccaggaugcagcuc 21
<210> 563
   <211> 22
   <212> RNA
   <213> Homo sapiens
   <400> 563
   uauagggauuggagccguggcg 22
<210> 564
   <211> 22
   <212> RNA
   <213> Homo sapiens
   <400> 564
   agaucagaaggugauuguggcu 22
<210> 565
   <211> 22
   <212> RNA
   <213> Homo sapiens
   <400> 565
   ucugcccccuccgcugcugcca 22
<210> 566
   <211> 23
   <212> RNA
   <213> Homo sapiens
   <400> 566
   gaagugcuucgauuuuggggugu 23
<210> 567
   <211> 20
   <212> RNA
   <213> Homo sapiens
   <400> 567
   acucaaacugugggggcacu 20
<210> 568
   <211> 24
   <212> RNA
   <213> Homo sapiens
   <400> 568
   agcagaagcagggagguucuccca 24
<210> 569
   <211> 22
   <212> RNA
   <213> Homo sapiens
   <400> 569
   uuugugaccugguccacuaacc 22
<210> 570
   <211> 23
   <212> RNA
   <213> Homo sapiens
   <400> 570
   acuucaccugguccacuagccgu 23
<210> 571
   <211> 23
   <212> RNA
   <213> Homo sapiens
   <400> 571
   caaagcgcuucucuuuagagugu 23
<210> 572
   <211> 24
   <212> RNA
   <213> Homo sapiens
   <400> 572
   ucagaacaaaugccgguucccaga 24
<210> 573
   <211> 22
   <212> RNA
   <213> Homo sapiens
   <400> 573
   acuuguaugcuagcucagguag 22
<210> 574
   <211> 22
   <212> RNA
   <213> Homo sapiens
   <400> 574
   uugugucaauaugcgaugaugu 2
<210> 575
   <211> 21
   <212> RNA
   <213> Homo sapiens
   <400> 575
   cacuguguccuuucugcguag 21
<210> 576
   <211> 22
   <212> RNA
   <213> Homo sapiens
   <400> 576
   aaauuauuguacaucggaugag 22
<210> 577
   <211> 22
   <212> RNA
   <213> Homo sapiens
   <400> 577
   aagauguggaaaaauuggaauc 22
<210> 578
   <211> 21
   <212> RNA
   <213> Homo sapiens
   <400> 578
   ucuuguguucucuagaucagu 21
<210> 579
   <211> 22
   <212> RNA
   <213> Homo sapiens
   <400> 579
   gacuauagaacuuucccccuca 22
<210> 580
   <211> 18
   <212> RNA
   <213> Homo sapiens
   <400> 580
   aucccaccucugccacca 18
<210> 581
   <211> 17
   <212> RNA
   <213> Homo sapiens
   <400> 581
   ucgccuccuccucuccc 17
<210> 582
   <211> 21
   <212> RNA
   <213> Homo sapiens
   <400> 582
   gaacggcuucauacaggaguu 21
<210> 583
   <211> 22
   <212> RNA
   <213> Homo sapiens
   <400> 583
   uuugguccccuucaaccagcua 22
<210> 584
   <211> 22
   <212> RNA
   <213> Homo sapiens
   <400> 584
   ggguggggauuuguugcauuac 22
<210> 585
   <211> 22
   <212> RNA
   <213> Homo sapiens
   <400> 585
   caagcuuguaucuauagguaug 22
<210> 586
   <211> 22
   <212> RNA
   <213> Homo sapiens
   <400> 586
   ugagaaccacgucugcucugag 22
<210> 587
   <211> 21
   <212> RNA
   <213> Homo sapiens
   <400> 587
   uugugcuugaucuaaccaugu 21
<210> 588
   <211> 21
   <212> RNA
   <213> Homo sapiens
   <400> 588
   caagucacuagugguuccguu 21
<210> 589
   <211> 22
   <212> RNA
   <213> Homo sapiens
   <400> 589
   ccaauauuacugugcugcuuua 22
<210> 590
   <211> 23
   <212> RNA
   <213> Homo sapiens
   <400> 590
   cagugcaaugauauugucaaagc 23
<210> 591
   <211> 21
   <212> RNA
   <213> Homo sapiens
   <400> 591
   ugauauguuugauauuggguu 21
<210> 592
   <211> 22
   <212> RNA
   <213> Homo sapiens
   <400> 592
   uuuguucguucggcucgcguga 22
<210> 593
   <211> 22
   <212> RNA
   <213> Homo sapiens
   <400> 593
   uagcaaaaacugcaguuacuuu 22
<210> 594
   <211> 22
   <212> RNA
   <213> Homo sapiens
   <400> 594
   aggggcuggcuuuccucugguc 22
<210> 595
   <211> 22
   <212> RNA
   <213> Homo sapiens
   <400> 595
   caaagugccucccuuuagagug 22
<210> 596
   <211> 23
   <212> RNA
   <213> Homo sapiens
   <400> 596
   uaaaucccauggugccuucuccu 23
<210> 597
   <211> 20
   <212> RNA
   <213> Homo sapiens
   <400> 597
   guagaggagauggcgcaggg 20
<210> 598
   <211> 22
   <212> RNA
   <213> Homo sapiens
   <400> 598
   acaggugagguucuugggagcc 22
<210> 599
   <211> 22
   <212> RNA
   <213> Homo sapiens
   <400> 599
   cuguugccacuaaccucaaccu 22
<210> 600
   <211> 22
   <212> RNA
   <213> Homo sapiens
   <400> 600
   cucuagagggaagcacuuucug 22
<210> 601
   <211> 22
   <212> RNA
   <213> Homo sapiens
   <400> 601
   aaaguucugagacacuccgacu 22
<210> 602
   <211> 21
   <212> RNA
   <213> Homo sapiens
   <400> 602
   uaacagucuccagucacggcc 21
<210> 603
   <211> 22
   <212> RNA
   <213> Homo sapiens
   <400> 603
   cgucaacacuugcugguuuccu 22
<210> 604
   <211> 22
   <212> RNA
   <213> Homo sapiens
   <400> 604
   ugaguauuacauggccaaucuc 22
<210> 605
   <211> 22
   <212> RNA
   <213> Homo sapiens
   <400> 605
   ucaaaacugaggggcauuuucu 22
<210> 606
   <211> 22
   <212> RNA
   <213> Homo sapiens
   <400> 606
   aaaaacugagacuacuuuugca 22
<210> 607
   <211> 21
   <212> RNA
   <213> Homo sapiens
   <400> 607
   ucuaguaagaguggcagucga 21
<210> 608
   <211> 22
   <212> RNA
   <213> Homo sapiens
   <400> 608
   cccugugcccggcccacuucug 22
<210> 609
   <211> 22
   <212> RNA
   <213> Homo sapiens
   <400> 609
   uuaugguuugccugggacugag 22
<210> 610
   <211> 22
   <212> RNA
   <213> Homo sapiens
   <400> 610
   auguagggcuaaaagccauggg 22
<210> 611
   <211> 21
   <212> RNA
   <213> Homo sapiens
   <400> 611
   uuaggccgcagaucuggguga 21
<210> 612
   <211> 22
   <212> RNA
   <213> Homo sapiens
   <400> 612
   uucaacggguauuuauugagca 22
<210> 613
   <211> 22
   <212> RNA
   <213> Homo sapiens
   <400> 613
   uuugguccccuucaaccagcug 22
<210> 614
   <211> 22
   <212> RNA
   <213> Homo sapiens
   <400> 614
   gucauacacggcucuccucucu 22
<210> 615
   <211> 25
   <212> RNA
   <213> Homo sapiens
   <400> 615
   aaaggauucugcugucggucccacu 25
<210> 616
   <211> 22
   <212> RNA
   <213> Homo sapiens
   <400> 616
   auauaauacaaccugcuaagug 22
<210> 617
   <211> 22
   <212> RNA
   <213> Homo sapiens
   <400> 617
   aacacaccugguuaaccucuuu 22
<210> 618
   <211> 22
   <212> RNA
   <213> Homo sapiens
   <400> 618
   aagacgggaggaaagaagggag 22
<210> 619
   <211> 22
   <212> RNA
   <213> Homo sapiens
   <400> 619
   aggcagcgggguguaguggaua 22
<210> 620
   <211> 22
   <212> RNA
   <213> Homo sapiens
   <400> 620
   cugcgcaagcuacugccuugcu 22
<210> 621
   <211> 23
   <212> RNA
   <213> Homo sapiens
   <400> 621
   ccaguuaccgcuuccgcuaccgc 23
<210> 622
   <211> 22
   <212> RNA
   <213> Homo sapiens
   <400> 622
   cagugcaaugaugaaagggcau 22
<210> 623
   <211> 18
   <212> RNA
   <213> Homo sapiens
   <400> 623
   gucccuguucaggcgcca 18
<210> 624
   <211> 22
   <212> RNA
   <213> Homo sapiens
   <400> 624
   ucaccagcccuguguucccuag 22
<210> 625
   <211> 22
   <212> RNA
   <213> Homo sapiens
   <400> 625
   cucuagagggaagcgcuuucug 22
<210> 626
   <211> 22
   <212> RNA
   <213> Homo sapiens
   <400> 626
   ugagccccugugccgcccccag 22
<210> 627
   <211> 21
   <212> RNA
   <213> Homo sapiens
   <400> 627
   gucagcggaggaaaagaaacu 21
<210> 628
   <211> 22
   <212> RNA
   <213> Homo sapiens
   <400> 628
   cggcaacaagaaacugccugag 22
<210> 629
   <211> 23
   <212> RNA
   <213> Homo sapiens
   <400> 629
   cuggagauauggaagagcugugu 23
<210> 630
   <211> 22
   <212> RNA
   <213> Homo sapiens
   <400> 630
   cuuggcaccuagcaagcacuca 22
<210> 631
   <211> 21
   <212> RNA
   <213> Homo sapiens
   <400> 631
   ugagcuaaaugugugcuggga 21
<210> 632
   <211> 22
   <212> RNA
   <213> Homo sapiens
   <400> 632
   cacgcucaugcacacacccaca 22
<210> 633
   <211> 20
   <212> RNA
   <213> Homo sapiens
   <400> 633
   ucguuugccuuuuucugcuu 20
<210> 634
   <211> 22
   <212> RNA
   <213> Homo sapiens
   <400> 634
   acagauucgauucuaggggaau 22
<210> 635
   <211> 22
   <212> RNA
   <213> Homo sapiens
   <400> 635
   uaaucucagcuggcaacuguga 22
<210> 636
   <211> 22
   <212> RNA
   <213> Homo sapiens
   <400> 636
   ggauaucaucauauacuguaag 22
<210> 637
   <211> 22
   <212> RNA
   <213> Homo sapiens
   <400> 637
   gggguuccuggggaugggauuu 22
<210> 638
   <211> 21
   <212> RNA
   <213> Homo sapiens
   <400> 638
   uuaagacuugcagugauguuu 21
<210> 639
   <211> 22
   <212> RNA
   <213> Homo sapiens
   <400> 639
   uauggcacugguagaauucacu 22
<210> 640
   <211> 22
   <212> RNA
   <213> Homo sapiens
   <400> 640
   caaccuggaggacuccaugcug 22
<210> 641
   <211> 23
   <212> RNA
   <213> Homo sapiens
   <400> 641
   gcaaagcacacggccugcagaga 23
<210> 642
   <211> 21
   <212> RNA
   <213> Homo sapiens
   <400> 642
   cuguacaggccacugccuugc 21
<210> 643
   <211> 21
   <212> RNA
   <213> Homo sapiens
   <400> 643
   ucacuccucuccucccgucuu 21
<210> 644
   <211> 22
   <212> RNA
   <213> Homo sapiens
   <400> 644
   acagcaggcacagacaggcagu 22
<210> 645
   <211> 23
   <212> RNA
   <213> Homo sapiens
   <400> 645
   uaggcagugucauuagcugauug 23
<210> 646
   <211> 22
   <212> RNA
   <213> Homo sapiens
   <400> 646
   acuuuaacauggaggcacuugc 22
<210> 647
   <211> 22
   <212> RNA
   <213> Homo sapiens
   <400> 647
   gaaguuguucgugguggauucg 22
<210> 648
   <211> 22
   <212> RNA
   <213> Homo sapiens
   <400> 648
   acccuaucaauauugucucugc 22
<210> 649
   <211> 21
   <212> RNA
   <213> Homo sapiens
   <400> 649
   gugccagcugcagugggggag 21
<210> 650
   <211> 20
   <212> RNA
   <213> Homo sapiens
   <400> 650
   agagguauagggcaugggaa 20
<210> 651
   <211> 22
   <212> RNA
   <213> Homo sapiens
   <400> 651
   auucugcauuuuuagcaaguuc 22
<210> 652
   <211> 19
   <212> RNA
   <213> Homo sapiens
   <400> 652
   ugucucugcugggguuucu 19
<210> 653
   <211> 20
   <212> RNA
   <213> Homo sapiens
   <400> 653
   cggcucugggucugugggga 20
<210> 654
   <211> 21
   <212> RNA
   <213> Homo sapiens
   <400> 654
   aaggcagggcccccgcucccc 21
<210> 655
   <211> 22
   <212> RNA
   <213> Homo sapiens
   <400> 655
   cuauacggccuccuagcuuucc 22
<210> 656
   <211> 21
   <212> RNA
   <213> Homo sapiens
   <400> 656
   uccuucugcuccgucccccag 21
<210> 657
   <211> 22
   <212> RNA
   <213> Homo sapiens
   <400> 657
   ugcccuaaaugccccuucuggc 22
<210> 658
   <211> 22
   <212> RNA
   <213> Homo sapiens
   <400> 658
   aguauucuguaccagggaaggu 22
<210> 659
   <211> 22
   <212> RNA
   <213> Homo sapiens
   <400> 659
   uucuccaaaagggagcacuuuc 22
<210> 660
   <211> 22
   <212> RNA
   <213> Homo sapiens
   <400> 660
   aacuguuugcagaggaaacuga 22
<210> 661
   <211> 22
   <212> RNA
   <213> Homo sapiens
   <400> 661
   ccuguucuccauuacuuggcuc 22
<210> 662
   <211> 22
   <212> RNA
   <213> Homo sapiens
   <400> 662
   aucuggagguaagaagcacuuu 22
<210> 663
   <211> 22
   <212> RNA
   <213> Homo sapiens
   <400> 663
   uaugugggaugguaaaccgcuu 22
<210> 664
   <211> 22
   <212> RNA
   <213> Homo sapiens
   <400> 664
   uauacaagggcaagcucucugu 22
<210> 665
   <211> 22
   <212> RNA
   <213> Homo sapiens
   <400> 665
   uuauaaagcaaugagacugauu 22
<210> 666
   <211> 22
   <212> RNA
   <213> Homo sapiens
   <400> 666
   uaacagucuacagccauggucg 22
<210> 667
   <211> 23
   <212> RNA
   <213> Homo sapiens
   <400> 667
   uguaguguuuccuacuuuaugga 23
<210> 668
   <211> 22
   <212> RNA
   <213> Homo sapiens
   <400> 668
   acggguuaggcucuugggagcu 22
<210> 669
   <211> 22
   <212> RNA
   <213> Homo sapiens
   <400> 669
   cugggagaaggcuguuuacucu 22
<210> 670
   <211> 22
   <212> RNA
   <213> Homo sapiens
   <400> 670
   gugagucucuaagaaaagagga 22
<210> 671
   <211> 21
   <212> RNA
   <213> Homo sapiens
   <400> 671
   cggcggggacggcgauugguc 21
<210> 672
   <211> 21
   <212> RNA
   <213> Homo sapiens
   <400> 672
   ccuguugaaguguaaucccca 21
<210> 673
   <211> 21
   <212> RNA
   <213> Homo sapiens
   <400> 673
   uuuugcaccuuuuggagugaa 21
<210> 674
   <211> 21
   <212> RNA
   <213> Homo sapiens
   <400> 674
   caucccuugcaugguggaggg 21
<210> 675
   <211> 21
   <212> RNA
   <213> Homo sapiens
   <400> 675
   cggggcagcucaguacaggau 21
<210> 676
   <211> 21
   <212> RNA
   <213> Homo sapiens
   <400> 676
   aagccugcccggcuccucggg 21
<210> 677
   <211> 22
   <212> RNA
   <213> Homo sapiens
   <400> 677
   ugggucuuugcgggcgagauga 22
<210> 678
   <211> 21
   <212> RNA
   <213> Homo sapiens
   <400> 678
   uccgguucucagggcuccacc 21
<210> 679
   <211> 22
   <212> RNA
   <213> Homo sapiens
   <400> 679
   ugccuacugagcugauaucagu 22
<210> 680
   <211> 22
   <212> RNA
   <213> Homo sapiens
   <400> 680
   aguuuugcagguuugcauuuca 22
<210> 681
   <211> 18
   <212> RNA
   <213> Homo sapiens
   <400> 681
   gcaugggugguucagugg 18
<210> 682
   <211> 22
   <212> RNA
   <213> Homo sapiens
   <400> 682
   auaagacgagcaaaaagcuugu 22
<210> 683
   <211> 23
   <212> RNA
   <213> Homo sapiens
   <400> 683
   uauggcuuuuuauuccuauguga 23
<210> 684
   <211> 22
   <212> RNA
   <213> Homo sapiens
   <400> 684
   cuagguauggucccagggaucc 22
<210> 685
   <211> 22
   <212> RNA
   <213> Homo sapiens
   <400> 685
   aacauucaaccugucggugagu 22
<210> 686
   <211> 21
   <212> RNA
   <213> Homo sapiens
   <400> 686
   augauccaggaaccugccucu 21
<210> 687
   <211> 22
   <212> RNA
   <213> Homo sapiens
   <400> 687
   cgcaggggccgggugcucaccg 22
<210> 688
   <211> 21
   <212> RNA
   <213> Homo sapiens
   <400> 688
   uggguuuacguugggagaacu 21
<210> 689
   <211> 23
   <212> RNA
   <213> Homo sapiens
   <400> 689
   uacccuguagauccgaauuugug 23
<210> 690
   <211> 22
   <212> RNA
   <213> Homo sapiens
   <400> 690
   aguggggaacccuuccaugagg 22
<210> 691
   <211> 23
   <212> RNA
   <213> Homo sapiens
   <400> 691
   aggaccugcgggacaagauucuu 23
<210> 692
   <211> 23
   <212> RNA
   <213> Homo sapiens
   <400> 692
   uucucgaggaaagaagcacuuuc 23
<210> 693
   <211> 22
   <212> RNA
   <213> Homo sapiens
   <400> 693
   uacucaggagaguggcaaucac 22
<210> 694
   <211> 20
   <212> RNA
   <213> Homo sapiens
   <400> 694
   ccccagggcgacgcggcggg 20
<210> 695
   <211> 23
   <212> RNA
   <213> Homo sapiens
   <400> 695
   ucucuggagggaagcacuuucug 23
<210> 696
   <211> 25
   <212> RNA
   <213> Homo sapiens
   <400> 696
   gggcgacaaagcaagacucuuucuu 25
<210> 697
   <211> 21
   <212> RNA
   <213> Homo sapiens
   <400> 697
   aggcggagacuugggcaauug 21
<210> 698
   <211> 22
   <212> RNA
   <213> Homo sapiens
   <400> 698
   ugcggggcuagggcuaacagca 22
<210> 699
   <211> 23
   <212> RNA
   <213> Homo sapiens
   <400> 699
   agugccugagggaguaagagccc 23
<210> 700
   <211> 21
   <212> RNA
   <213> Homo sapiens
   <400> 700
   uacuuggaaaggcaucaguug 21
<210> 701
   <211> 22
   <212> RNA
   <213> Homo sapiens
   <400> 701
   uuuagagacggggucuugcucu 22
<210> 702
   <211> 21
   <212> RNA
   <213> Homo sapiens
   <400> 702
   aggaggcagcgcucucaggac 21
<210> 703
   <211> 20
   <212> RNA
   <213> Homo sapiens
   <400> 703
   cgugccacccuuuuccccag 20
<210> 704
   <211> 21
   <212> RNA
   <213> Homo sapiens
   <400> 704
   gaagugugccguggugugucu 21
<210> 705
   <211> 22
   <212> RNA
   <213> Homo sapiens
   <400> 705
   cggaugagcaaagaaagugguu 22
<210> 706
   <211> 22
   <212> RNA
   <213> Homo sapiens
   <400> 706
   agaaggaaauugaauucauuua 22
<210> 707
   <211> 21
   <212> RNA
   <213> Homo sapiens
   <400> 707
   gcaguccaugggcauauacac 21
<210> 708
   <211> 22
   <212> RNA
   <213> Homo sapiens
   <400> 708
   gcugacuccuaguccagggcuc 22
<210> 709
   <211> 23
   <212> RNA
   <213> Homo sapiens
   <400> 709
   uuuggcacuagcacauuuuugcu 23
<210> 710
   <211> 18
   <212> RNA
   <213> Homo sapiens
   <400> 710
   cagggaggugaaugugau 18
<210> 711
   <211> 22
   <212> RNA
   <213> Homo sapiens
   <400> 711
   uucucaaggaggugucguuuau 22
<210> 712
   <211> 22
   <212> RNA
   <213> Homo sapiens
   <400> 712
   aaaaguaauugcgguuuuugcc 22
<210> 713
   <211> 22
   <212> RNA
   <213> Homo sapiens
   <400> 713
   aggcggggcgccgcgggaccgc 22
<210> 714
   <211> 20
   <212> RNA
   <213> Homo sapiens
   <400> 714
   aaaagcuggguugagagggu 20
<210> 715
   <211> 22
   <212> RNA
   <213> Homo sapiens
   <400> 715
   aaaagcuggguugagagggcaa 22
<210> 716
   <211> 22
   <212> RNA
   <213> Homo sapiens
   <400> 716
   uggugggccgcagaacaugugc 22
<210> 717
   <211> 20
   <212> RNA
   <213> Homo sapiens
   <400> 717
   ccucugggcccuuccuccag 20
<210> 718
   <211> 18
   <212> RNA
   <213> Homo sapiens
   <400> 718
   uccagugcccuccucucc 18
<210> 719
   <211> 22
   <212> RNA
   <213> Homo sapiens
   <400> 719
   cuggcccucucugcccuuccgu 22
<210> 720
   <211> 22
   <212> RNA
   <213> Homo sapiens
   <400> 720
   ugagaacugaauuccauaggcu 22
<210> 721
   <211> 21
   <212> RNA
   <213> Homo sapiens
   <400> 721
   cgcgggugcuuacugacccuu 21
<210> 722
   <211> 21
   <212> RNA
   <213> Homo sapiens
   <400> 722
   ugagugccggugccugcccug 21
<210> 723
   <211> 22
   <212> RNA
   <213> Homo sapiens
   <400> 723
   cucggcgcggggcgcgggcucc 22
<210> 724
   <211> 22
   <212> RNA
   <213> Homo sapiens
   <400> 724
   uccagcaucagugauuuuguug 22
<210> 725
   <211> 23
   <212> RNA
   <213> Homo sapiens
   <400> 725
   cgggucggaguuagcucaagcgg 23
<210> 726
   <211> 22
   <212> RNA
   <213> Homo sapiens
   <400> 726
   uggucuaggauuguuggaggag 22
<210> 727
   <211> 22
   <212> RNA
   <213> Homo sapiens
   <400> 727
   uucauucggcuguccagaugua 22
<210> 728
   <211> 21
   <212> RNA
   <213> Homo sapiens
   <400> 728
   ccaguccugugccugccgccu 21
<210> 729
   <211> 26
   <212> RNA
   <213> Homo sapiens
   <400> 729
   gugagggcaugcaggccuggaugggg 26
<210> 730
   <211> 23
   <212> RNA
   <213> Homo sapiens
   <400> 730
   aucaacagacauuaauugggcgc 23
<210> 731
   <211> 22
   <212> RNA
   <213> Homo sapiens
   <400> 731
   gcccgcguguggagccaggugu 22
<210> 732
   <211> 22
   <212> RNA
   <213> Homo sapiens
   <400> 732
   cugguacaggccugggggacag 22
<210> 733
   <211> 23
   <212> RNA
   <213> Homo sapiens
   <400> 733
   cucucaccacugcccucccacag 23
<210> 734
   <211> 22
   <212> RNA
   <213> Homo sapiens
   <400> 734
   acugcagugaaggcacuuguag 22
<210> 735
   <211> 19
   <212> RNA
   <213> Homo sapiens
   <400> 735
   aaaagcuggguugagagga 19
<210> 736
   <211> 23
   <212> RNA
   <213> Homo sapiens
   <400> 736
   uacccuguagaaccgaauuugug 23
<210> 737
   <211> 22
   <212> RNA
   <213> Homo sapiens
   <400> 737
   acuccagccccacagccucagc 22
<210> 738
   <211> 23
   <212> RNA
   <213> Homo sapiens
   <400> 738
   acuuacagacaagagccuugcuc 23
<210> 739
   <211> 24
   <212> RNA
   <213> Homo sapiens
   <400> 739
   aaagacauaggauagagucaccuc 24
<210> 740
   <211> 22
   <212> RNA
   <213> Homo sapiens
   <400> 740
   uccgucucaguuacuuuauagc 22
<210> 741
   <211> 22
   <212> RNA
   <213> Homo sapiens
   <400> 741
   acucaaaacccuucagugacuu 22
<210> 742
   <211> 21
   <212> RNA
   <213> Homo sapiens
   <400> 742
   cuccagagggaaguacuuucu 21
<210> 743
   <211> 21
   <212> RNA
   <213> Homo sapiens
   <400> 743
   aagcauucuuucauugguugg 21
<210> 744
   <211> 21
   <212> RNA
   <213> Homo sapiens
   <400> 744
   uugcucacuguucuucccuag 21
<210> 745
   <211> 22
   <212> RNA
   <213> Homo sapiens
   <400> 745
   cugggagguggauguuuacuuc 22
<210> 746
   <211> 22
   <212> RNA
   <213> Homo sapiens
   <400> 746
   cuccuacauauuagcauuaaca 22
<210> 747
   <211> 22
   <212> RNA
   <213> Homo sapiens
   <400> 747
   gcuacuucacaacaccagggcc 22
<210> 748
   <211> 22
   <212> RNA
   <213> Homo sapiens
   <400> 748
   aauccuuugucccugggugaga 22
<210> 749
   <211> 23
   <212> RNA
   <213> Homo sapiens
   <400> 749
   caacggaaucccaaaagcagcug 23
<210> 750
   <211> 23
   <212> RNA
   <213> Homo sapiens
   <400> 750
   agcagcauuguacagggcuauca 23
<210> 751
   <211> 23
   <212> RNA
   <213> Homo sapiens
   <400> 751
   aucgcugcgguugcgagcgcugu 23
<210> 752
   <211> 21
   <212> RNA
   <213> Homo sapiens
   <400> 752
   caaagcgcuucccuuuggagc 21
<210> 753
   <211> 21
   <212> RNA
   <213> Homo sapiens
   <400> 753
   uaaagugcugacagugcagau 21
<210> 754
   <211> 22
   <212> RNA
   <213> Homo sapiens
   <400> 754
   aagcccuuaccccaaaaagcau 22
<210> 755
   <211> 18
   <212> RNA
   <213> Homo sapiens
   <400> 755
   acguuggcucugguggug 18
<210> 756
   <211> 22
   <212> RNA
   <213> Homo sapiens
   <400> 756
   ggcuacaacacaggacccgggc 22
<210> 757
   <211> 22
   <212> RNA
   <213> Homo sapiens
   <400> 757
   ucccugagacccuaacuuguga 22
<210> 758
   <211> 22
   <212> RNA
   <213> Homo sapiens
   <400> 758
   gucccucuccaaaugugucuug 22
<210> 759
   <211> 22
   <212> RNA
   <213> Homo sapiens
   <400> 759
   cuuucagucggauguuugcagc 22
<210> 760
   <211> 22
   <212> RNA
   <213> Homo sapiens
   <400> 760
   ucuacagugcacgugucuccag 22
<210> 761
   <211> 22
   <212> RNA
   <213> Homo sapiens
   <400> 761
   acucggcguggcgucggucgug 22
<210> 762
   <211> 23
   <212> RNA
   <213> Homo sapiens
   <400> 762
   cugggaucuccggggucuugguu 23
<210> 763
   <211> 22
   <212> RNA
   <213> Homo sapiens
   <400> 763
   caugccuugaguguaggaccgu 22
<210> 764
   <211> 22
   <212> RNA
   <213> Homo sapiens
   <400> 764
   caacaaaucacagucugccaua 22
<210> 765
   <211> 22
   <212> RNA
   <213> Homo sapiens
   <400> 765
   uagguaguuucauguuguuggg 22
<210> 766
   <211> 22
   <212> RNA
   <213> Homo sapiens
   <400> 766
   uuagggcccuggcuccaucucc 22
<210> 767
   <211> 22
   <212> RNA
   <213> Homo sapiens
   <400> 767
   gcugcgcuuggauuucgucccc 22
<210> 768
   <211> 23
   <212> RNA
   <213> Homo sapiens
   <400> 768
   agcuacauugucugcuggguuuc 23
<210> 769
   <211> 23
   <212> RNA
   <213> Homo sapiens
   <400> 769
   agguugggaucgguugcaaugcu 23
<210> 770
   <211> 22
   <212> RNA
   <213> Homo sapiens
   <400> 770
   ucugggcaacaaagugagaccu 22
<210> 771
   <211> 22
   <212> RNA
   <213> Homo sapiens
   <400> 771
   cucuagagggaagcacuuucuc 22
<210> 772
   <211> 20
   <212> RNA
   <213> Homo sapiens
   <400> 772
   ugagcccuguccucccgcag 20
<210> 773
   <211> 19
   <212> RNA
   <213> Homo sapiens
   <400> 773
   uggauuuuuggaucaggga 19
<210> 774
   <211> 22
   <212> RNA
   <213> Homo sapiens
   <400> 774
   uacgucaucguugucaucguca 22
<210> 775
   <211> 22
   <212> RNA
   <213> Homo sapiens
   <400> 775
   ugagaccucuggguucugagcu 22
<210> 776
   <211> 23
   <212> RNA
   <213> Homo sapiens
   <400> 776
   gaacgccuguucuugccaggugg 23
<210> 777
   <211> 21
   <212> RNA
   <213> Homo sapiens
   <400> 777
   cuucuugugcucuaggauugu 21
<210> 778
   <211> 24
   <212> RNA
   <213> Homo sapiens
   <400> 778
   uugcagcugccugggagugacuuc 24
<210> 779
   <211> 24
   <212> RNA
   <213> Homo sapiens
<400> 779
   uucuccaaaagaaagcacuuucug 24
<210> 780
   <211> 19
   <212> RNA
   <213> Homo sapiens
   <400> 780
   aggcacggugucagcaggc 19
<210> 781
   <211> 22
   <212> RNA
   <213> Homo sapiens
   <400> 781
   aaccagcaccccaacuuuggac 22
<210> 782
   <211> 22
   <212> RNA
   <213> Homo sapiens
   <400> 782
   caaagcgcuccccuuuagaggu 22
<210> 783
   <211> 23
   <212> RNA
   <213> Homo sapiens
   <400> 783
   cacccggcugugugcacaugugc 23
<210> 784
   <211> 21
   <212> RNA
   <213> Homo sapiens
   <400> 784
   aacauagaggaaauuccacgu 21
<210> 785
   <211> 22
   <212> RNA
   <213> Homo sapiens
   <400> 785
   ccaauauuggcugugcugcucc 22
<210> 786
   <211> 20
   <212> RNA
   <213> Homo sapiens
   <400> 786
   cugcaaagggaagcccuuuc 20
<210> 787
   <211> 23
   <212> RNA
   <213> Homo sapiens
   <400> 787
   guuugcacgggugggccuugucu 23
<210> 788
   <211> 21
   <212> RNA
   <213> Homo sapiens
   <400> 788
   gugggcgggggcaggugugug 21
<210> 789
   <211> 22
   <212> RNA
   <213> Homo sapiens
   <400> 789
   aguucuucaguggcaagcuuua 22
<210> 790
   <211> 22
   <212> RNA
   <213> Homo sapiens
   <400> 790
   ucggccugaccacccaccccac 22
<210> 791
   <211> 21
   <212> RNA
   <213> Homo sapiens
   <400> 791
   agggagggacgggggcugugc 21
<210> 792
   <211> 22
   <212> RNA
   <213> Homo sapiens
   <400> 792
   cugggagaggguuguuuacucc 22
<210> 793
   <211> 22
   <212> RNA
   <213> Homo sapiens
   <400> 793
   cgucuuacccagcaguguuugg 22
<210> 794
   <211> 21
   <212> RNA
   <213> Homo sapiens
   <400> 794
   ccgucgccgccacccgagccg 21
<210> 795
   <211> 22
   <212> RNA
   <213> Homo sapiens
   <400> 795
   aggugguccguggcgcguucgc 22
<210> 796
   <211> 20
   <212> RNA
   <213> Homo sapiens
   <400> 796
   gugucugggcggacagcugc 20
<210> 797
   <211> 22
   <212> RNA
   <213> Homo sapiens
   <400> 797
   gugaaauguuuaggaccacuag 22
<210> 798
   <211> 22
   <212> RNA
   <213> Homo sapiens
   <400> 798
   uuuaacauggggguaccugcug 22
<210> 799
   <211> 22
   <212> RNA
   <213> Homo sapiens
   <400> 799
   aacccguagauccgaucuugug 22
<210> 800
   <211> 22
   <212> RNA
   <213> Homo sapiens
   <400> 800
   ugagaacugaauuccauggguu 22
<210> 801
   <211> 21
   <212> RNA
   <213> Homo sapiens
   <400> 801
   aauauuauacagucaaccucu 21
<210> 802
   <211> 22
   <212> RNA
   <213> Homo sapiens
   <400> 802
   gaaagugcuuccuuuuagaggc 22
<210> 803
   <211> 22
   <212> RNA
   <213> Homo sapiens
   <400> 803
   aaguucuguuauacacucaggc 22
<210> 804
   <211> 23
   <212> RNA
   <213> Homo sapiens
   <400> 804
   aacauucaacgcugucggugagu 23
<210> 805
   <211> 21
   <212> RNA
   <213> Homo sapiens
   <400> 805
   acagucugcugagguuggagc 21
<210> 806
   <211> 24
   <212> RNA
   <213> Homo sapiens
   <400> 806
   ucccugagacccuuuaaccuguga 24
<210> 807
   <211> 21
   <212> RNA
   <213> Homo sapiens
   <400> 807
   ucagugcaugacagaacuugg 21
<210> 808
   <211> 22
   <212> RNA
   <213> Homo sapiens
   <400> 808
   uucaccaccuucuccacccagc 22
<210> 809
   <211> 21
   <212> RNA
   <213> Homo sapiens
   <400> 809
   uucacaguggcuaaguucugc 21
<210> 810
   <211> 22
   <212> RNA
   <213> Homo sapiens
   <400> 810
   ccucuuccccuugucucuccag 22
<210> 811
   <211> 22
   <212> RNA
   <213> Homo sapiens
   <400> 811
   aaacaaacauggugcacuucuu 22
<210> 812
   <211> 21
   <212> RNA
   <213> Homo sapiens
   <400> 812
   ugagaugaagcacuguagcuc 21
<210> 813
   <211> 22
   <212> RNA
   <213> Homo sapiens
   <400> 813
   aacacaccuauucaaggauuca 22
<210> 814
   <211> 23
   <212> RNA
   <213> Homo sapiens
   <400> 814
   uggugcggagagggcccacagug 23
<210> 815
   <211> 17
   <212> RNA
   <213> Homo sapiens
   <400> 815
   ucccuguucgggcgcca 17
<210> 816
   <211> 22
   <212> RNA
   <213> Homo sapiens
   <400> 816
   ggagacgcggcccuguuggagu 22
<210> 817
   <211> 21
   <212> RNA
   <213> Homo sapiens
   <400> 817
   acucuuucccuguugcacuac 21
<210> 818
   <211> 20
   <212> RNA
   <213> Homo sapiens
   <400> 818
   ucacaccugccucgcccccc 20
<210> 819
   <211> 22
   <212> RNA
   <213> Homo sapiens
   <400> 819
   uuuccggcucgcgugggugugu 22
<210> 820
   <211> 19
   <212> RNA
   <213> Homo sapiens
   <400> 820
   gagccaguuggacaggagc 19
<210> 821
   <211> 22
   <212> RNA
   <213> Homo sapiens
   <400> 821
   ugugacugguugaccagagggg 22
<210> 822
   <211> 21
   <212> RNA
   <213> Homo sapiens
   <400> 822
   ccuggaaacacugagguugug 21
<210> 823
   <211> 22
   <212> RNA
   <213> Homo sapiens
   <400> 823
   agggacgggacgcggugcagug 22
<210> 824
   <211> 22
   <212> RNA
   <213> Homo sapiens
   <400> 824
   uacccauugcauaucggaguug 22
<210> 825
   <211> 23
   <212> RNA
   <213> Homo sapiens
   <400> 825
   cucuugagggaagcacuuucugu 23
<210> 826
   <211> 22
   <212> RNA
   <213> Homo sapiens
   <400> 826
   acuggacuuagggucagaaggc 22
<210> 827
   <211> 21
   <212> RNA
   <213> Homo sapiens
   <400> 827
   acggugcuggauguggccuuu 21
<210> 828
   <211> 22
   <212> RNA
   <213> Homo sapiens
   <400> 828
   ugcccuuaaaggugaacccagu 22
<210> 829
   <211> 25
   <212> RNA
   <213> Homo sapiens
   <400> 829
   aggggugguguugggacagcuccgu 25
<210> 830
   <211> 17
   <212> RNA
   <213> Homo sapiens
   <400> 830
   ucauauugcuucuuucu 17
<210> 831
   <211> 22
   <212> RNA
   <213> Homo sapiens
   <400> 831
   acgcccuucccccccuucuuca 22
<210> 832
   <211> 24
   <212> RNA
   <213> Homo sapiens
   <400> 832
   ugccugggucucuggccugcgcgu 24
<210> 833
   <211> 20
   <212> RNA
   <213> Homo sapiens
   <400> 833
   ucacuguucagacaggcgga 20
<210> 834
   <211> 22
   <212> RNA
   <213> Homo sapiens
   <400> 834
   cagugcaauguuaaaagggcau 22
<210> 835
   <211> 22
   <212> RNA
   <213> Homo sapiens
   <400> 835
   uuuugcaauauguuccugaaua 22
<210> 836
   <211> 23
   <212> RNA
   <213> Homo sapiens
   <400> 836
   ucuuggaguaggucauugggugg 23
<210> 837
   <211> 22
   <212> RNA
   <213> Homo sapiens
   <400> 837
   gaauguugcucggugaaccccu 22
<210> 838
   <211> 20
   <212> RNA
   <213> Homo sapiens
   <400> 838
   cugcaaagggaagcccuuuc 20
<210> 839
   <211> 21
   <212> RNA
   <213> Homo sapiens
   <400> 839
   uccucuucucccuccucccag 21
<210> 840
   <211> 20
   <212> RNA
   <213> Homo sapiens
   <400> 840
   cuuccucgucugucugcccc 20
<210> 841
   <211> 22
   <212> RNA
   <213> Homo sapiens
   <400> 841
   ccucuagauggaagcacugucu 22
<210> 842
   <211> 22
   <212> RNA
   <213> Homo sapiens
   <400> 842
   cgggguuuugagggcgagauga 22
<210> 843
   <211> 22
   <212> RNA
   <213> Homo sapiens
   <400> 843
   cuacaaagggaagcacuuucuc 22
<210> 844
   <211> 21
   <212> RNA
   <213> Homo sapiens
   <400> 844
   aguuaggauuaggucguggaa 21
<210> 845
   <211> 22
   <212> RNA
   <213> Homo sapiens
   <400> 845
   uagguuauccguguugccuucg 22
<210> 846
   <211> 24
   <212> RNA
   <213> Homo sapiens
   <400> 846
   acugggggcuuucgggcucugcgu 24
<210> 847
   <211> 21
   <212> RNA
   <213> Homo sapiens
   <400> 847
   uuggccacaauggguuagaac 21
<210> 848
   <211> 23
   <212> RNA
   <213> Homo sapiens
   <400> 848
   uuaaugcuaaucgugauaggggu 23
<210> 849
   <211> 24
   <212> RNA
   <213> Homo sapiens
   <400> 849
   acuggcuagggaaaaugauuggau 24
<210> 850
   <211> 21
   <212> RNA
   <213> Homo sapiens
   <400> 850
   gcccuccgcccgugcaccccg 21
<210> 851
   <211> 22
   <212> RNA
   <213> Homo sapiens
   <400> 851
   acggauguuugagcaugugcua 22
<210> 852
   <211> 23
   <212> RNA
   <213> Homo sapiens
   <400> 852
   cgcauccccuagggcauuggugu 23
<210> 853
   <211> 22
   <212> RNA
   <213> Homo sapiens
   <400> 853
   aagcccuuaccccaaaaaguau 22
<210> 854
   <211> 21
   <212> RNA
   <213> Homo sapiens
   <400> 854
   agggggaaaguucuauagucc 21
<210> 855
   <211> 22
   <212> RNA
   <213> Homo sapiens
   <400> 855
   cucuagagggaagcgcuuucug 22
<210> 856
   <211> 22
   <212> RNA
   <213> Homo sapiens
   <400> 856
   accacugaccguugacuguacc 22
<210> 857
   <211> 23
   <212> RNA
   <213> Homo sapiens
   <400> 857
   cccaguguuuagacuaucuguuc 23
<210> 858
   <211> 21
   <212> RNA
   <213> Homo sapiens
   <400> 858
   uucacaguggcuaaguuccgc 21
<210> 859
   <211> 22
   <212> RNA
   <213> Homo sapiens
   <400> 859
   gaaagcgcuucccuuugcugga 22
<210> 860
   <211> 22
   <212> RNA
   <213> Homo sapiens
   <400> 860
   caggauguggucaaguguuguu 22
<210> 861
   <211> 22
   <212> RNA
   <213> Homo sapiens
   <400> 861
   uauugcacuugucccggccugu 22
<210> 862
   <211> 24
   <212> RNA
   <213> Homo sapiens
   <400> 862
   gcugguuucauauggugguuuaga 24
<210> 863
   <211> 22
   <212> RNA
   <213> Homo sapiens
   <400> 863
   ucucccaacccuuguaccagug 22
<210> 864
   <211> 23
   <212> RNA
   <213> Homo sapiens
   <400> 864
   ucaagagcaauaacgaaaaaugu 23
<210> 865
   <211> 25
   <212> RNA
   <213> Homo sapiens
   <400> 865
   agggaucgcgggcggguggcggccu 25
210> 866
   <211> 20
   <212> RNA
   <213> Homo sapiens
   <400> 866
   aaaguagcuguaccauuugc 20